# EUROPEAN PATENT APPLICATION

(11) **EP 4 834 896 A1**
(43) Date of publication of application: **04.11.2026**
(21) Application number: 24911203.8
(22) Date of filing: 25.12.2024
(51) Int. Cl.: C07D 401/12, A61K 31/5377, A61P 21/00

(54) **QUATERNARY AMMONIUM COMPOUND AND PHARMACEUTICAL USE THEREOF**

(30) Priority: 25.12.2023 CN 202311793501; 21.05.2024 CN 202410632066; 20.08.2024 CN 202411140696
(71) Applicant: Shanghai Senhui Medicine Co., Ltd., Shanghai 201203 (CN); Shanghai Shengdi Pharmaceutical Co., Ltd, Shanghai 201210 (CN); Jiangsu Hengrui Pharmaceuticals Co., Ltd., Lianyungang, Jiangsu 222047 (CN)
(72) Inventor: ZHU, Lingjian, Shanghai 201203 (CN); MENG, Lijuan, Shanghai 201203 (CN); HUANG, Jian, Shanghai 201203 (CN); GUAN, Zhongjun, Shanghai 201203 (CN); WU, Yuan, Shanghai 201203 (CN); ZONG, Haichao, Shanghai 201203 (CN); WU, Qimei, Shanghai 201203 (CN)
(74) Representative: Regimbeau
(86) International application number: PCT/CN2024/142210
(87) International publication number: WO 2025/140279

(57) **Abstract**

The present disclosure relates to a quaternary ammonium compound and a pharmaceutical use thereof. Specifically, the present disclosure relates to a compound represented by formula IA or a pharmaceutically acceptable salt thereof, each substituent being as defined in the description.

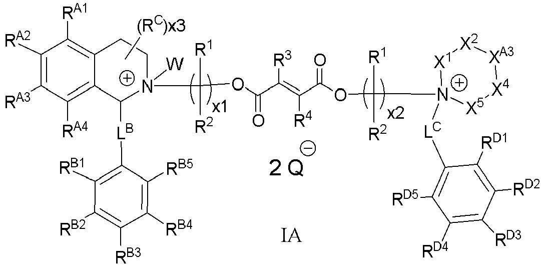

## Description

### Technical Field

The present invention belongs to the field of medicine, and relates to a quaternary ammonium compound and the pharmaceutical use thereof.

### Background Art

Neuromuscular-blocking drugs (NMBDs) play an important role in the field of anesthesiology, and can be classified into non-depolarizing and depolarizing types according to their mechanisms of action and effects. Non-depolarizing drugs, such as vecuronium bromide, block the action of acetylcholine by competitively binding to cholinergic receptors at the neuromuscular junction, leading to muscle paralysis. In contrast, depolarizing drugs, such as succinylcholine, prevent normal muscle contraction by interfering with the membrane potential of nerve terminals.

WO 2014005122 discloses a series of ultra-short-acting, short-acting, and intermediate-acting non-depolarizing neuromuscular-blocking drugs, wherein the neuromuscular blockade (NMB) induced by the agents is reversible, e.g., by administering cysteine or related compounds.

### Summary of the Invention

The present invention provides a compound represented by formula IA or a pharmaceutically acceptable salt thereof, 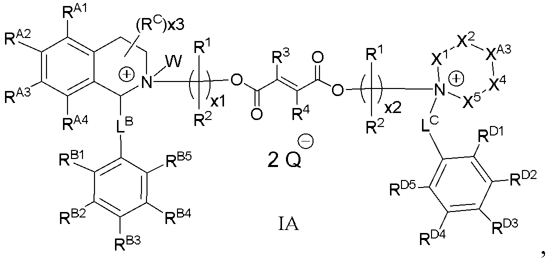 wherein:
W is selected from the group consisting of -C(R^{a}R^{b}R^{c}) or 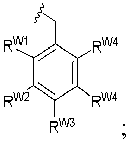
each Q^{Θ} is independently selected from the group consisting of pharmaceutically acceptable anions;
R³ and R⁴ are each independently selected from the group consisting of hydrogen, halogen, C₁₋₆ alkyl, or C₁₋₆ alkoxy, wherein the C₁₋₆ alkyl or C₁₋₆ alkoxy is optionally substituted with one or more halogen, hydroxy, mercapto, carboxy, amino, or cyano;
L^{B} and L^{C} are each independently selected from the group consisting of - C(R^{a}R^{b}R^{c}) or alkylene, wherein the alkylene is optionally substituted with one or more halogen, hydroxy, mercapto, nitro, carboxy, amino, cyano, C₁₋₆ alkyl, C₁₋₆ alkoxy, oxo, thioxo, or sulfonyl, and the C₁₋₆ alkyl or C₁₋₆ alkoxy is optionally substituted with one or more halogen, hydroxy, mercapto, carboxy, amino, or cyano;
when L^{B} is -C(R^{a}R^{b}R^{c}), the phenyl with R^{B1} to R^{B5} substituents is absent;
when L^{C} is -C(R^{a}R^{b}R^{c}), the phenyl with R^{D1} to R^{D5} substituents is absent;
X^{A3} is selected from the group consisting of a chemical bond, -O-, -C(R^{d}R^{e})-, - N(R^{f})-, or -S(O)₂-;
X¹ and X⁵ are each independently -C(R^{g}R^{h})-;
X² and X⁴ are each independently -C(RⁱR^{j})-;
R^{f} is selected from the group consisting of hydrogen, C₁₋₆ alkyl, or C₁₋₆ alkoxy, wherein the alkyl or alkoxy is optionally substituted with one or more halogen, hydroxy, mercapto, carboxy, amino, or cyano;
R¹, R², R^{A1}, R^{A2}, R^{A3}, R^{A2}, R^{B1}, R^{B2}, R^{B3}, R^{B4}, R^{B5}, R^{C}, R^{D1}, R^{D2}, R^{D3}, R^{D4}, R^{D5}, R^{W1}, R^{W2}, R^{W3}, R^{W4}, R^{W5}, R^{a}, R^{b}, R^{c}, R^{d}, R^{e}, R^{g}, R^{h}, Rⁱ, and R^{j} are each independently selected from the group consisting of hydrogen, halogen, hydroxy, mercapto, carboxy, amino, cyano, C₁₋₆ alkyl, C₁₋₆ alkoxy, -C(O)-C₁₋₆ alkyl, -C(O)-C₁₋₆ alkoxy, methanesulfonyl, a 3- to 10-membered carbocyclic ring, a 3- to 10-membered heterocyclic ring, a 6- to 10-membered aromatic ring, or a 5- to 10-membered heteroaromatic ring, wherein the alkyl, alkoxy, carbocyclic ring, heterocyclic ring, aromatic ring, or heteroaromatic ring is optionally substituted with one or more halogen, hydroxy, mercapto, carboxy, amino, or cyano,
or alternatively, one or more pairs of R¹ and R², R^{d} and R^{e}, R^{g} and R^{h}, or Rⁱ and R^{j} together with the atoms to which they are attached form oxo, thioxo, a 3- to 10-membered carbocyclic ring, a 3- to 10-membered heterocyclic ring, a 6- to 10-membered aromatic ring, or a 5- to 10-membered heteroaromatic ring, wherein the carbocyclic ring, heterocyclic ring, aromatic ring, or heteroaromatic ring is optionally substituted with one or more halogen, hydroxy, mercapto, carboxy, amino, cyano, C₁₋₆ alkyl, or C₁₋₆ alkoxy, and the alkyl or alkoxy is optionally substituted with one or more halogen, hydroxy, mercapto, carboxy, amino, or cyano,
or alternatively, R^{g} and Rⁱ together with the atoms to which they are attached form a 3- to 10-membered carbocyclic ring, a 3- to 10-membered heterocyclic ring, a 6- to 10-membered aromatic ring, or a 5- to 10-membered heteroaromatic ring, wherein the carbocyclic ring, heterocyclic ring, aromatic ring, or heteroaromatic ring is optionally substituted with one or more halogen, hydroxy, mercapto, carboxy, amino, cyano, C₁₋₆ alkyl, or C₁₋₆ alkoxy, and the alkyl or alkoxy is optionally substituted with one or more halogen, hydroxy, mercapto, carboxy, amino, or cyano,
or alternatively, any two adjacent groups of R^{A1}, R^{A2}, R^{A3}, and R^{A4} together form methylenedioxy, ethylenedioxy, or -CH₂-O-CH₂-,
or alternatively, any two adjacent groups of R^{B1}, R^{B2}, R^{B3}, R^{B4}, and R^{B5} together form methylenedioxy, ethylenedioxy, or -CH₂-O-CH₂-,
or alternatively, any two adjacent groups of R^{D1}, R^{D2}, R^{D3}, R^{D4}, and R^{D5} together form methylenedioxy, ethylenedioxy, or -CH₂-O-CH₂-,
or alternatively, any two adjacent groups of R^{W1}, R^{W2}, R^{W3}, R^{W4}, and R^{W5} together form methylenedioxy, ethylenedioxy, or -CH₂-O-CH₂-;
when X^{A3} is selected from the group consisting of a chemical bond or -C(R^{d}R^{e})-,
   (i) R^{d} and R^{e} cannot both be hydrogen, or alternatively,
   (ii) one or more pairs of R^{d} and R^{e}, R^{g} and R^{h}, or Rⁱ and R^{j} together with the atoms to which they are attached form oxo, thioxo, a 3- to 10-membered carbocyclic ring, a 3- to 10-membered heterocyclic ring, a 6- to 10-membered aromatic ring, or a 5- to 10-membered heteroaromatic ring, wherein the carbocyclic ring, heterocyclic ring, aromatic ring, or heteroaromatic ring is optionally substituted with one or more halogen, hydroxy, mercapto, carboxy, amino, cyano, C₁₋₆ alkyl, or C₁₋₆ alkoxy, and the alkyl or alkoxy is optionally substituted with one or more halogen, hydroxy, mercapto, carboxy, amino, or cyano, or alternatively,
   (iii) R^{g} and Rⁱ together with the atoms to which they are attached form a 3- to 10-membered carbocyclic ring, a 3- to 10-membered heterocyclic ring, a 6- to 10-membered aromatic ring, or a 5- to 10-membered heteroaromatic ring, wherein the carbocyclic ring, heterocyclic ring, aromatic ring, or heteroaromatic ring is optionally substituted with one or more halogen, hydroxy, mercapto, carboxy, amino, cyano, C₁₋₆ alkyl, or C₁₋₆ alkoxy, and the alkyl or alkoxy is optionally substituted with one or more halogen, hydroxy, mercapto, carboxy, amino, or cyano;
when X^{A3} is -O-,
   (i) one or both pairs of R^{g} and R^{h} or Rⁱ and R^{j} together with the atoms to which they are attached form oxo, thioxo, a 3- to 10-membered carbocyclic ring, a 3- to 10-membered heterocyclic ring, a 6- to 10-membered aromatic ring, or a 5- to 10-membered heteroaromatic ring, wherein the carbocyclic ring, heterocyclic ring, aromatic ring, or heteroaromatic ring is optionally substituted with one or more halogen, hydroxy, mercapto, carboxy, amino, cyano, C₁₋₆ alkyl, or C₁₋₆ alkoxy, and the alkyl or alkoxy is optionally substituted with one or more halogen, hydroxy, mercapto, carboxy, amino, or cyano, or alternatively,
   (ii) R^{g} and Rⁱ together with the atoms to which they are attached form a 3- to 10-membered carbocyclic ring, a 3- to 10-membered heterocyclic ring, a 6- to 10-membered aromatic ring, or a 5- to 10-membered heteroaromatic ring, wherein the carbocyclic ring, heterocyclic ring, aromatic ring, or heteroaromatic ring is optionally substituted with one or more halogen, hydroxy, mercapto, carboxy, amino, cyano, C₁₋₆ alkyl, or C₁₋₆ alkoxy, and the alkyl or alkoxy is optionally substituted with one or more halogen, hydroxy, mercapto, carboxy, amino, or cyano;
x1 is selected from the group consisting of 1, 2, 3, 4, 5, or 6;
x2 is selected from the group consisting of 1, 2, 3, 4, 5, or 6; and
x3 is selected from the group consisting of 0, 1, 2, 3, 4, or 5.

In some embodiments, X^{A3} is selected from the group consisting of a chemical bond, -C(R^{d}R^{e})-, or -S(O)₂-, wherein R^{d} and R^{e} are as defined in the foregoing.

In some embodiments, the compound represented by formula IA is selected from the group consisting of the compound represented by formula IIA1, formula IIA2, formula IIA3, formula IIA4, or formula IIA5, or a pharmaceutically acceptable salt thereof, 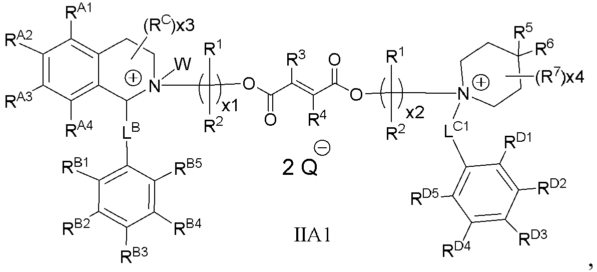 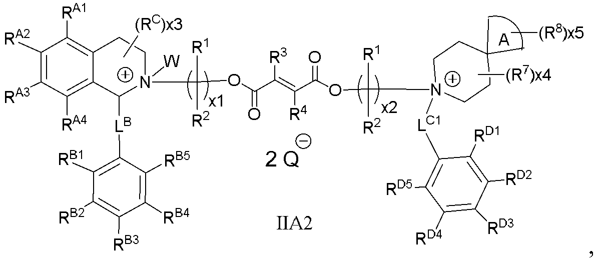 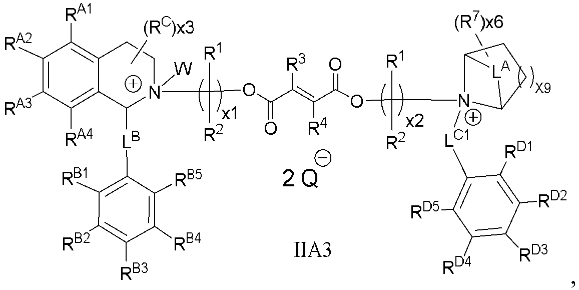 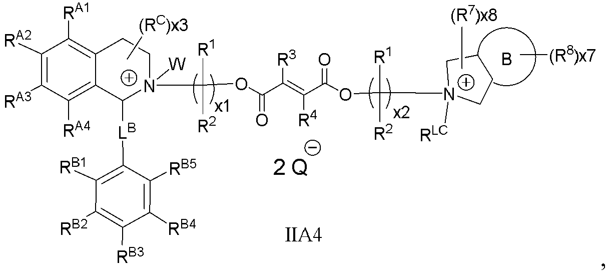 or 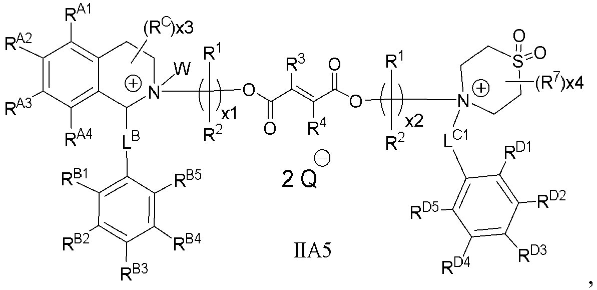 wherein
ring A and ring B are each independently selected from the group consisting of a 3- to 10-membered carbocyclic ring, a 3- to 10-membered heterocyclic ring, a 6-to 10-membered aromatic ring, or a 5- to 10-membered heteroaromatic ring;
L^{A} is selected from the group consisting of alkylene or heteroalkylene, wherein the alkylene or heteroalkylene is optionally substituted with one or more halogen, hydroxy, mercapto, nitro, carboxy, amino, cyano, C₁₋₆ alkyl, C₁₋₆ alkoxy, oxo, thioxo, or sulfonyl, and the C₁₋₆ alkyl or C₁₋₆ alkoxy is optionally substituted with one or more halogen, hydroxy, mercapto, carboxy, amino, or cyano;
L^{C1} is alkylene, wherein the alkylene is optionally substituted with one or more halogen, hydroxy, mercapto, nitro, carboxy, amino, cyano, C₁₋₆ alkyl, C₁₋₆ alkoxy, oxo, thioxo, or sulfonyl, and the C₁₋₆ alkyl or C₁₋₆ alkoxy is optionally substituted with one or more halogen, hydroxy, mercapto, carboxy, amino, or cyano;
R^{LC} is C₁₋₆ alkyl, wherein the alkyl is optionally substituted with one or more halogen, hydroxy, mercapto, carboxy, amino, or cyano;
R⁵, R⁶, R⁷, and R⁸ are each independently selected from the group consisting of hydrogen, halogen, hydroxy, mercapto, carboxy, amino, cyano, C₁₋₆ alkyl, C₁₋₆ alkoxy, -C(O)-C₁₋₆ alkyl, -C(O)-C₁₋₆ alkoxy, methanesulfonyl, a 3- to 10-membered carbocyclic ring, a 3- to 10-membered heterocyclic ring, a 6- to 10-membered aromatic ring, or a 5- to 10-membered heteroaromatic ring, wherein the alkyl, alkoxy, carbocyclic ring, heterocyclic ring, aromatic ring, or heteroaromatic ring is optionally substituted with one or more halogen, hydroxy, mercapto, carboxy, amino, or cyano, and R⁵ and R⁶ cannot both be hydrogen,
or alternatively, R⁵ and R⁶ together with the atoms to which they are attached form oxo or thioxo;
each x4 is independently selected from the group consisting of 0, 1, 2, 3, 4, 5, 6, 7, or 8;
x5 is selected from the group consisting of 0, 1, 2, 3, or 4;
each x6 is independently selected from the group consisting of 0, 1, 2, 3, 4, 5, or 6;
x7 is selected from the group consisting of 0, 1, 2, 3, or 4;
x8 is selected from the group consisting of 0, 1, 2, 3, or 4;
x9 is selected from the group consisting of 0, 1, 2, or 3; and
W, Q^{Θ}, L^{B}, R¹, R², R³, R⁴, R^{A1}, R^{A2}, R^{A3}, R^{A2}, R^{B1}, R^{B2}, R^{B3}, R^{B4}, R^{B5}, R^{C}, R^{D1}, R^{D2}, R^{D3}, R^{D4}, R^{D5}, x1, x2, and x3 are as defined in the foregoing.

In some embodiments, ring A is a 3- to 10-membered heterocyclic ring. In some embodiments, ring A is selected from the group consisting of an oxetane ring, an azetidine ring, a thietane ring, a tetrahydropyrrole ring, a tetrahydrofuran ring, a tetrahydrothiophene ring, a piperidine ring, a tetrahydropyran ring, or a tetrahydrothiopyran ring. In some embodiments, ring A is an oxetane ring.

In some embodiments, ring B is selected from the group consisting of a 6- to 10-membered aromatic ring or a 5- to 10-membered heteroaromatic ring. In some embodiments, ring B is selected from the group consisting of a benzene ring, a pyridine ring, a pyran ring, or a thiopyran ring. In some embodiments, ring B is selected from the group consisting of a benzene ring or a pyridine ring.

In some embodiments, L^{A} is selected from the group consisting of -(CR^{k}R^{l})ₐ-O-(CR^{k}R^{l})_{b}-, -(CR^{k}R^{l})ₐ-S-(CR^{k}R^{l})_{b}-, -(CR^{k}R^{l})ₐ-N(R^{f})-(CR^{k}R^{l})_{b}-, or -(CR^{k}R^{l})_{c}-, preferably -(CR^{k}R^{l})ₐ-O-(CR^{k}R^{l})_{b}- or -(CR^{k}R^{l})_{c}-, wherein
R^{k} and R^{l} are each independently selected from the group consisting of hydrogen, halogen, hydroxy, mercapto, carboxy, amino, cyano, C₁₋₆ alkyl, C₁₋₆ alkoxy, -C(O)-C₁₋₆ alkyl, -C(O)-C₁₋₆ alkoxy, methanesulfonyl, a 3- to 10-membered carbocyclic ring, a 3- to 10-membered heterocyclic ring, a 6- to 10-membered aromatic ring, or a 5- to 10-membered heteroaromatic ring, wherein the alkyl, alkoxy, carbocyclic ring, heterocyclic ring, aromatic ring, or heteroaromatic ring is optionally substituted with one or more halogen, hydroxy, mercapto, carboxy, amino, or cyano,
or alternatively, R^{k} and R^{l} together with the atoms to which they are attached form oxo, thioxo, a 3- to 10-membered carbocyclic ring, a 3- to 10-membered heterocyclic ring, a 6- to 10-membered aromatic ring, or a 5- to 10-membered heteroaromatic ring, wherein the carbocyclic ring, heterocyclic ring, aromatic ring, or heteroaromatic ring is optionally substituted with one or more halogen, hydroxy, mercapto, carboxy, amino, cyano, C₁₋₆ alkyl, or C₁₋₆ alkoxy, and the alkyl or alkoxy is optionally substituted with one or more halogen, hydroxy, mercapto, carboxy, amino, or cyano;
each a is independently selected from the group consisting of 0, 1, 2, 3, 4, or 5;
each b is independently selected from the group consisting of 0, 1, 2, 3, 4, or 5;
each c is independently selected from the group consisting of 1, 2, 3, 4, 5, or 6; and
R^{f} is as defined in the foregoing.

In some embodiments, a is 1.

In some embodiments, b is 1.

In some embodiments, L^{A} is -(CR^{k}R^{l})-O-(CR^{k}R^{l})-.

In some embodiments, R⁵ and R⁶ cannot both be hydrogen, and are each independently selected from the group consisting of hydrogen, halogen, or C₁₋₆ alkoxy. In some embodiments, R⁵ and R⁶ cannot both be hydrogen, and are each independently selected from the group consisting of hydrogen, fluorine, chlorine, bromine, iodine, methoxy, ethoxy, or propoxy. In some embodiments, R⁵ and R⁶ cannot both be hydrogen, and are each independently selected from the group consisting of hydrogen, fluorine, or methoxy.

In some embodiments, the compound represented by formula IA is selected from the group consisting of the compound represented by formula IIIA1, formula IIIA2, formula IIIA3, formula IIIA4, or formula IIIA5, or a pharmaceutically acceptable salt thereof, 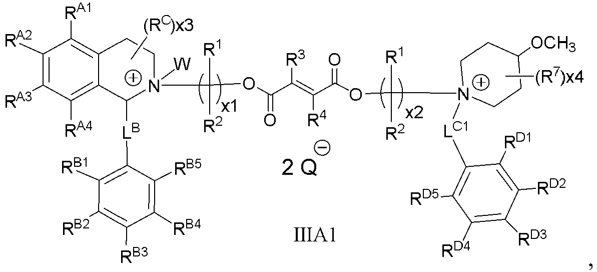 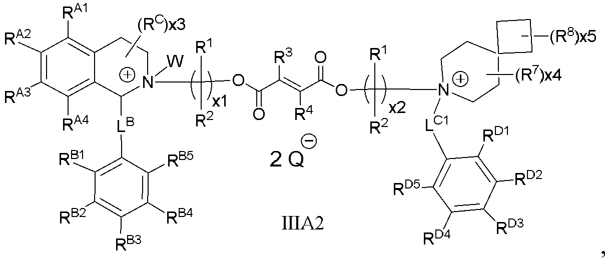 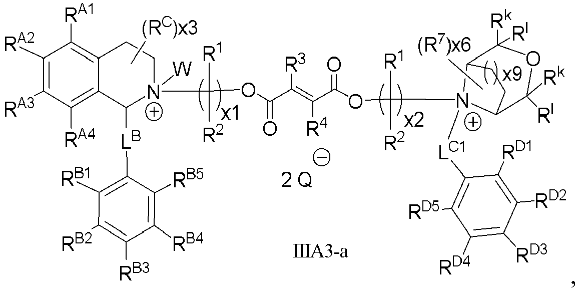 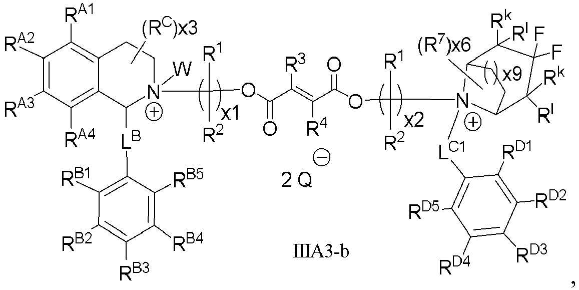 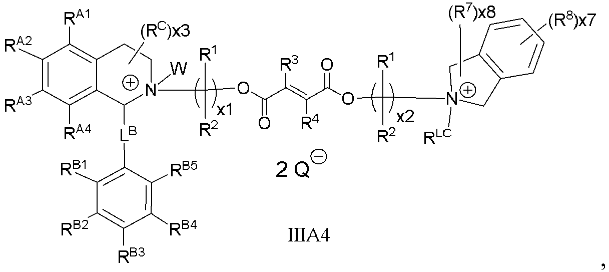 or 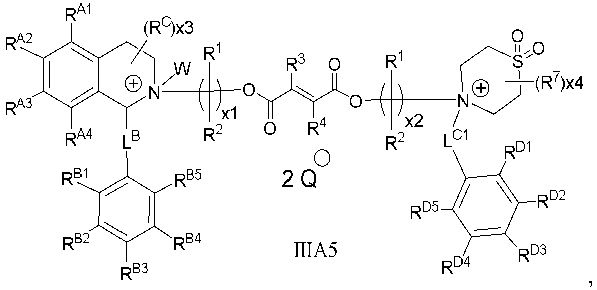 wherein
W, Q^{Θ}, L^{B}, R¹, R², R³, R⁴, R^{A1}, R^{A2}, R^{A3}, R^{A2}, R^{B1}, R^{B2}, R^{B3}, R^{B4}, R^{B5}, R^{C}, R^{D1}, R^{D2}, R^{D3}, R^{D4}, R^{D5}, x1, x2, x3, L^{C1}, R^{LC}, R⁷, R⁸, x4, x5, x6, x7, x8, x9, R^{k}, and R^{l} are as defined in the foregoing.

In some embodiments, each L^{B} is independently selected from the group consisting of a chemical bond or C₁₋₆ alkylene, preferably a chemical bond, methylene, ethylene, or propylene, preferably methylene.

In some embodiments, each L^{C1} is independently selected from the group consisting of a chemical bond or C₁₋₆ alkylene, preferably a chemical bond, methylene, ethylene, or propylene, preferably methylene.

In some embodiments, the compound represented by formula IA is selected from the group consisting of the compound represented by formula IVA1, formula IVA2, formula IVA3, formula IVA4, or formula IVA5, or a pharmaceutically acceptable salt thereof, 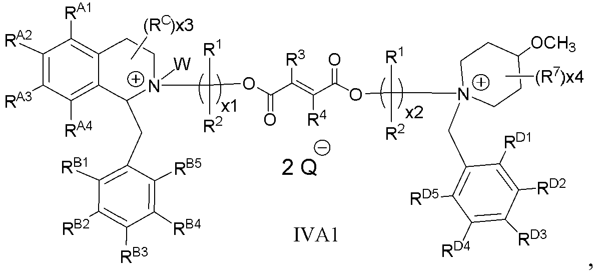 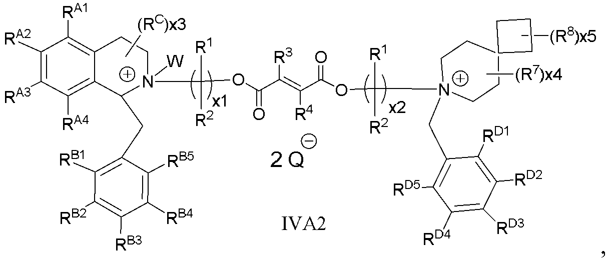 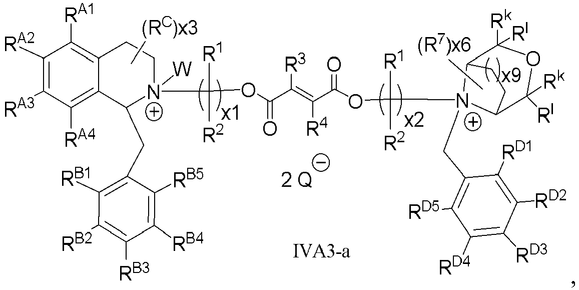 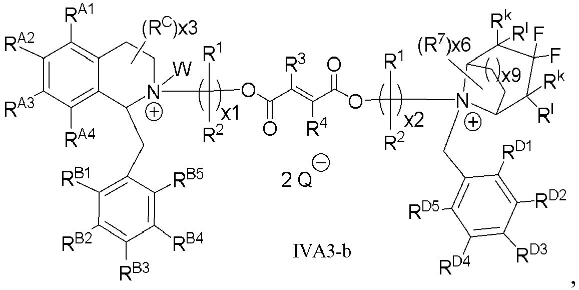 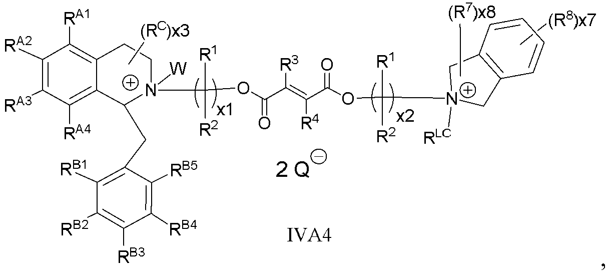 or 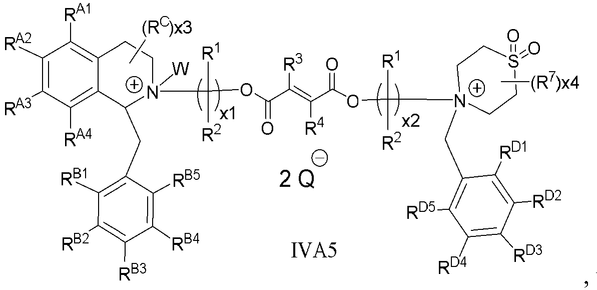 wherein
W, Q^{Θ}, R¹, R², R³, R⁴, R^{A1}, R^{A2}, R^{A3}, R^{A4}, R^{B1}, R^{B2}, R^{B3}, R^{B4}, R^{B5}, R^{C}, R^{D1}, R^{D2}, R^{D3}, R^{D4}, R^{D5}, x1, x2, x3, R^{LC}, R⁷, R⁸, x4, x5, x6, x7, x8, x9, R^{k}, and R^{l} are as defined in the foregoing.

In some embodiments, R^{A1}, R^{A4}, R^{B1}, R^{B2}, and R^{B5} are all hydrogen.

In some embodiments, R^{A2}, R^{A3}, R^{B3}, and R^{B4} are each independently selected from the group consisting of hydrogen or C₁₋₆ alkoxy. In some embodiments, R^{A2}, R^{A3}, R^{B3}, and R^{B4} are each independently selected from the group consisting of hydrogen, methoxy, ethoxy, or propoxy. In some embodiments, R^{A2}, R^{A3}, R^{B3}, and R^{B4} are each independently selected from the group consisting of hydrogen or methoxy.

In some embodiments, W is methyl.

In some embodiments, R^{D1}, R^{D4}, and R^{D5} are all hydrogen.

In some embodiments, R^{D2} and R^{D3} are each independently selected from the group consisting of hydrogen, halogen, -C(O)-C₁₋₆ alkyl, C₁₋₆ alkoxy, or methanesulfonyl. In some embodiments, R^{D2} and R^{D3} are each independently selected from the group consisting of hydrogen, fluorine, chlorine, bromine, iodine, -C(O)-methyl, -C(O)-ethyl, methoxy, ethoxy, or methanesulfonyl. In some embodiments, R^{D2} and R^{D3} are each independently selected from the group consisting of hydrogen, fluorine, bromine, -C(O)-ethyl, methoxy, or methanesulfonyl.

In some embodiments, the compound represented by formula IA or the pharmaceutically acceptable salt thereof is selected from the group consisting of the following compounds or pharmaceutically acceptable salts thereof, 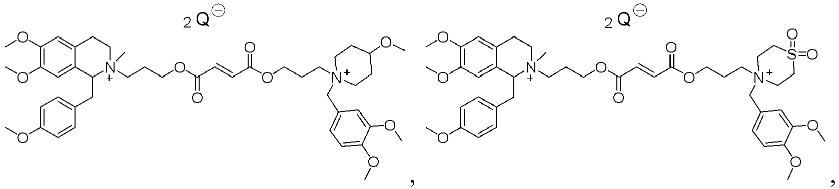 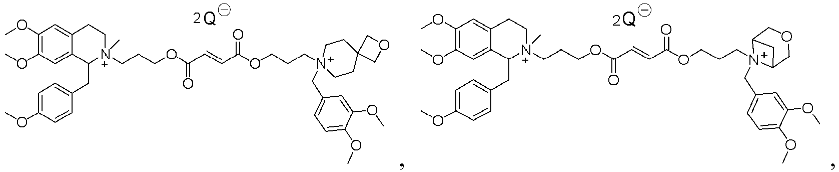 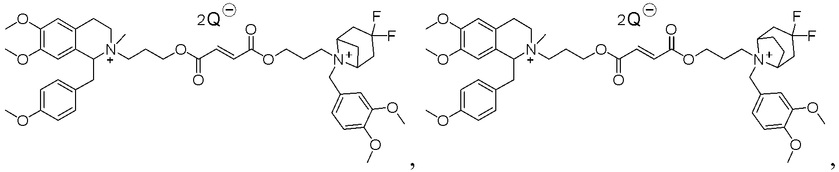 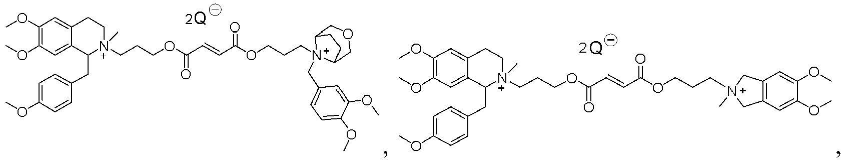 or 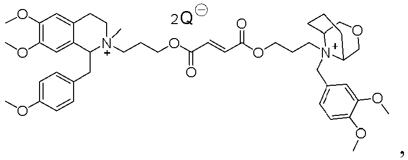 wherein Q^{Θ} is as defined in the foregoing.

In some embodiments, the compound represented by formula IA or the pharmaceutically acceptable salt thereof is selected from the group consisting of the following compounds or pharmaceutically acceptable salts thereof, 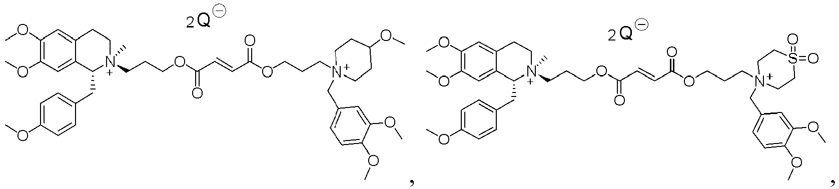 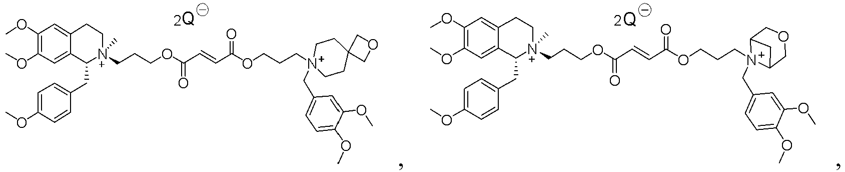 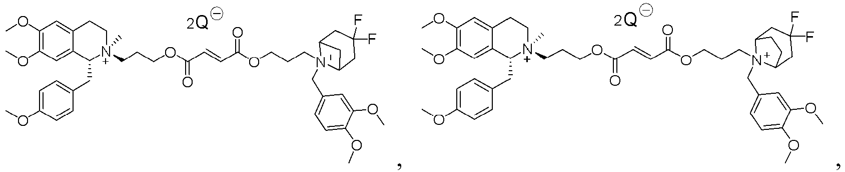 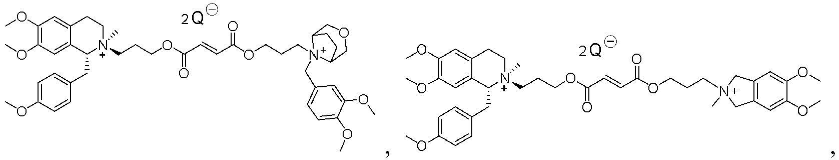 or 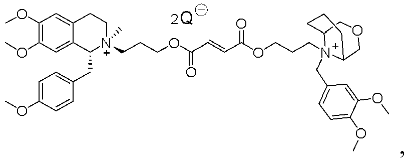 wherein Q^{Θ} is as defined in the foregoing.

In some embodiments, each Q^{Θ} is independently selected from the group consisting of halide ions, acetate, formate, benzoate, benzenesulfonate, camphorsulfonate, citrate, ethanedisulfonate, fumarate, glucoheptonate, gluconate, glucuronate, isethionate, lactate, lactobionate, lauryl sulfate, malate, maleate, methanesulfonate, naphthoate, naphthalenesulfonate, nitrate, stearate, oleate, oxalate, pamoate, phosphate, hydrogen phosphate, dihydrogen phosphate, polygalacturonate, succinate, sulfate, sulfosalicylate, tartrate, toluenesulfonate, or trifluoroacetate. In some embodiments, each Q^{Θ} is independently selected from the group consisting of chloride ion, bromide ion, fluoride ion, iodide ion, trifluoroacetate, formate, methanesulfonate, or benzenesulfonate. In some embodiments, each Q^{Θ} is independently selected from the group consisting of chloride ion, bromide ion, trifluoroacetate, formate, methanesulfonate, or benzenesulfonate.

In some embodiments, the compound represented by formula IA or the pharmaceutically acceptable salt thereof is selected from the group consisting of the following compounds or pharmaceutically acceptable salts thereof,

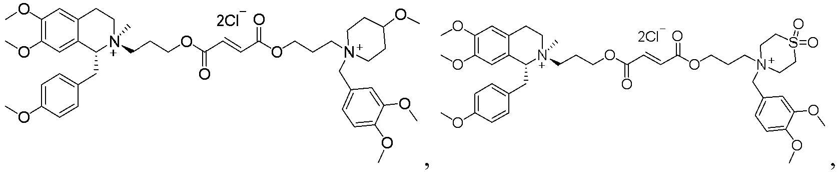

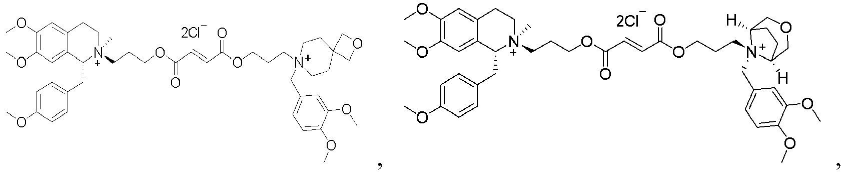

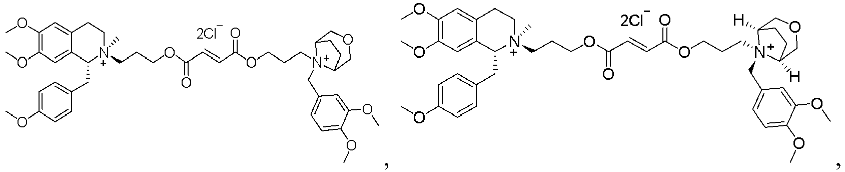

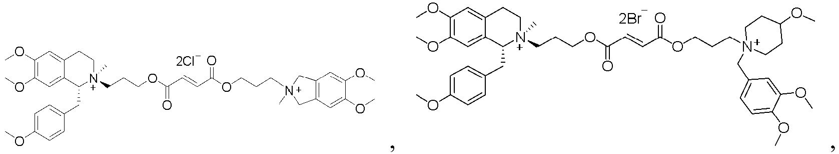

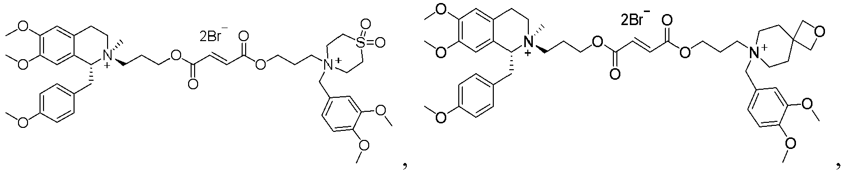

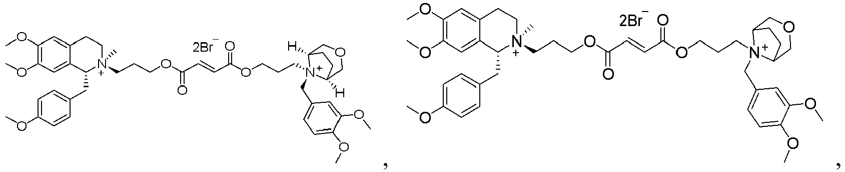

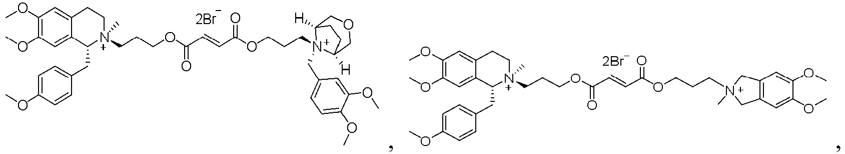

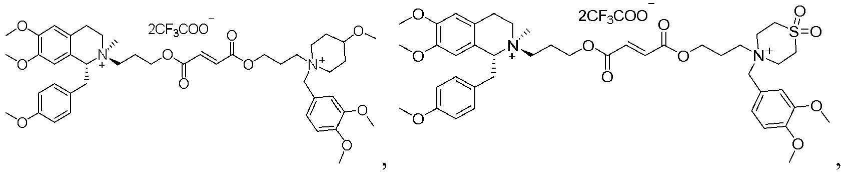

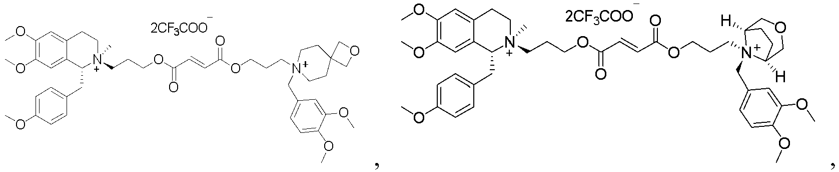

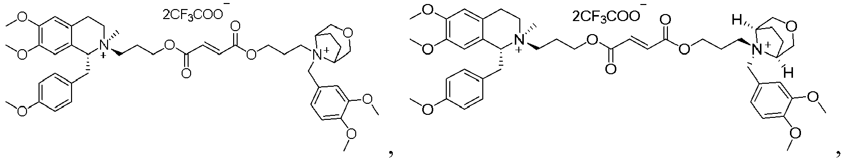

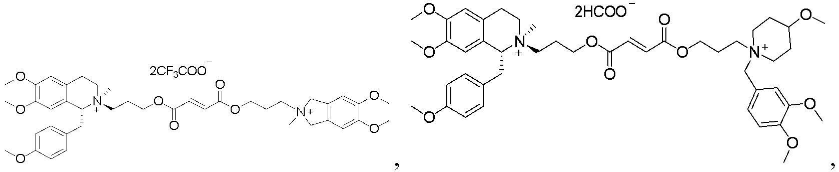

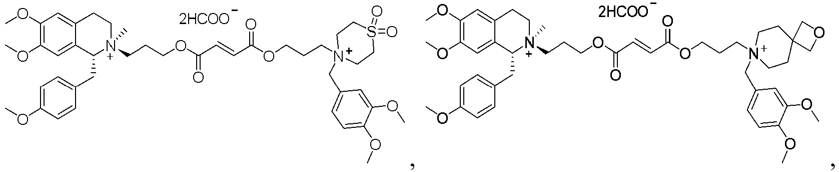

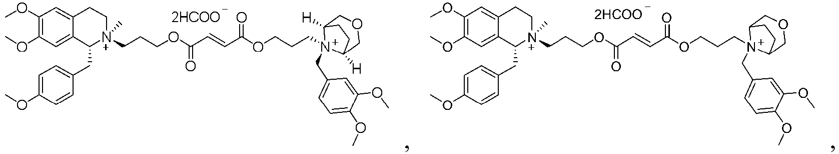

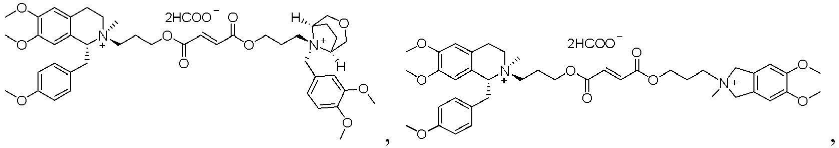

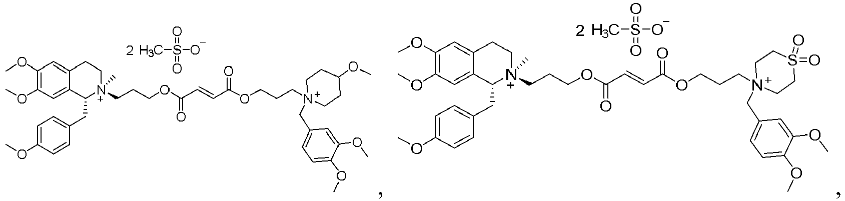

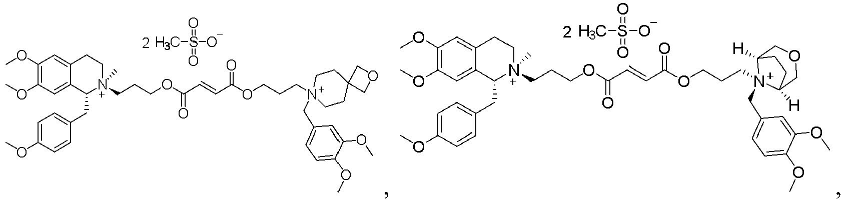

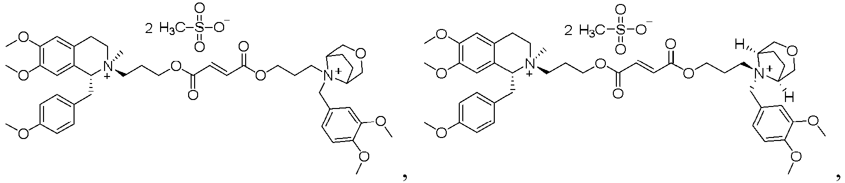

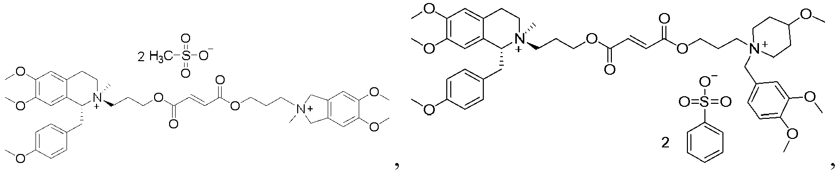

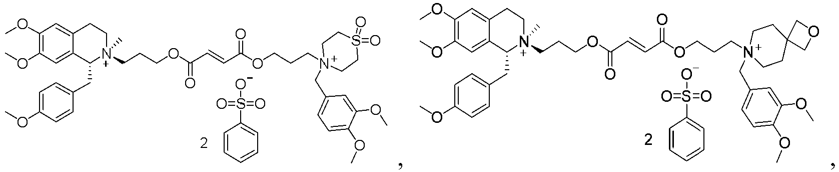

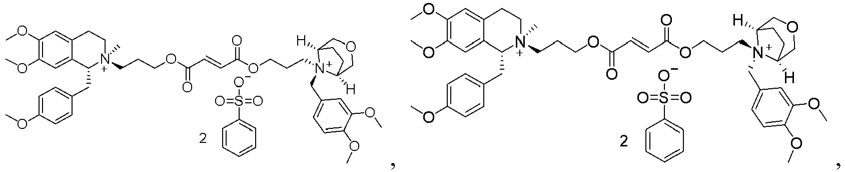

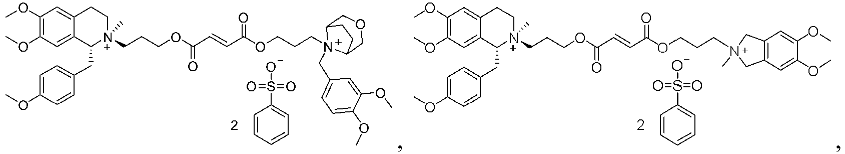

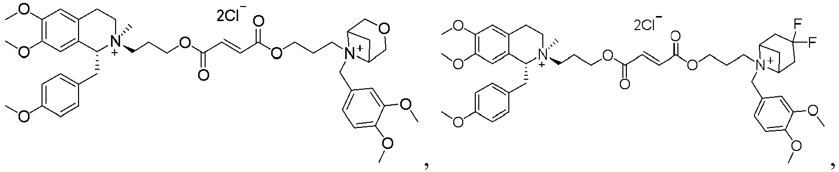

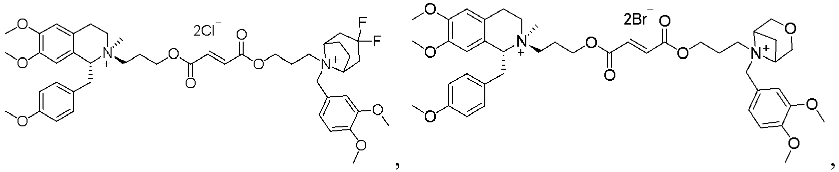

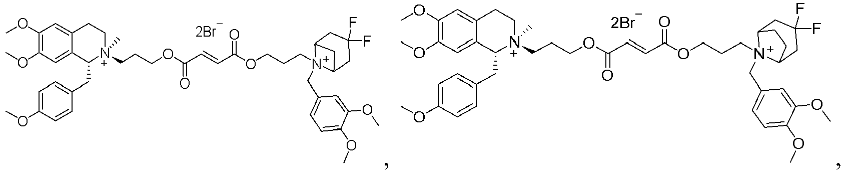

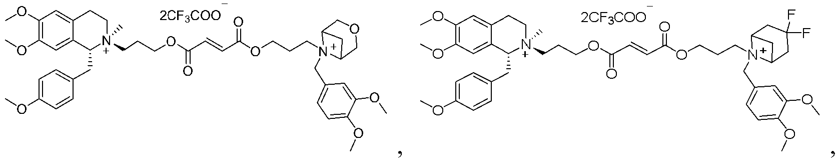

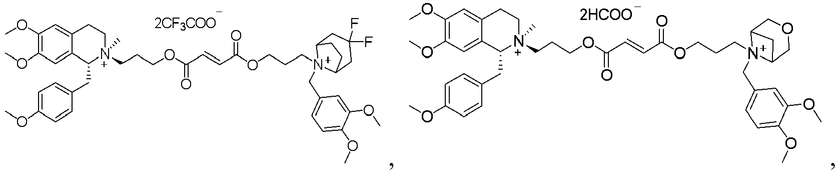

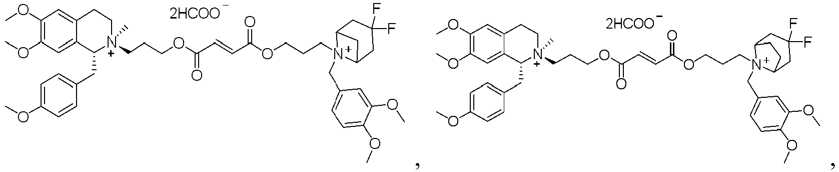

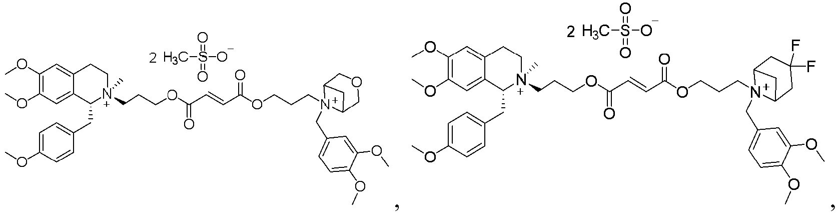

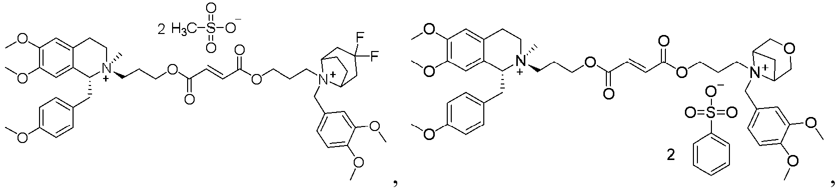

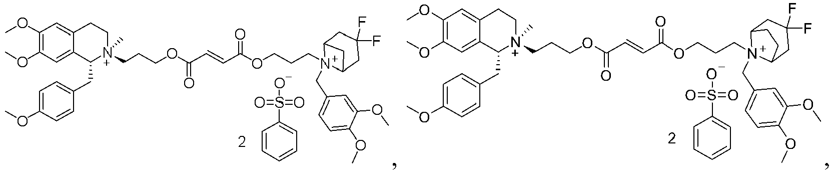

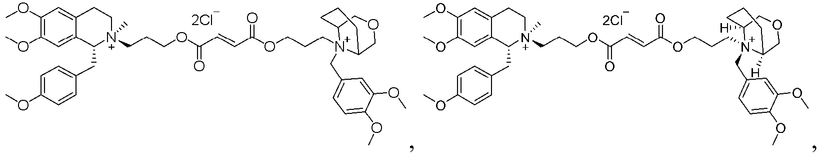

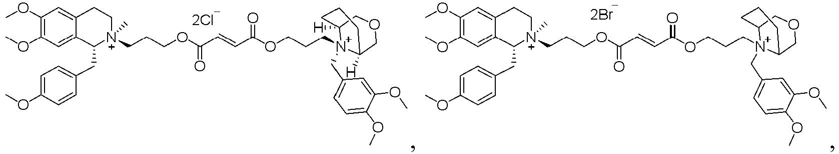

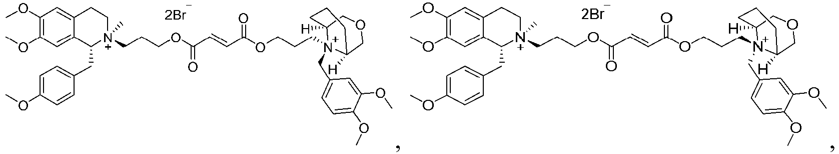

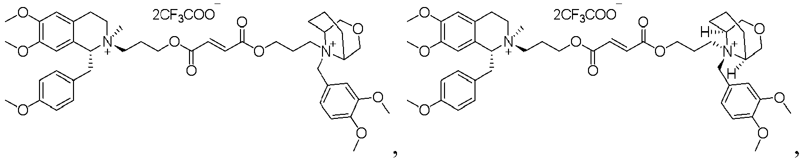

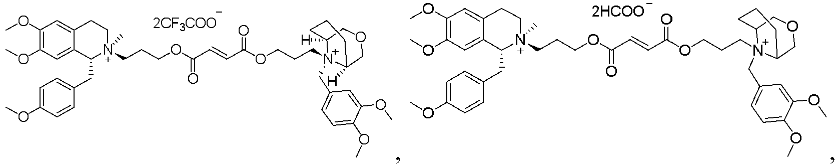

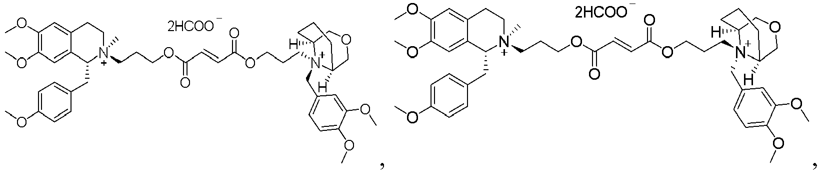

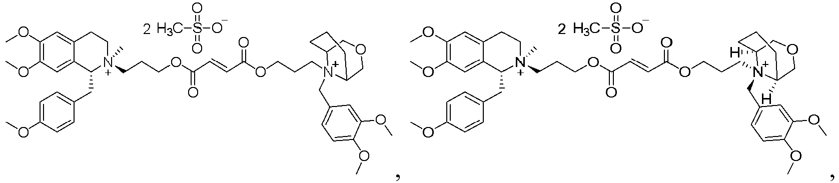

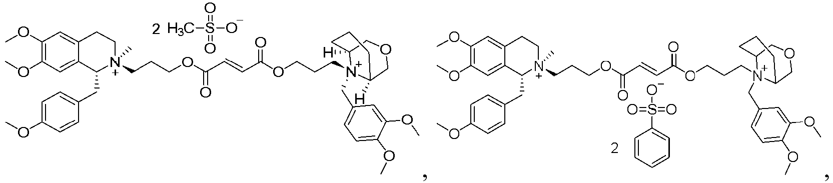

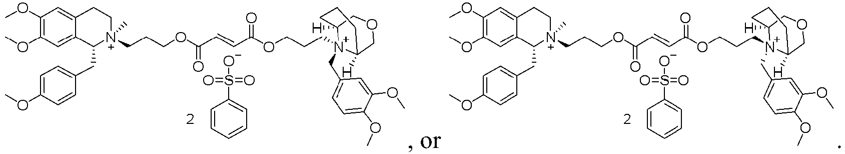

In another aspect, the present invention also provides a compound represented by formula IB or a pharmaceutically acceptable salt thereof, 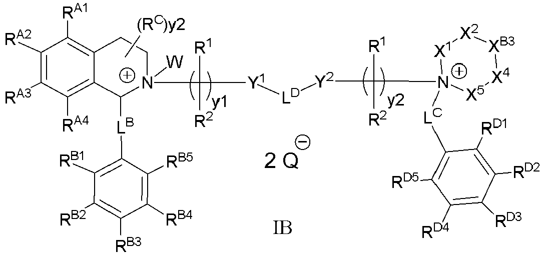 wherein:
X^{B3} is selected from the group consisting of a chemical bond, -O-, -C(R^{m}Rⁿ)-, or -N(R^{f})-;
Y¹ and Y² are absent, or are each independently selected from the group consisting of -O-, -N(R^{f})-, or -S-;
when Y¹ and Y² are both -O-, L^{D} is 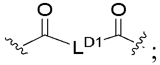
when Y¹ and Y² are both absent, L^{D} is 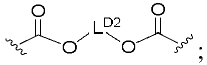
L^{D1} and L^{D2} are each independently selected from the group consisting of alkylene or heteroalkylene, wherein the alkylene or heteroalkylene is optionally substituted with one or more halogen, hydroxy, mercapto, nitro, carboxy, amino, cyano, C₁₋₆ alkyl, C₁₋₆ alkoxy, oxo, thioxo, or sulfonyl, and the C₁₋₆ alkyl or C₁₋₆ alkoxy is optionally substituted with one or more halogen, hydroxy, mercapto, carboxy, amino, or cyano;
R^{m} and Rⁿ are each independently selected from the group consisting of hydrogen, halogen, hydroxy, mercapto, carboxy, amino, cyano, C₁₋₆ alkyl, C₁₋₆ alkoxy, -C(O)-C₁₋₆ alkyl, -C(O)-C₁₋₆ alkoxy, methanesulfonyl, a 3- to 10-membered carbocyclic ring, a 3- to 10-membered heterocyclic ring, a 6- to 10-membered aromatic ring, or a 5- to 10-membered heteroaromatic ring, wherein the alkyl, alkoxy, carbocyclic ring, heterocyclic ring, aromatic ring, or heteroaromatic ring is optionally substituted with one or more halogen, hydroxy, mercapto, carboxy, amino, or cyano,
or alternatively, R^{m} and Rⁿ together with the atoms to which they are attached form oxo, thioxo, a 3- to 10-membered carbocyclic ring, a 3- to 10-membered heterocyclic ring, a 6- to 10-membered aromatic ring, or a 5- to 10-membered heteroaromatic ring, wherein the carbocyclic ring, heterocyclic ring, aromatic ring, or heteroaromatic ring is optionally substituted with one or more halogen, hydroxy, mercapto, carboxy, amino, cyano, C₁₋₆ alkyl, or C₁₋₆ alkoxy, and the alkyl or alkoxy is optionally substituted with one or more halogen, hydroxy, mercapto, carboxy, amino, or cyano;
y1 is selected from the group consisting of 1, 2, 3, 4, 5, or 6;
y2 is selected from the group consisting of 1, 2, 3, 4, 5, or 6;
y3 is selected from the group consisting of 0, 1, 2, 3, 4, or 5; and
W, Q^{Θ}, L^{B}, L^{C}, R¹, R², R^{A1}, R^{A2}, R^{A3}, R^{A2}, R^{B1}, R^{B2}, R^{B3}, R^{B4}, R^{B5}, R^{C}, R^{D1}, R^{D2}, R^{D3}, R^{D4}, R^{D5}, X¹, X², X⁴, X⁵, and R^{f} are as defined in the foregoing.

In some embodiments, X^{B3} is -O-.

In some embodiments, the compound represented by formula IB is selected from the group consisting of the compound represented by formula IIB1 or formula IIB2, or a pharmaceutically acceptable salt thereof, 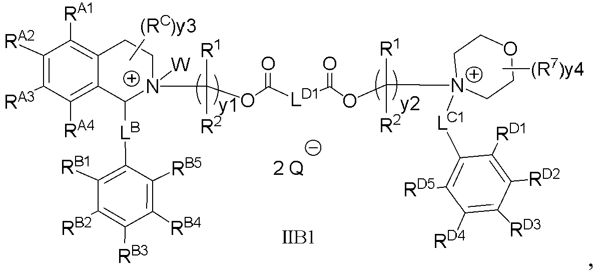 or 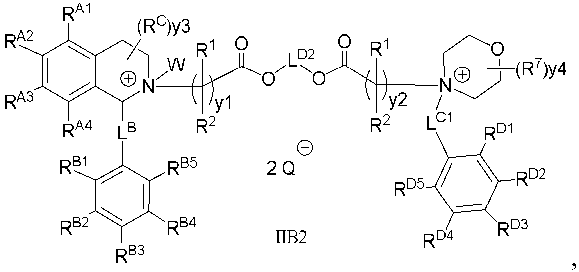 wherein
each y4 is independently selected from the group consisting of 0, 1, 2, 3, 4, 5, 6, 7, or 8; and
W, Q^{Θ}, L^{B}, R¹, R², R^{A1}, R^{A2}, R^{A3}, R^{A2}, R^{B1}, R^{B2}, R^{B3}, R^{B4}, R^{B5}, R^{C}, R^{D1}, R^{D2}, R^{D3}, R^{D4}, R^{D5}, L^{C1}, R⁷, L^{D1}, L^{D2}, y1, y2, and y3 are as defined in the foregoing.

In some embodiments, 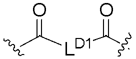 is selected from the group consisting of: 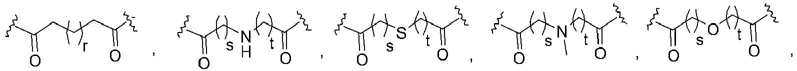 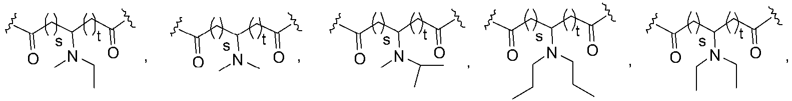 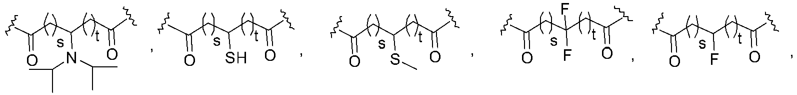 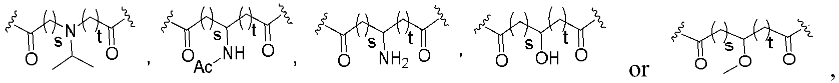 wherein
each r is independently selected from the group consisting of 0, 1, 2, 3, or 4;
each s is independently selected from the group consisting of 0, 1, 2, 3, or 4; and
each t is independently selected from the group consisting of 0, 1, 2, 3, or 4.

In some embodiments, 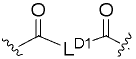 is selected from the group consisting of 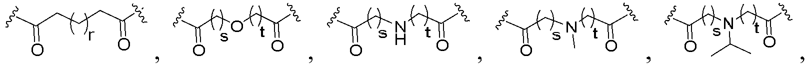 or 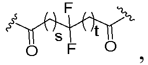 wherein r, s, and t are as defined in the foregoing.

In some embodiments, 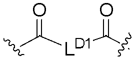 is selected from the group consisting of 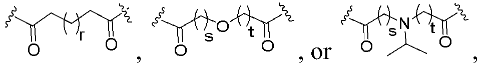 wherein r, s, and t are as defined in the foregoing.

In some embodiments, L^{D2} is -(CR^{m}Rⁿ)_{d}-, wherein d is selected from the group consisting of 1, 2, 3, 4, 5, or 6, and R^{m} and Rⁿ are as defined in the foregoing.

In some embodiments, the compound represented by formula IB is selected from the group consisting of the compound represented by formula IIIB 1-a, formula IIIB1-b, or formula IIIB2, or a pharmaceutically acceptable salt thereof, 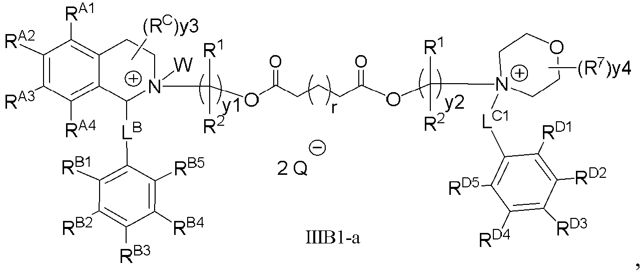 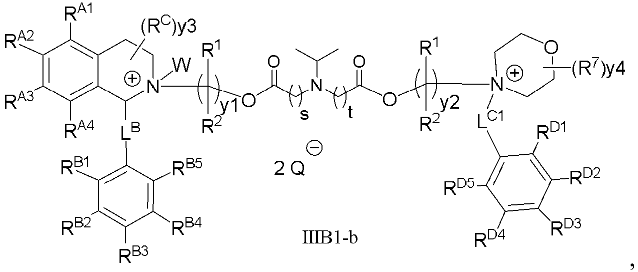 or 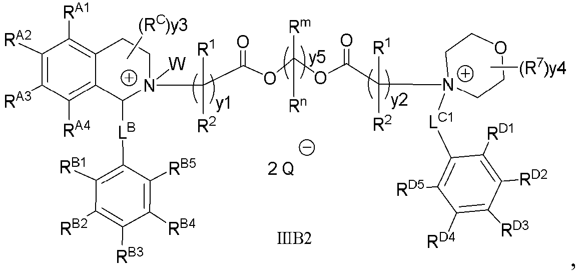 wherein
each y4 is independently selected from the group consisting of 0, 1, 2, 3, 4, 5, 6, 7, or 8;
y5 is selected from the group consisting of 1, 2, 3, 4, 5, or 6; and
W, Q^{Θ}, L^{B}, R¹, R², R^{A1}, R^{A2}, R^{A3}, R^{A2}, R^{B1}, R^{B4}, R^{B3}, R^{B4}, R^{B5}, R^{C}, R^{D1}, R^{D2}, R^{D3}, R^{D4}, R^{D5}, L^{C1}, R⁷, R^{m}, Rⁿ, y1, y2, y3, r, s, and t are as defined in the foregoing.

In some embodiments, the compound represented by formula IB is selected from the group consisting of the compound represented by formula IVB1-a, formula IVB1-b, or formula IVB2, or a pharmaceutically acceptable salt thereof, 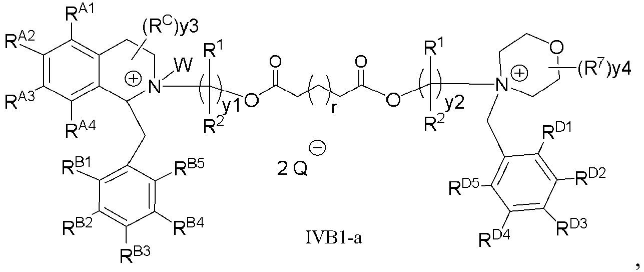 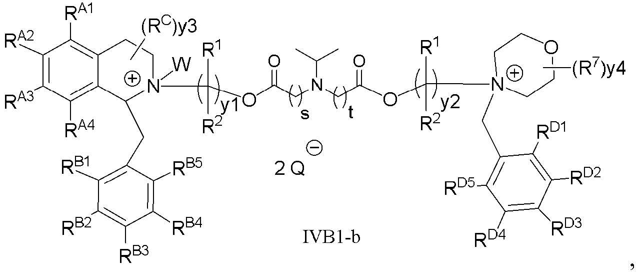 or 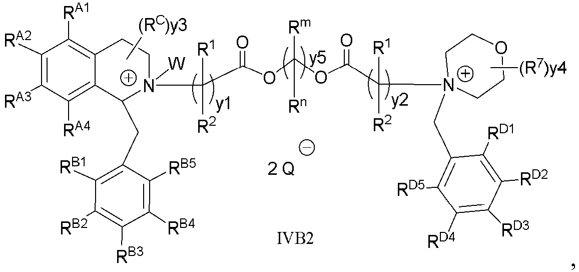 wherein
W, Q^{Θ}, R¹, R², R^{A1}, R^{A2}, R^{A3}, R^{A2}, R^{B1}, R^{B2}, R^{B3}, R^{B4}, R^{B5}, R^{C}, R^{D1}, R^{D2}, R^{D3}, R^{D4}, R^{D5}, R⁷, R^{m}, Rⁿ, y1, y2, y3, r, s, t, y4, and y5 are as defined in the foregoing.

In some embodiments, the compound represented by formula IB or the pharmaceutically acceptable salt thereof is selected from the group consisting of the following compounds or pharmaceutically acceptable salts thereof, 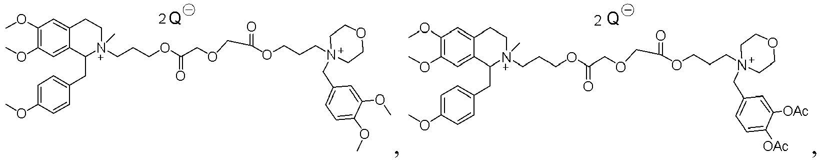 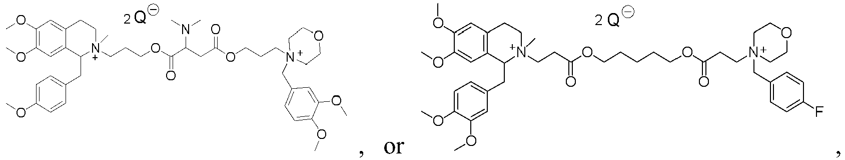 wherein
Q^{Θ} is as defined in the foregoing.

In some embodiments, the compound represented by formula IB or the pharmaceutically acceptable salt thereof is selected from the group consisting of the following compounds or pharmaceutically acceptable salts thereof, 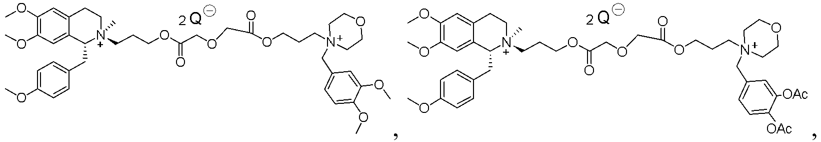 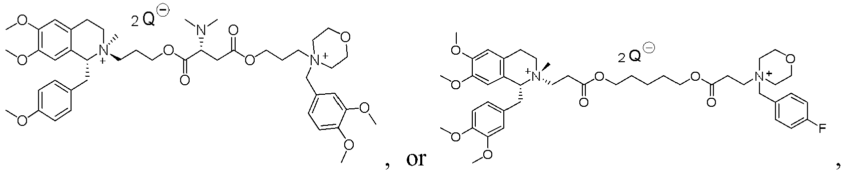 wherein
Q^{Θ} is as defined in the foregoing.

In some embodiments, the compound represented by formula IB or the pharmaceutically acceptable salt thereof is selected from the group consisting of the following compounds or pharmaceutically acceptable salts thereof,

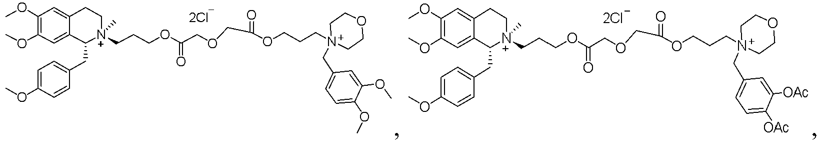

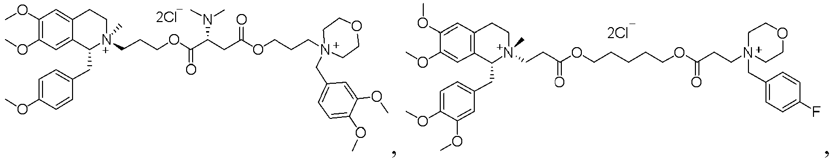

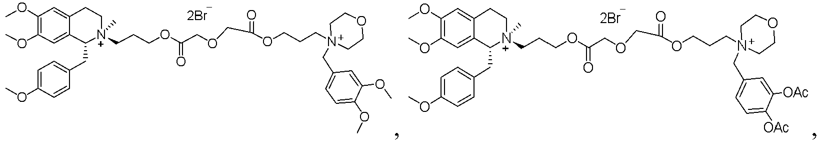

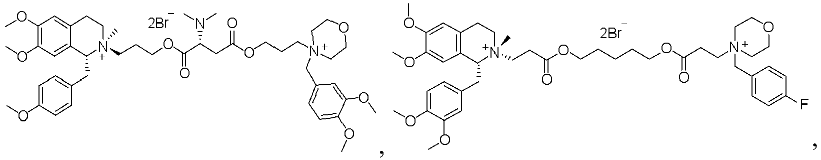

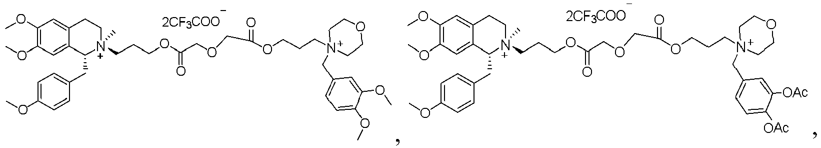

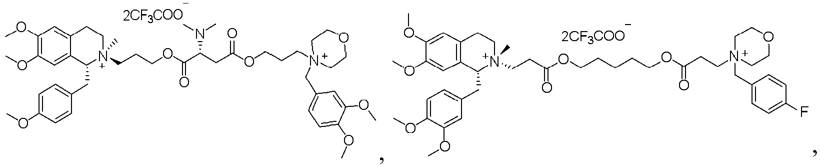

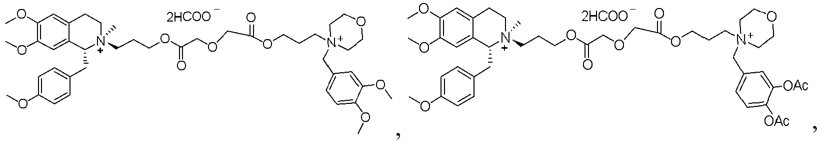

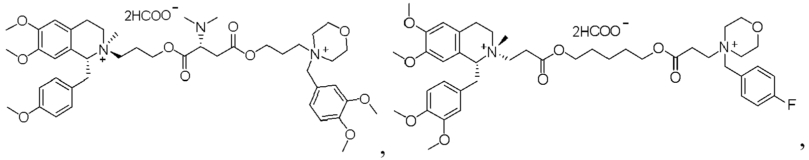

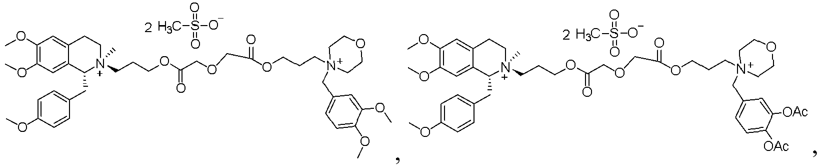

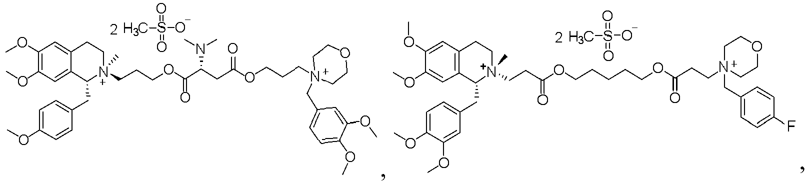

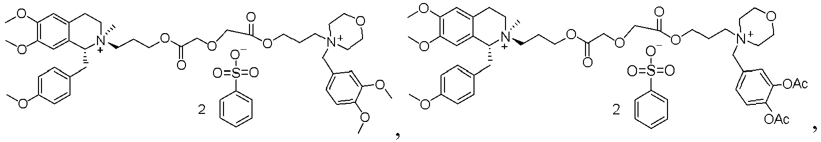

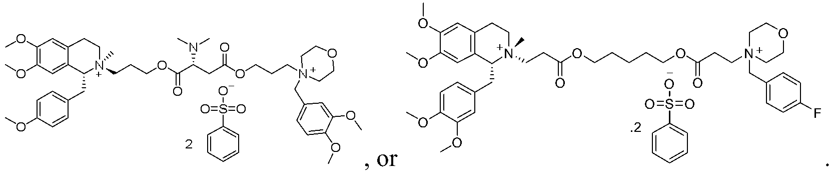

In another aspect, the present invention also provides a compound represented by formula IC or a pharmaceutically acceptable salt thereof, 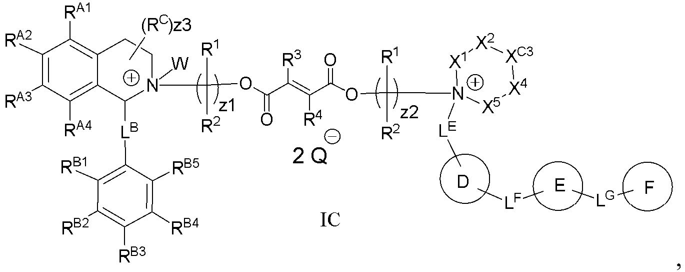 wherein:
X^{C3} is selected from the group consisting of a chemical bond, -O-, -C(R^{p}R^{q})-, or -N(R^{f})-;
L^{E} and L^{F} are each independently selected from the group consisting of - (CR^{p}R^{q})ᵢ-, -(CR^{p}R^{q})ₑ-O-(CR^{p}R^{q})_{f}-, -(CR^{p}R^{q})ₑ-S-(CR^{p}R^{q})_{f}-, -(CR^{p}R^{q})ₑ-N(R^{f})-(CR^{p}R^{q})_{f}-, or -(CR^{p}R^{q})ₑ-C(O)-N(R^{f})-(CR^{p}R^{q})_{f}-;
R^{p} and R^{q} are each independently selected from the group consisting of hydrogen, halogen, hydroxy, mercapto, carboxy, amino, cyano, C₁₋₆ alkyl, C₁₋₆ alkoxy, -C(O)-C₁₋₆ alkyl, -C(O)-C₁₋₆ alkoxy, methanesulfonyl, a 3- to 10-membered carbocyclic ring, a 3- to 10-membered heterocyclic ring, a 6- to 10-membered aromatic ring, or a 5- to 10-membered heteroaromatic ring, wherein the alkyl, alkoxy, carbocyclic ring, heterocyclic ring, aromatic ring, or heteroaromatic ring is optionally substituted with one or more halogen, hydroxy, mercapto, carboxy, amino, or cyano,
or alternatively, R^{p} and R^{q} together with the atoms to which they are attached form oxo, thioxo, a 3- to 10-membered carbocyclic ring, a 3- to 10-membered heterocyclic ring, a 6- to 10-membered aromatic ring, or a 5- to 10-membered heteroaromatic ring, wherein the carbocyclic ring, heterocyclic ring, aromatic ring, or heteroaromatic ring is optionally substituted with one or more halogen, hydroxy, mercapto, carboxy, amino, cyano, C₁₋₆ alkyl, or C₁₋₆ alkoxy, and the alkyl or alkoxy is optionally substituted with one or more halogen, hydroxy, mercapto, carboxy, amino, or cyano;
ring D is selected from the group consisting of a 6- to 10-membered arylene group or a 5- to 10-membered heteroarylene group, wherein the arylene group or heteroarylene group is optionally substituted with one or more halogen, hydroxy, mercapto, carboxy, amino, cyano, oxo, thioxo, C₁₋₆ alkyl, or C₁₋₆ alkoxy, and the alkyl or alkoxy is optionally substituted with one or more halogen, hydroxy, mercapto, carboxy, amino, or cyano;
when L^{G} and ring F are both absent,
ring E is selected from the group consisting of a 3- to 10-membered carbocyclic ring, a 3- to 10-membered heterocyclic ring, a 6- to 10-membered aromatic ring, or a 5- to 10-membered heteroaromatic ring, wherein the carbocyclic ring, heterocyclic ring, aromatic ring, or heteroaromatic ring is optionally substituted with one or more halogen, hydroxy, mercapto, carboxy, amino, cyano, oxo, thioxo, C₁₋₆ alkyl, or C₁₋₆ alkoxy, and the alkyl or alkoxy is optionally substituted with one or more halogen, hydroxy, mercapto, carboxy, amino, or cyano;
when L^{G} and ring F are both present,
ring E is selected from the group consisting of a 3- to 10-membered cycloalkylene group, a 3- to 10-membered heterocyclylene group, a 6- to 10-membered arylene group, or a 5- to 10-membered heteroarylene group, wherein the alkylene group, heterocyclylene group, arylene group, or heteroarylene group is optionally substituted with one or more halogen, hydroxy, mercapto, carboxy, amino, cyano, oxo, thioxo, C₁₋₆ alkyl, or C₁₋₆ alkoxy, and the alkyl or alkoxy is optionally substituted with one or more halogen, hydroxy, mercapto, carboxy, amino, or cyano,
L^{G} is selected from the group consisting of -(CR^{p}R^{q})ᵢ-, -(CR^{p}R^{q})ₑ-O-(CR^{p}R^{q})_{f}-, -(CR^{p}R^{q})ₑ-S-(CR^{p}R^{q})_{f}-, -(CR^{p}R^{q})ₑ-N(R^{f})-(CR^{p}R^{g})_{f}-, or -(CR^{p}R^{q})ₑ-C(O)-N(R^{f})-(CR^{p}R^{q})_{f}-,
ring F is selected from the group consisting of a 3- to 10-membered carbocyclic ring, a 3- to 10-membered heterocyclic ring, a 6- to 10-membered aromatic ring, or a 5- to 10-membered heteroaromatic ring, wherein the carbocyclic ring, heterocyclic ring, aromatic ring, or heteroaromatic ring is optionally substituted with one or more halogen, hydroxy, mercapto, carboxy, amino, cyano, oxo, thioxo, C₁₋₆ alkyl, or C₁₋₆ alkoxy, and the alkyl or alkoxy is optionally substituted with one or more halogen, hydroxy, mercapto, carboxy, amino, or cyano;
z1 is selected from the group consisting of 1, 2, 3, 4, 5, or 6;
z2 is selected from the group consisting of 1, 2, 3, 4, 5, or 6;
z3 is selected from the group consisting of 0, 1, 2, 3, 4, or 5;
each e is independently selected from the group consisting of 0, 1, 2, 3, 4, 5, or 6;
each f is independently selected from the group consisting of 0, 1, 2, 3, 4, 5, or 6;
each i is independently selected from the group consisting of 0, 1, 2, 3, 4, 5, or 6; and
W, Q^{Θ}, L^{B}, R¹, R², R³, R⁴, R^{A1}, R^{A2}, R^{A3}, R^{A2}, R^{B1}, R^{B2}, R^{B3}, R^{B4}, R^{B5}, R^{C}, R^{f}, X¹, X², X⁴, and X⁵ are as defined in the foregoing.

In some embodiments, X^{C3} is -O-.

In some embodiments, ring D is selected from the group consisting of: 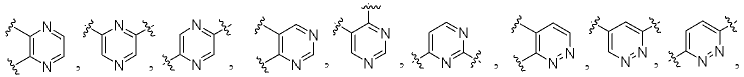 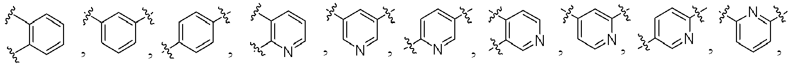 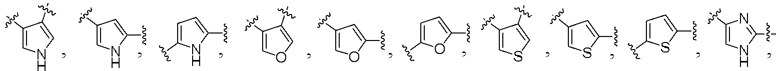 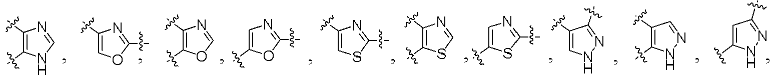 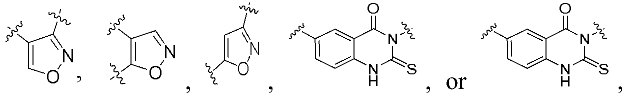 and the ring D is optionally substituted with one or more halogen, hydroxy, mercapto, carboxy, amino, cyano, oxo, thioxo, C₁₋₆ alkyl, or C₁₋₆ alkoxy, wherein the alkyl or alkoxy is optionally substituted with one or more halogen, hydroxy, mercapto, carboxy, amino, or cyano.

In some embodiments, ring D is selected from the group consisting of: 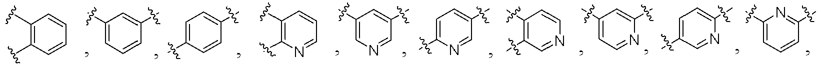 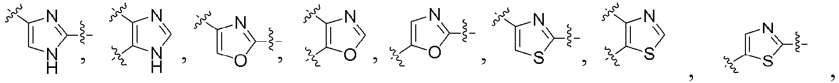 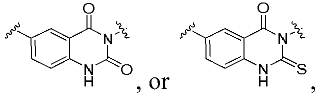 and the ring D is optionally substituted with one or more halogen, hydroxy, mercapto, carboxy, amino, cyano, oxo, thioxo, C₁₋₆ alkyl, or C₁₋₆ alkoxy, wherein the alkyl or alkoxy is optionally substituted with one or more halogen, hydroxy, mercapto, carboxy, amino, or cyano.

In some embodiments, ring D is selected from the group consisting of 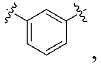 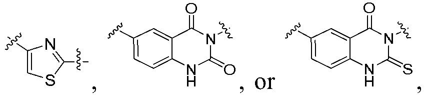 and the ring D is optionally substituted with one or more halogen, hydroxy, mercapto, carboxy, amino, cyano, oxo, thioxo, C₁₋₆ alkyl, or C₁₋₆ alkoxy, wherein the alkyl or alkoxy is optionally substituted with one or more halogen, hydroxy, mercapto, carboxy, amino, or cyano.

In some embodiments, when L^{G} and ring F are both present, ring E is selected from the group consisting of: 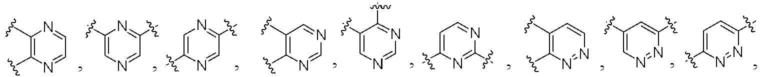 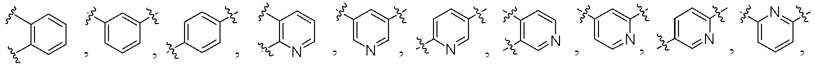 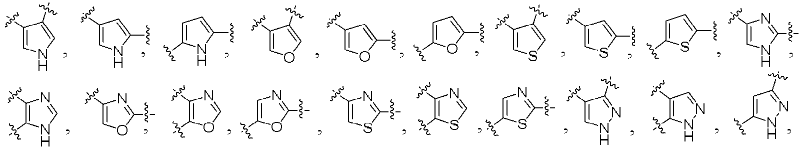 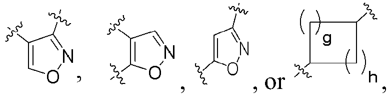 wherein
g is selected from the group consisting of 0, 1, 2, or 3; and
h is selected from the group consisting of 1, 2, 3, 4, or 5; and
the ring E is optionally substituted with one or more halogen, hydroxy, mercapto, carboxy, amino, cyano, oxo, thioxo, C₁₋₆ alkyl, or C₁₋₆ alkoxy, wherein the alkyl or alkoxy is optionally substituted with one or more halogen, hydroxy, mercapto, carboxy, amino, or cyano.

In some embodiments, when L^{G} and ring F are both present, ring E is selected from the group consisting of: 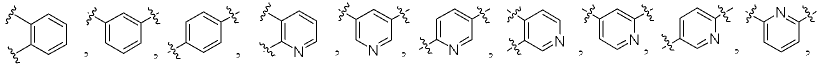 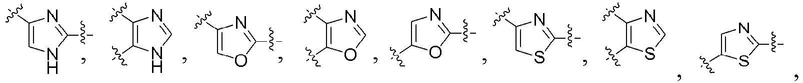 or 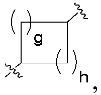 the ring E is optionally substituted with one or more halogen, hydroxy, mercapto, carboxy, amino, cyano, oxo, thioxo, C₁₋₆ alkyl, or C₁₋₆ alkoxy, wherein the alkyl or alkoxy is optionally substituted with one or more halogen, hydroxy, mercapto, carboxy, amino, or cyano, and g and h are as defined in the foregoing.

In some embodiments, when L^{G} and ring F are both present, ring E is selected from the group consisting of 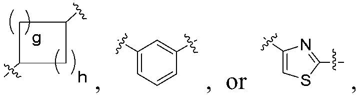 the ring E is optionally substituted with one or more halogen, hydroxy, mercapto, carboxy, amino, cyano, oxo, thioxo, C₁₋₆ alkyl, or C₁₋₆ alkoxy, wherein the alkyl or alkoxy is optionally substituted with one or more halogen, hydroxy, mercapto, carboxy, amino, or cyano, and g and h are as defined in the foregoing.

In some embodiments, when L^{G} and ring F are both present, ring F is selected from the group consisting of a benzene ring, a pyridine ring, a thiazine ring, a pyrimidine ring, a pyridazine ring, a furan ring, a pyrrole ring, a thiophene ring, an imidazole ring, a pyrazole ring, an oxazole ring, an isoxazole ring, a thiazole ring, a benzofuran ring, an isobenzofuran ring, an isoindole ring, an indole ring, a benzothiophene ring, a benzimidazole ring, indazole, benzoxazole, benzisoxazole, purine, a benzothiazole ring, a quinoline ring, or an isoquinoline ring, and the ring F is optionally substituted with one or more halogen, hydroxy, mercapto, carboxy, amino, cyano, oxo, thioxo, C₁₋₆ alkyl, or C₁₋₆ alkoxy, wherein the alkyl or alkoxy is optionally substituted with one or more halogen, hydroxy, mercapto, carboxy, amino, or cyano. In some embodiments, when LG and ring F are both present, ring F is selected from the group consisting of a benzene ring, a pyridine ring, an imidazole ring, a pyrazole ring, an oxazole ring, an isoxazole ring, a thiazole ring, a quinoline ring, or an isoquinoline ring, and the ring F is optionally substituted with one or more halogen, hydroxy, mercapto, carboxy, amino, cyano, oxo, thioxo, C₁₋₆ alkyl, or C₁₋₆ alkoxy, wherein the alkyl or alkoxy is optionally substituted with one or more halogen, hydroxy, mercapto, carboxy, amino, or cyano.

In some embodiments, when L^{G} and ring F are both present, ring F is selected from the group consisting of a benzene ring, a pyridine ring, a quinoline ring, or an isoquinoline ring, and the ring F is optionally substituted with one or more halogen, hydroxy, mercapto, carboxy, amino, cyano, oxo, thioxo, C₁₋₆ alkyl, or C₁₋₆ alkoxy, wherein the alkyl or alkoxy is optionally substituted with one or more halogen, hydroxy, mercapto, carboxy, amino, or cyano.

In some embodiments, when L^{G} and ring F are both present, L^{G} is selected from the group consisting of -(CR^{p}R^{q})ₑ-O-(CR^{p}R^{q})_{f}-, -(CR^{p}R^{q})ₑ-S-(CR^{p}R^{q})_{f}-, -(CR^{p}R^{q})ₑ-N(R^{f})-(CR^{p}R^{q})_{f}-, or -(CR^{p}R^{q})ₑ-C(O)-N(R^{f})-(CR^{p}R^{q})_{f}-, wherein R^{f}, R^{p}, R^{q}, e, and f are as defined in the foregoing.

In some embodiments, when L^{G} and ring F are both present, L^{G} is selected from the group consisting of -(CR^{p}R^{q})ₑ-N(R^{f})-(CR^{p}R^{q})_{f}- or -(CR^{p}R^{q})ₑ-C(O)-N(R^{f})-(CR^{p}R^{q})_{f}-, wherein R^{f}, R^{p}, R^{q}, e, and f are as defined in the foregoing.

In some embodiments, when L^{G} and ring F are both present, L^{G} is -(CR^{p}R^{q})ₑ-N(R^{f})-(CR^{p}R^{q})_{f}-, wherein R^{f}, R^{p}, R^{q}, e, and f are as defined in the foregoing.

In some embodiments, when L^{G} and ring F are both absent, ring E is selected from the group consisting of a benzene ring, a pyridine ring, a thiazine ring, a pyrimidine ring, a pyridazine ring, a furan ring, a pyrrole ring, a thiophene ring, an imidazole ring, a pyrazole ring, an oxazole ring, an isoxazole ring, a thiazole ring, a benzofuran ring, an isobenzofuran ring, an isoindole ring, an indole ring, a benzothiophene ring, a benzimidazole ring, an indazole ring, a benzoxazole ring, a benzisoxazole ring, a purine ring, a benzothiazole ring, or a quinazoline ring, and the ring E is optionally substituted with one or more halogen, hydroxy, mercapto, carboxy, amino, cyano, oxo, thioxo, C₁₋₆ alkyl, or C₁₋₆ alkoxy, wherein the alkyl or alkoxy is optionally substituted with one or more halogen, hydroxy, mercapto, carboxy, amino, or cyano.

In some embodiments, when L^{G} and ring F are both absent, ring E is selected from the group consisting of a benzene ring, a pyridine ring, an imidazole ring, a pyrazole ring, an oxazole ring, an isoxazole ring, a thiazole ring, or a quinazoline ring, and the ring E is optionally substituted with one or more halogen, hydroxy, mercapto, carboxy, amino, cyano, oxo, thioxo, C₁₋₆ alkyl, or C₁₋₆ alkoxy, wherein the alkyl or alkoxy is optionally substituted with one or more halogen, hydroxy, mercapto, carboxy, amino, or cyano.

In some embodiments, when L^{G} and ring F are both absent, ring E is selected from the group consisting of a benzene ring, a thiazole ring, or a quinazoline ring, and the ring E is optionally substituted with one or more halogen, hydroxy, mercapto, carboxy, amino, cyano, oxo, thioxo, C₁₋₆ alkyl, or C₁₋₆ alkoxy, wherein the alkyl or alkoxy is optionally substituted with one or more halogen, hydroxy, mercapto, carboxy, amino, or cyano.

In some embodiments, L^{E} is -(CR^{p}R^{q})ᵢ-, wherein R^{p}, R^{q}, and i are as defined in the foregoing.

In some embodiments, L^{F} is selected from the group consisting of -(CR^{p}R^{q})ₑ-O-(CR^{p}R^{q})_{f}-, -(CR^{p}R^{q})ₑ-S-(CR^{p}R^{q})_{f}-, -(CR^{p}R^{q})ₑ-N(R^{f})-(CR^{p}R^{q})_{f}-, or -(CR^{p}R^{q})ₑ-C(O)-N(R^{f})-(CR^{p}R^{q})_{f}-, preferably -(CR^{p}R^{q})ₑ-N(R^{f})-(CR^{p}R^{q})_{f}- or -(CR^{p}R^{q})ₑ-C(O)-N(R^{f})-(CR^{p}R^{q})_{f}-, and most preferably -(CR^{p}R^{q})ₑ-C(O)-N(R^{f})-(CR^{p}R^{q})_{f}-, wherein R^{f}, R^{p}, R^{q}, e, and f are as defined in the foregoing.

In some embodiments, the compound represented by formula IC is selected from the group consisting of the compound represented by formula IIC1, formula IIC2, or formula IIC3, or a pharmaceutically acceptable salt thereof, 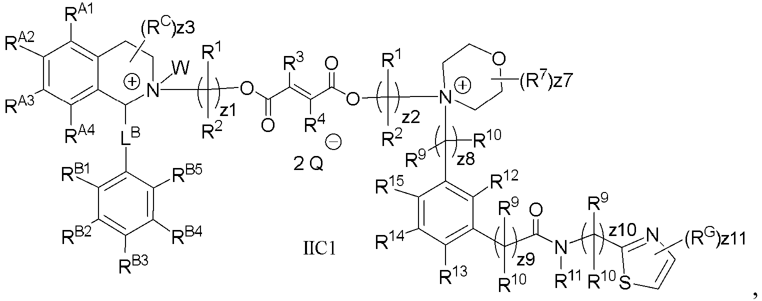 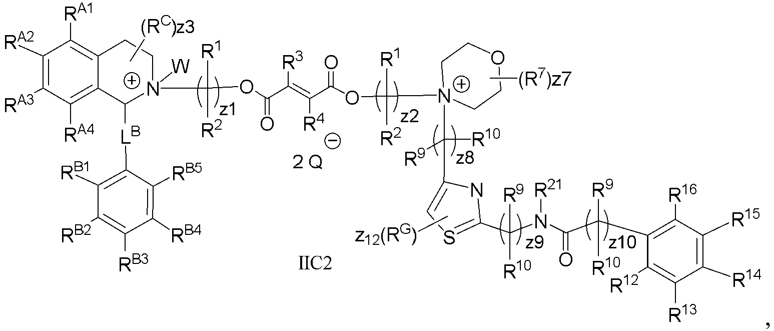 or 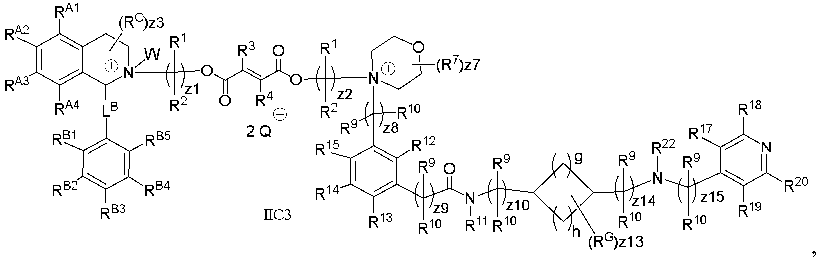 wherein:
R¹¹, R²¹, and R²² are each independently selected from the group consisting of hydrogen, halogen, C₁₋₆ alkyl, or C₁₋₆ alkoxy, wherein the C₁₋₆ alkyl or C₁₋₆ alkoxy is optionally substituted with one or more halogen, hydroxy, mercapto, carboxy, amino, or cyano;
each R^{G} is independently selected from the group consisting of halogen, hydroxy, mercapto, carboxy, amino, cyano, C₁₋₆ alkyl, C₁₋₆ alkoxy, -C(O)-C₁₋₆ alkyl, -C(O)-C₁₋₆ alkoxy, methanesulfonyl, a 3- to 10-membered carbocyclic ring, a 3- to 10-membered heterocyclic ring, a 6- to 10-membered aromatic ring, or a 5- to 10-membered heteroaromatic ring, wherein the alkyl, alkoxy, carbocyclic ring, heterocyclic ring, aromatic ring, or heteroaromatic ring is optionally substituted with one or more halogen, hydroxy, mercapto, carboxy, amino, or cyano,
R⁹, R¹⁰, R¹¹, R¹², R¹³, R¹⁴, R¹⁵, R¹⁶, R¹⁷, R¹⁸, R¹⁹, and R²⁰ are each independently selected from the group consisting of hydrogen, halogen, hydroxy, mercapto, carboxy, amino, cyano, C₁₋₆ alkyl, C₁₋₆ alkoxy, -C(O)-C₁₋₆ alkyl, -C(O)-C₁₋₆ alkoxy, methanesulfonyl, a 3- to 10-membered carbocyclic ring, a 3- to 10-membered heterocyclic ring, a 6- to 10-membered aromatic ring, or a 5- to 10-membered heteroaromatic ring, wherein the alkyl, alkoxy, carbocyclic ring, heterocyclic ring, aromatic ring, or heteroaromatic ring is optionally substituted with one or more halogen, hydroxy, mercapto, carboxy, amino, or cyano,
or alternatively, R⁹ and R¹⁰ together with the atoms to which they are attached form oxo, thioxo, a 3- to 10-membered carbocyclic ring, a 3- to 10-membered heterocyclic ring, a 6- to 10-membered aromatic ring, or a 5- to 10-membered heteroaromatic ring, wherein the carbocyclic ring, heterocyclic ring, aromatic ring, or heteroaromatic ring is optionally substituted with one or more halogen, hydroxy, mercapto, carboxy, amino, cyano, C₁₋₆ alkyl, or C₁₋₆ alkoxy, and the alkyl or alkoxy is optionally substituted with one or more halogen, hydroxy, mercapto, carboxy, amino, or cyano,
or alternatively, R¹² and R¹¹, R¹³ and R¹¹, R¹⁴ and R¹¹, R¹⁵ and R¹¹, R¹⁶ and R¹¹, R¹² and R²¹, R¹³ and R²¹, R¹⁴ and R²¹, R¹⁵ and R²¹, or R¹⁶ and R²¹ together with the atoms to which they are attached form a carbocyclic ring, a heterocyclic ring, an aromatic ring, or a heteroaromatic ring, wherein the carbocyclic ring, heterocyclic ring, aromatic ring, or heteroaromatic ring is optionally substituted with one or more oxo, thioxo, halogen, hydroxy, mercapto, carboxy, amino, cyano, C₁₋₆ alkyl, or C₁₋₆ alkoxy, and the alkyl or alkoxy is optionally substituted with one or more halogen, hydroxy, mercapto, carboxy, amino, or cyano,
or alternatively, R¹⁷ and R¹⁸ or R¹⁹ and R²⁰ together with the atoms to which they are attached form a carbocyclic ring, a heterocyclic ring, an aromatic ring, or a heteroaromatic ring, wherein the carbocyclic ring, heterocyclic ring, aromatic ring, or heteroaromatic ring is optionally substituted with one or more oxo, thioxo, halogen, hydroxy, mercapto, carboxy, amino, cyano, C₁₋₆ alkyl, or C₁₋₆ alkoxy, and the alkyl or alkoxy is optionally substituted with one or more halogen, hydroxy, mercapto, carboxy, amino, or cyano;
each z7 is independently selected from the group consisting of 0, 1, 2, 3, 4, 5, 6, 7, or 8;
each z8 is independently selected from the group consisting of 0, 1, 2, 3, 4, 5, or 6;
each z9 is independently selected from the group consisting of 0, 1, 2, 3, 4, 5, or 6;
each z10 is independently selected from the group consisting of 0, 1, 2, 3, 4, 5, or 6;
z11 is selected from the group consisting of 0, 1, or 2;
z12 is selected from the group consisting of 0 or 1;
z13 is selected from the group consisting of 0, 1, 2, 3, 4, 5, or 6;
each z14 is independently selected from the group consisting of 0, 1, 2, 3, 4, 5, or 6;
each z15 is independently selected from the group consisting of 0, 1, 2, 3, 4, 5, or 6; and
W, Q^{Θ}, L^{B}, R¹, R², R³, R⁴, R^{A1}, R^{A2}, R^{A3}, R^{A2}, R^{B1}, R^{B4}, R^{B3}, R^{B4}, R^{B5}, R^{C}, R⁷, z1, z2, z3, g, and h are as defined in the foregoing.

In some embodiments, R¹³ and R¹¹ together with the atoms to which they are attached form a 3- to 6-membered heterocyclic ring, wherein
the heterocyclic ring is optionally substituted with one or more oxo, thioxo, halogen, hydroxy, mercapto, carboxy, amino, cyano, C₁₋₆ alkyl, or C₁₋₆ alkoxy, and the alkyl or alkoxy is optionally substituted with one or more halogen, hydroxy, mercapto, carboxy, amino, or cyano.

In some embodiments, R¹² and R²¹ together with the atoms to which they are attached form a 3- to 6-membered heterocyclic ring, wherein
the heterocyclic ring is optionally substituted with one or more oxo, thioxo, halogen, hydroxy, mercapto, carboxy, amino, cyano, C₁₋₆ alkyl, or C₁₋₆ alkoxy, and the alkyl or alkoxy is optionally substituted with one or more halogen, hydroxy, mercapto, carboxy, amino, or cyano.

In some embodiments, R¹⁷ and R¹⁸ together with the atoms to which they are attached form a 6-membered aromatic ring or a 5- to 6-membered heteroaromatic ring, wherein the aromatic ring or heteroaromatic ring is optionally substituted with one or more oxo, thioxo, halogen, hydroxy, mercapto, carboxy, amino, cyano, C₁₋₆ alkyl, or C₁₋₆ alkoxy, and the alkyl or alkoxy is optionally substituted with one or more halogen, hydroxy, mercapto, carboxy, amino, or cyano.

In some embodiments, g is 3.

In some embodiments, h is 3.

In some embodiments, the compound represented by formula IC is selected from the group consisting of the compound represented by formula IIIC1, formula IIIC2, or formula IIIC3, or a pharmaceutically acceptable salt thereof, 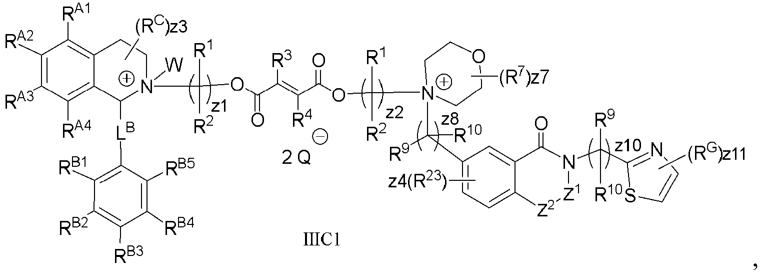 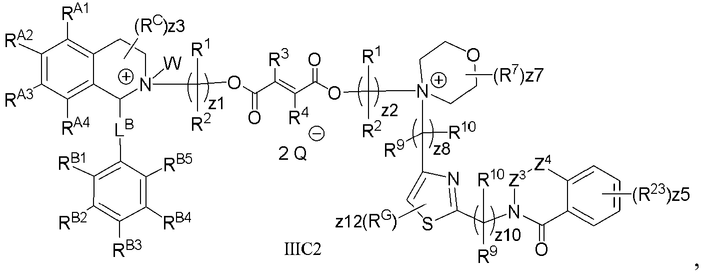 or 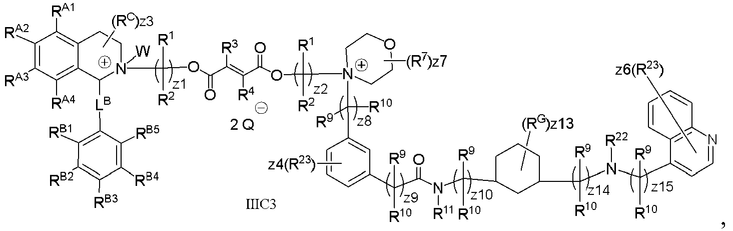 wherein
Z¹ and Z³ are each independently -C(R^{p}R^{q})-;
Z² and Z⁴ are each independently selected from the group consisting of -O-, - C(R^{p}R^{q})-, or -N(R^{f})-;
each R²³ is independently selected from the group consisting of halogen, hydroxy, mercapto, carboxy, amino, cyano, C₁₋₆ alkyl, C₁₋₆ alkoxy, -C(O)-C₁₋₆ alkyl, -C(O)-C₁₋₆ alkoxy, methanesulfonyl, a 3- to 10-membered carbocyclic ring, a 3- to 10-membered heterocyclic ring, a 6- to 10-membered aromatic ring, or a 5- to 10-membered heteroaromatic ring, wherein the alkyl, alkoxy, carbocyclic ring, heterocyclic ring, aromatic ring, or heteroaromatic ring is optionally substituted with one or more halogen, hydroxy, mercapto, carboxy, amino, or cyano;
z4 is selected from the group consisting of 0, 1, 2, or 3;
z5 is selected from the group consisting of 0, 1, 2, 3, or 4;
z6 is selected from the group consisting of 0, 1, 2, 3, 4, 5, or 6; and
W, Q^{Θ}, L^{B}, R¹, R², R³, R⁴, R^{A1}, R^{A2}, R^{A3}, R^{A2}, R^{B1}, R^{B2}, R^{B3}, R^{B4}, R^{B5}, R^{C}, R^{f}, R⁷, R^{p}, R^{q}, z1, z2, z3, g, h, R^{G}, R⁹, R¹⁰, R¹¹, R¹², R¹³, R¹⁴, R¹⁵, R¹⁶, R¹⁷, R¹⁸, R¹⁹, R²⁰, R²², z7, z8, z9, z10, z11, and z12 are as defined in the foregoing.

In some embodiments, Z² and Z⁴ are each independently -N(R^{f})-, wherein R^{f} is as defined in the foregoing.

In some embodiments, Z¹ and Z³ are each independently selected from the group consisting of -C(O)- or -C(S)-. In some embodiments, Z¹ and Z³ are each independently -C(S)-.

In some embodiments, the compound represented by formula IC is selected from the group consisting of the compound represented by formula IVC1, formula IVC2, or formula IVC3, or a pharmaceutically acceptable salt thereof, 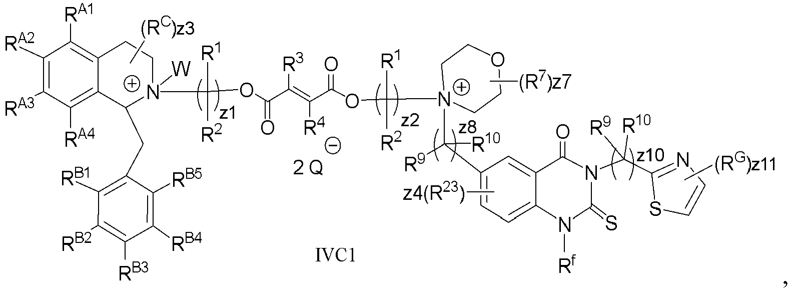 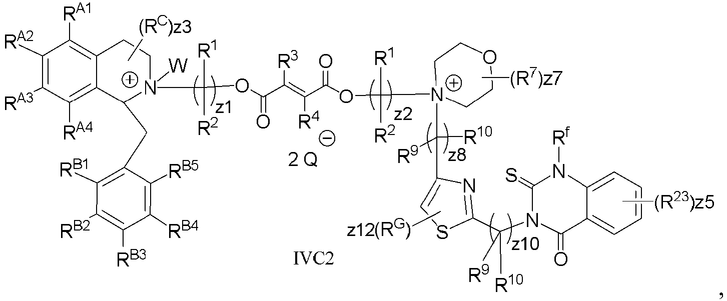 or 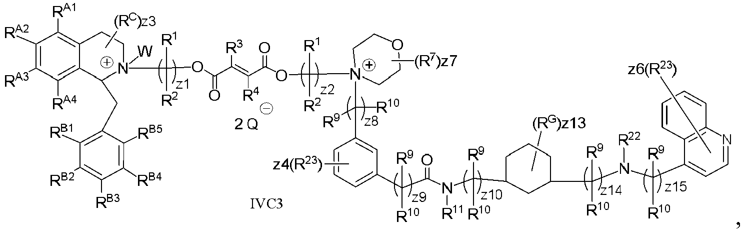 wherein W, Q^{Θ}, L^{B}, R¹, R², R³, R⁴, R^{A1}, R^{A2}, R^{A3}, R^{A2}, R^{B1}, R^{B2}, R^{B3}, R^{B4}, R^{B5}, R^{C}, R^{f}, R⁷, R^{p}, R^{q}, z1, z2, z3, g, h, R^{G}, R⁹, R¹⁰, R¹¹, R¹², R¹³, R¹⁴, R¹⁵, R¹⁶, R¹⁷, R¹⁸, R¹⁹, R²⁰, R²², z7, z8, z9, z10, z11, z12, R²³, z4, z5, and z6 are as defined in the foregoing.

In some embodiments, the compound represented by formula IC or the pharmaceutically acceptable salt thereof is selected from the group consisting of the following compounds or pharmaceutically acceptable salts thereof, 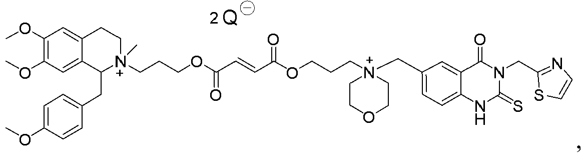 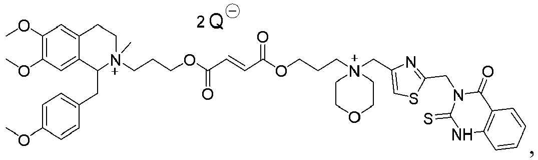 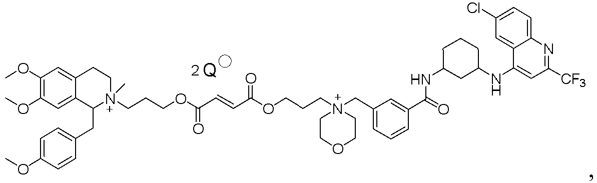 or 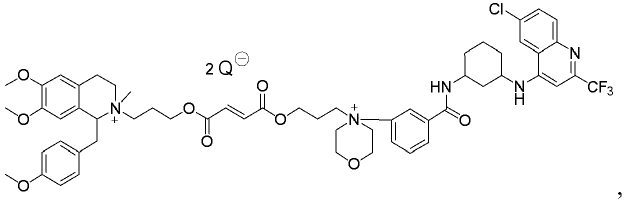 wherein Q^{Θ} is as defined in the foregoing.

In some embodiments, the compound represented by formula IC or the pharmaceutically acceptable salt thereof is selected from the group consisting of the following compounds or pharmaceutically acceptable salts thereof, 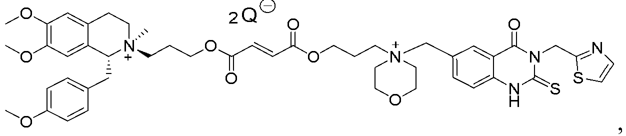 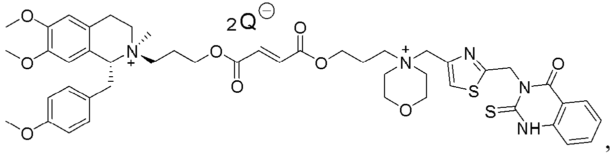 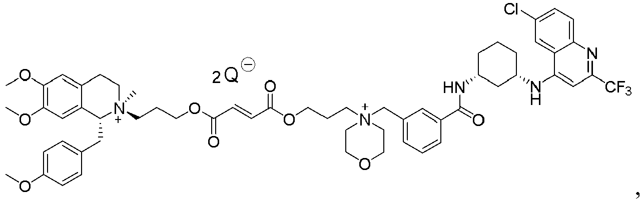 or 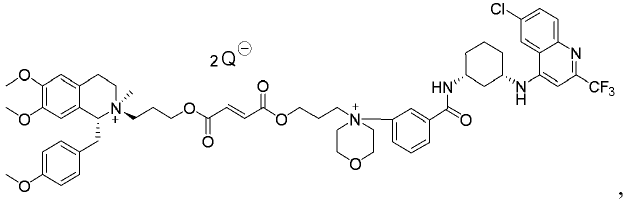 wherein Q^{Θ} is as defined in the foregoing.

In some embodiments, the compound represented by formula IC or the pharmaceutically acceptable salt thereof is selected from the group consisting of the following compounds or pharmaceutically acceptable salts thereof, 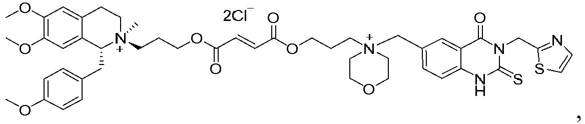 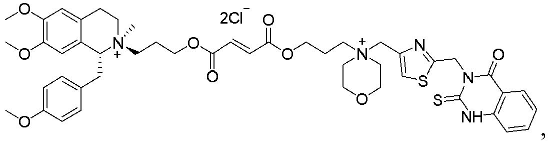 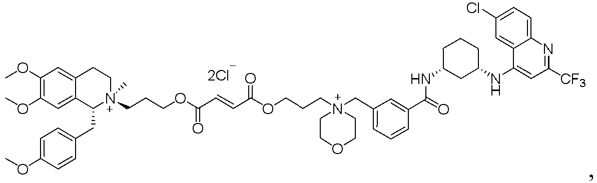 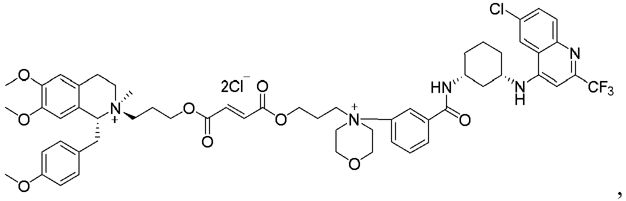 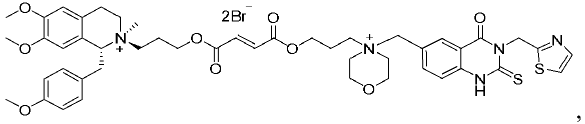 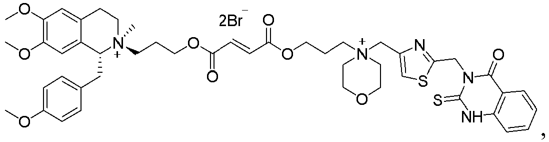 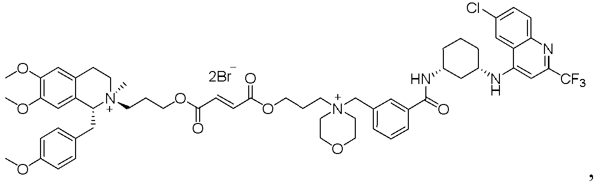 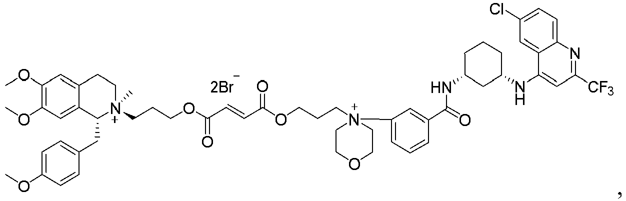 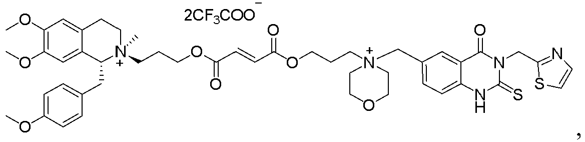 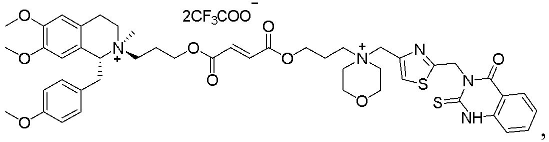 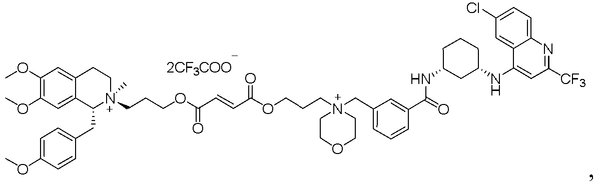 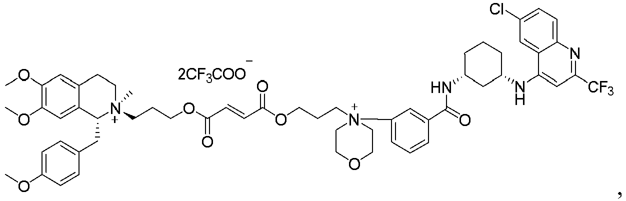 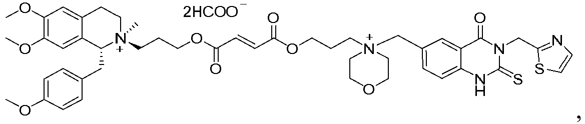 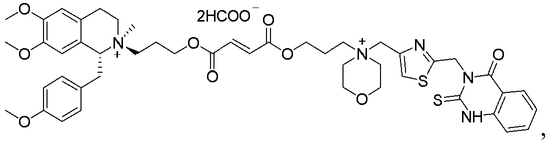 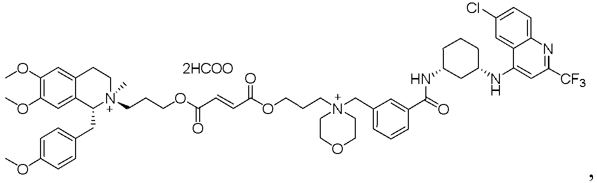 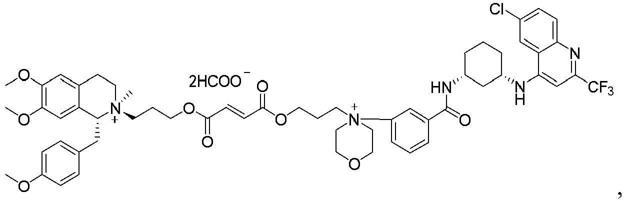 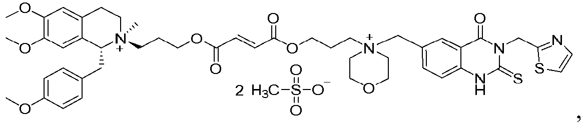 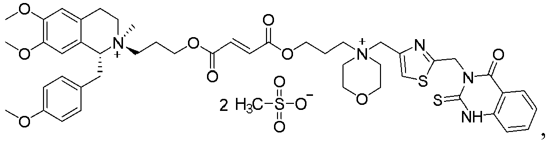 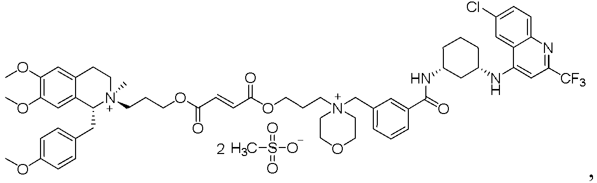 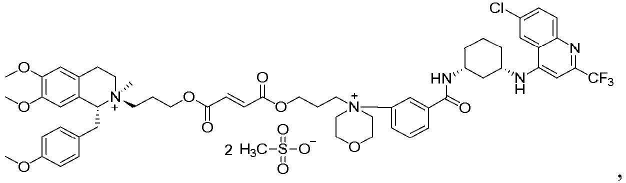 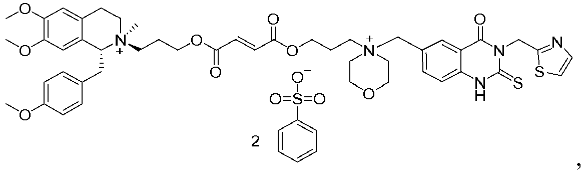 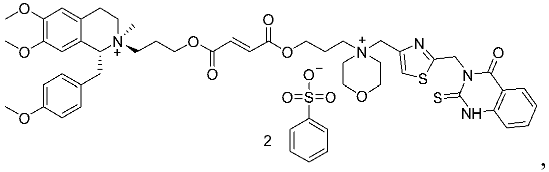 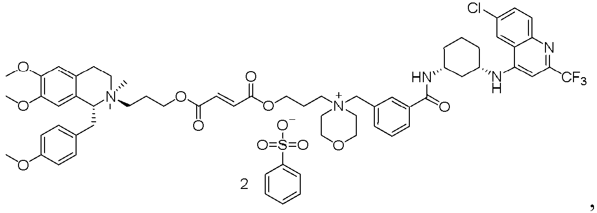 or

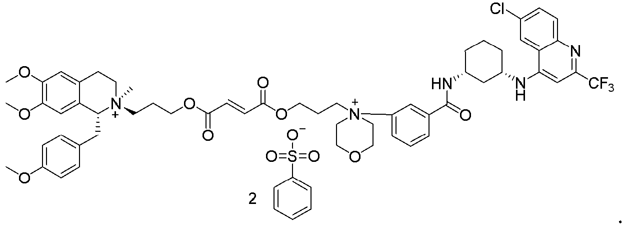

In another aspect, the present invention also provides a compound represented by formula ID or a pharmaceutically acceptable salt thereof, 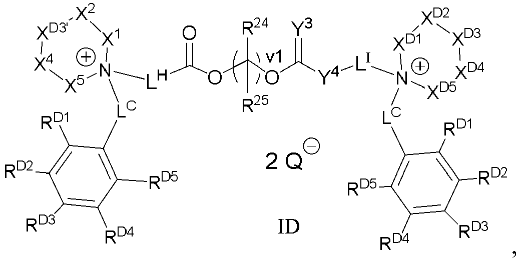 wherein:
Y³ is selected from the group consisting of -O-, -N(R^{f})-, or -S-;
Y⁴ is selected from the group consisting of -O-, -N(R^{f})-, -S-, or -C(R^{r}R^{s})-;
X^{D1} and X^{D5} are each independently -C(R^{g}R^{h})-;
X^{D3} and X^{D3'} are each independently selected from the group consisting of a chemical bond, -O-, -C(R^{d}R^{e})-, or -N(R^{f})-;
X^{D2} and X^{D4} are each independently selected from the group consisting of a chemical bond or -C(RⁱR^{j})-;
L^{H} and L^{I} are each independently selected from the group consisting of alkylene or heteroalkylene, wherein the alkylene or heteroalkylene is optionally substituted with one or more halogen, hydroxy, mercapto, nitro, carboxy, amino, cyano, C₁₋₆ alkyl, C₁₋₆ alkoxy, oxo, thioxo, or sulfonyl, and the C₁₋₆ alkyl or C₁₋₆ alkoxy is optionally substituted with one or more halogen, hydroxy, mercapto, carboxy, amino, or cyano;
R^{r}, R^{s}, R²⁴, and R²⁵ are each independently selected from the group consisting of hydrogen, halogen, hydroxy, mercapto, carboxy, amino, cyano, C₁₋₆ alkyl, C₁₋₆ alkoxy, -C(O)-C₁₋₆ alkyl, -C(O)-C₁₋₆ alkoxy, methanesulfonyl, a 3- to 10-membered carbocyclic ring, a 3- to 10-membered heterocyclic ring, a 6- to 10-membered aromatic ring, or a 5- to 10-membered heteroaromatic ring, wherein the alkyl, alkoxy, carbocyclic ring, heterocyclic ring, aromatic ring, or heteroaromatic ring is optionally substituted with one or more halogen, hydroxy, mercapto, carboxy, amino, or cyano,
or alternatively, R^{r} and R^{s} or R²⁴ and R²⁵ together with the atoms to which they are attached form oxo, thioxo, a 3- to 10-membered carbocyclic ring, a 3- to 10-membered heterocyclic ring, a 6- to 10-membered aromatic ring, or a 5- to 10-membered heteroaromatic ring, wherein the carbocyclic ring, heterocyclic ring, aromatic ring, or heteroaromatic ring is optionally substituted with one or more halogen, hydroxy, mercapto, carboxy, amino, cyano, C₁₋₆ alkyl, or C₁₋₆ alkoxy, and the alkyl or alkoxy is optionally substituted with one or more halogen, hydroxy, mercapto, carboxy, amino, or cyano;
when X^{D3} is selected from the group consisting of a chemical bond or -C(R^{d}R^{e})-,
   (i) R^{d} and R^{e} cannot both be hydrogen, or alternatively,
   (ii) one or more pairs of R^{d} and R^{e}, R^{g} and R^{h}, or Rⁱ and R^{j} together with the atoms to which they are attached form oxo, thioxo, a 3- to 10-membered carbocyclic ring, a 3- to 10-membered heterocyclic ring, a 6- to 10-membered aromatic ring, or a 5- to 10-membered heteroaromatic ring, wherein the carbocyclic ring, heterocyclic ring, aromatic ring, or heteroaromatic ring is optionally substituted with one or more halogen, hydroxy, mercapto, carboxy, amino, cyano, C₁₋₆ alkyl, or C₁₋₆ alkoxy, and the alkyl or alkoxy is optionally substituted with one or more halogen, hydroxy, mercapto, carboxy, amino, or cyano, or alternatively,
   (iii) R^{g} and Rⁱ together with the atoms to which they are attached form a 3- to 10-membered carbocyclic ring, a 3- to 10-membered heterocyclic ring, a 6- to 10-membered aromatic ring, or a 5- to 10-membered heteroaromatic ring, wherein the carbocyclic ring, heterocyclic ring, aromatic ring, or heteroaromatic ring is optionally substituted with one or more halogen, hydroxy, mercapto, carboxy, amino, cyano, C₁₋₆ alkyl, or C₁₋₆ alkoxy, and the alkyl or alkoxy is optionally substituted with one or more halogen, hydroxy, mercapto, carboxy, amino, or cyano;
when X^{D3} is -O-,
   (i) one or both pairs of R^{g} and R^{h} or Rⁱ and R^{j} together with the atoms to which they are attached form oxo, thioxo, a 3- to 10-membered carbocyclic ring, a 3- to 10-membered heterocyclic ring, a 6- to 10-membered aromatic ring, or a 5- to 10-membered heteroaromatic ring, wherein the carbocyclic ring, heterocyclic ring, aromatic ring, or heteroaromatic ring is optionally substituted with one or more halogen, hydroxy, mercapto, carboxy, amino, cyano, C₁₋₆ alkyl, or C₁₋₆ alkoxy, and the alkyl or alkoxy is optionally substituted with one or more halogen, hydroxy, mercapto, carboxy, amino, or cyano, or alternatively,
   (ii) R^{g} and Rⁱ together with the atoms to which they are attached form a 3- to 10-membered carbocyclic ring, a 3- to 10-membered heterocyclic ring, a 6- to 10-membered aromatic ring, or a 5- to 10-membered heteroaromatic ring, wherein the carbocyclic ring, heterocyclic ring, aromatic ring, or heteroaromatic ring is optionally substituted with one or more halogen, hydroxy, mercapto, carboxy, amino, cyano, C₁₋₆ alkyl, or C₁₋₆ alkoxy, and the alkyl or alkoxy is optionally substituted with one or more halogen, hydroxy, mercapto, carboxy, amino, or cyano;
v1 is selected from the group consisting of 1, 2, 3, 4, or 5; and
Q^{Θ}, L^{C}, R^{D1}, R^{D2}, R^{D3}, R^{D4}, R^{D5}, R^{d}, R^{e}, R^{f}, R^{g}, R^{h}, Rⁱ, R^{j}, X¹, X², X⁴, and X⁵ are as defined in the foregoing.

In some embodiments, X^{D3} is selected from the group consisting of a chemical bond or -C(R^{d}R^{e})-, wherein R^{d} and R^{e} are as defined in the foregoing.

In some embodiments, X^{D3'} is -O-.

In some embodiments, Y³ is O.

In some embodiments, Y⁴ is -O-.

In some embodiments, the compound represented by formula ID is selected from the group consisting of the compound represented by formula IID1 or formula IID2, or a pharmaceutically acceptable salt thereof, 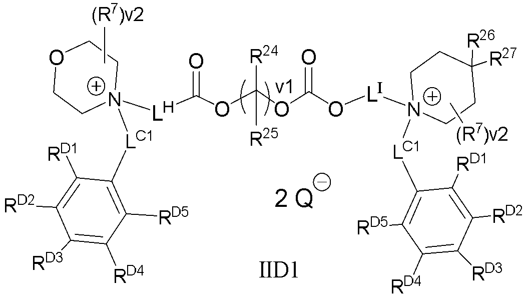 or 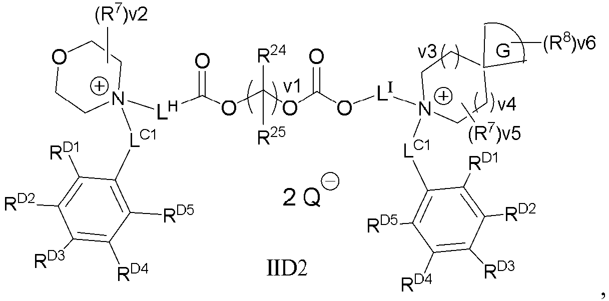 wherein
ring G is selected from the group consisting of a 3- to 10-membered carbocyclic ring, a 3- to 10-membered heterocyclic ring, a 6- to 10-membered aromatic ring, or a 5- to 10-membered heteroaromatic ring;
R²⁶ and R²⁷ are each independently selected from the group consisting of hydrogen, halogen, hydroxy, mercapto, carboxy, amino, cyano, C₁₋₆ alkyl, C₁₋₆ alkoxy, -C(O)-C₁₋₆ alkyl, -C(O)-C₁₋₆ alkoxy, methanesulfonyl, a 3- to 10-membered carbocyclic ring, a 3- to 10-membered heterocyclic ring, a 6- to 10-membered aromatic ring, or a 5- to 10-membered heteroaromatic ring, wherein the alkyl, alkoxy, carbocyclic ring, heterocyclic ring, aromatic ring, or heteroaromatic ring is optionally substituted with one or more halogen, hydroxy, mercapto, carboxy, amino, or cyano, and R²⁶ and R²⁷ cannot both be hydrogen,
or alternatively, R²⁶ and R²⁷ together with the atoms to which they are attached form oxo or thioxo;
each v2 is independently selected from the group consisting of 0, 1, 2, 3, 4, 5, 6, 7, or 8;
each v3 is independently selected from the group consisting of 0 or 1;
each v4 is independently selected from the group consisting of 0 or 1;
v5 is selected from the group consisting of 0, 1, 2, 3, or 4;
v6 is selected from the group consisting of 0, 1, 2, 3, or 4; and
Q^{Θ}, R^{D1}, R^{D2}, R^{D3}, R^{D4}, R^{D5}, L^{C1}, R⁷, R⁸, L^{H}, L^{I}, R²⁴, R²⁵, and v1 are as defined in the foregoing.

In some embodiments, when v3 and v4 are both 1, ring G is a 3- to 10-membered carbocyclic ring. In some embodiments, when v3 and v4 are both 1, ring G is selected from the group consisting of cyclopropane, cyclobutane, cyclopentane, or cyclohexane. In some embodiments, when v3 and v4 are both 1, ring G is cyclopropane.

In some embodiments, when v3 and v4 are both 0, ring G is a 3- to 10-membered heterocyclic ring. In some embodiments, when v3 and v4 are both 0, ring G is selected from the group consisting of an oxetane ring, an azetidine ring, a thietane ring, a tetrahydropyrrole ring, a tetrahydrofuran ring, a tetrahydrothiophene ring, a piperidine ring, a tetrahydropyran ring, or a tetrahydrothiopyran ring. In some embodiments, when v3 and v4 are both 0, ring G is an oxetane ring.

In some embodiments, R²⁶ and R²⁷ cannot both be hydrogen, and are each independently selected from the group consisting of hydrogen, halogen, or C₁₋₆ alkoxy. In some embodiments, R²⁶ and R²⁷ cannot both be hydrogen, and are each independently selected from the group consisting of hydrogen, fluorine, chlorine, bromine, iodine, methoxy, ethoxy, or propoxy. In some embodiments, R²⁶ and R²⁷ cannot both be hydrogen, and are each independently selected from the group consisting of hydrogen, fluorine, or methoxy.

In some embodiments, L^{H} is selected from the group consisting of -(CR^{s}R^{t})ⱼ-, - (CR^{s}R^{t})ₘ-O-(CR^{s}R^{t})ₙ-, -(CR^{s}R^{t})ₘ-S-(CR^{s}R^{t})ₙ-, -(CR^{s}R^{t})ₘ-N(R^{f})-(CR^{s}R^{t})ₙ-, or - (CR^{s}R^{t})ₘ-C(O)-N(R^{f})-(CR^{s}R^{t})ₙ-, preferably -(CR^{s}R^{t})ⱼ-, -(CR^{s}R^{t})ⱼ-, -(CR^{s}R^{t})ₘ-O-(CR^{s}R^{t})ₙ-, -(CR^{s}R^{t})ₘ-S-(CR^{s}R^{t})ₙ-, or -(CR^{s}R^{t})ₘ-N(R^{f})-(CR^{s}R^{t})ₙ-, and most preferably -(CR^{s}R^{t})ⱼ-, wherein
j is selected from the group consisting of 1, 2, 3, 4, 5, or 6;
each m is independently selected from the group consisting of 0, 1, 2, 3, 4, 5, or 6;
each n is independently selected from the group consisting of 0, 1, 2, 3, 4, 5, or 6; and
R^{f}, R^{s}, and R^{t} are as defined in the foregoing.

In some embodiments, L^{I} is selected from the group consisting of -(CR^{s}R^{t})ⱼ-, - (CR^{s}R^{t})ₘ-O-(CR^{s}R^{t})ₙ-, -(CR^{s}R^{t})ₘ-S-(CR^{s}R^{t})ₙ-, -(CR^{s}R^{t})ₘ-N(R^{f})-(CR^{s}R^{t})ₙ-, or - (CR^{s}R^{t})ₘ-C(O)-N(R^{f})-(CR^{s}R^{t})ₙ-, preferably -(CR^{s}R^{t})ⱼ-, -(CR^{s}R^{t})ₘ-O-(CR^{s}R^{t})ₙ-, - (CR^{s}R^{t})ₘ-S-(CR^{s}R^{t})ₙ-, or -(CR^{s}R^{t})ₘ-N(R^{f})-(CR^{s}R^{t})ₙ-, and most preferably -(CR^{s}R^{t})ⱼ- or -(CR^{s}R^{t})ₘ-O-(CR^{s}R^{t})ₙ-, wherein
R^{f}, R^{s}, R^{t}, j, m, and n are as defined in the foregoing.

In some embodiments, the compound represented by formula ID is selected from the group consisting of the compound represented by formula IIID1, formula IIID2-a, or formula IIID2-b, or a pharmaceutically acceptable salt thereof, 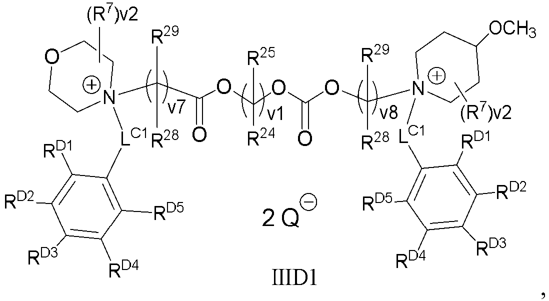 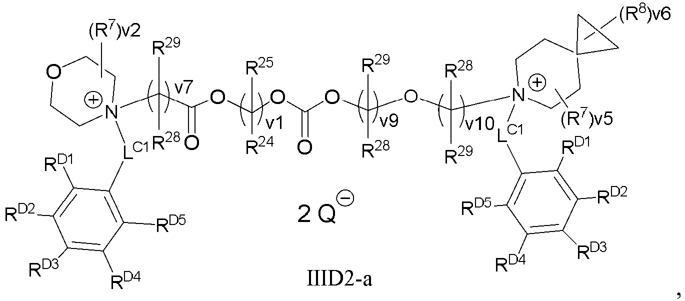 or 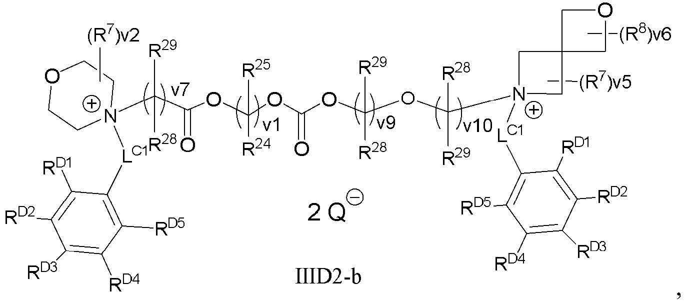 wherein:
R²⁸ and R²⁹ are each independently selected from the group consisting of hydrogen, halogen, hydroxy, mercapto, carboxy, amino, cyano, C₁₋₆ alkyl, C₁₋₆ alkoxy, -C(O)-C₁₋₆ alkyl, -C(O)-C₁₋₆ alkoxy, methanesulfonyl, a 3- to 10-membered carbocyclic ring, a 3- to 10-membered heterocyclic ring, a 6- to 10-membered aromatic ring, or a 5- to 10-membered heteroaromatic ring, wherein the alkyl, alkoxy, carbocyclic ring, heterocyclic ring, aromatic ring, or heteroaromatic ring is optionally substituted with one or more halogen, hydroxy, mercapto, carboxy, amino, or cyano, and R²⁶ and R²⁷ cannot both be hydrogen,
or alternatively, R²⁸ and R²⁹ together with the atoms to which they are attached form oxo or thioxo;
each v7 is independently selected from the group consisting of 1, 2, 3, 4, 5, or 6;
v8 is selected from the group consisting of 1, 2, 3, 4, 5, or 6;
v9 is selected from the group consisting of 1, 2, 3, 4, 5, or 6;
v10 is selected from the group consisting of 1, 2, 3, 4, 5, or 6; and
Q^{Θ}, R^{D1}, R^{D2}, R^{D3}, R^{D4}, R^{D5}, L^{C1}, R⁷, R⁸, R²⁴, R²⁵, v1, v2, v3, v4, v5, and v6 are as defined in the foregoing.

In some embodiments, the compound represented by formula ID is selected from the group consisting of the compound represented by formula IVD1, formula IVD2-a, or formula IVD2-b, or a pharmaceutically acceptable salt thereof, 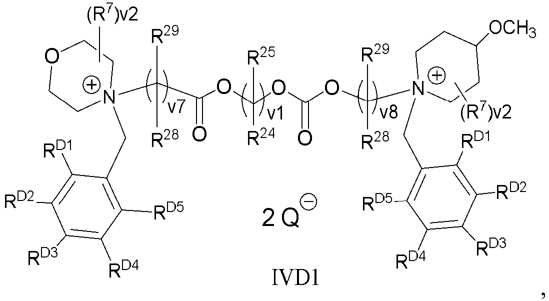 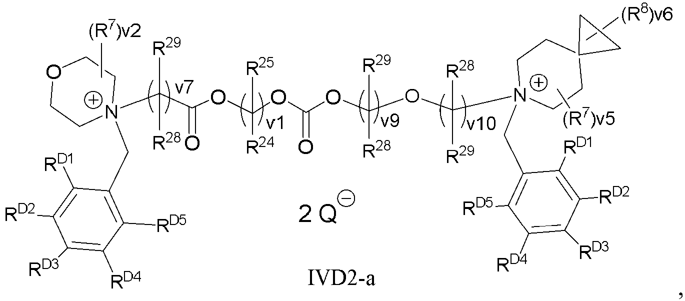 or 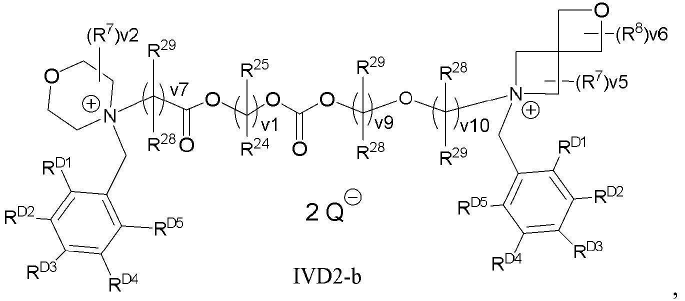 wherein
Q^{⊖}, R^{D1}, R^{D2}, R^{D3}, R^{D4}, R^{D5}, R⁷, R⁸, R²⁴, R²⁵, v1, v2, v3, v4, v5, v6, R²⁸, R²⁹, v7, v8, v9, and v10 are as defined in the foregoing.

In some embodiments, the compound represented by formula ID or the pharmaceutically acceptable salt thereof is selected from the group consisting of the following compounds or pharmaceutically acceptable salts thereof, 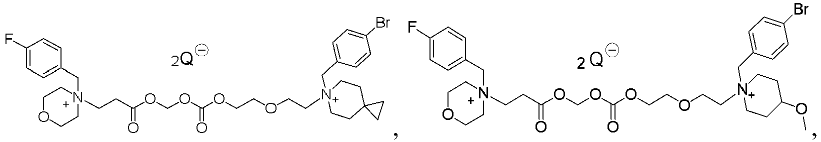 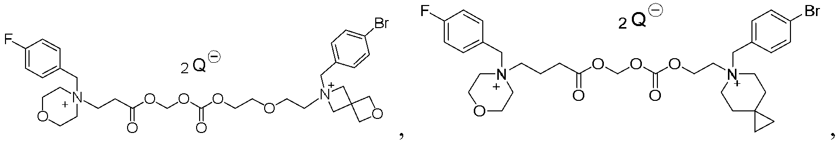 or 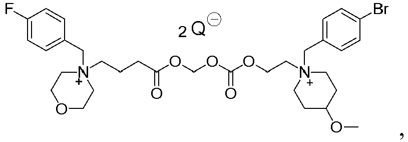 wherein Q^{⊖} is as defined in the foregoing.

In some embodiments, the compound represented by formula ID or the pharmaceutically acceptable salt thereof is selected from the group consisting of the following compounds or pharmaceutically acceptable salts thereof,

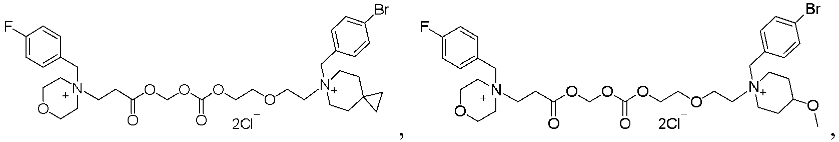

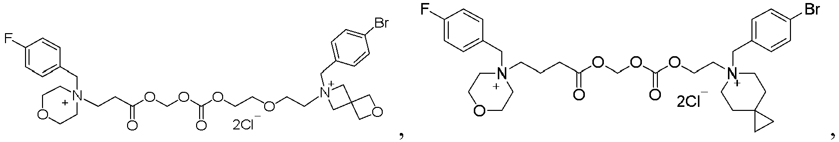

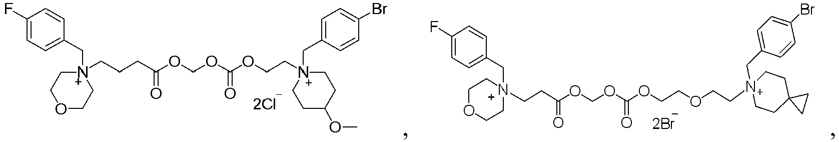

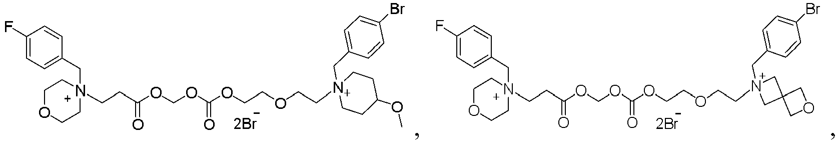

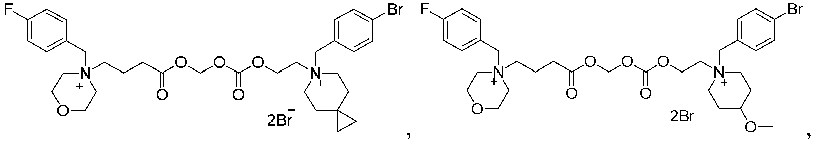

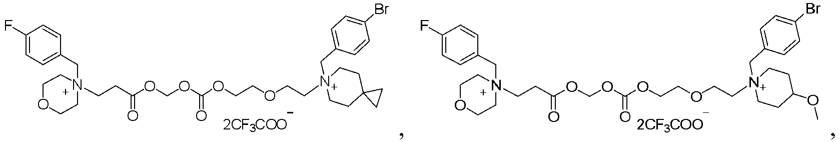

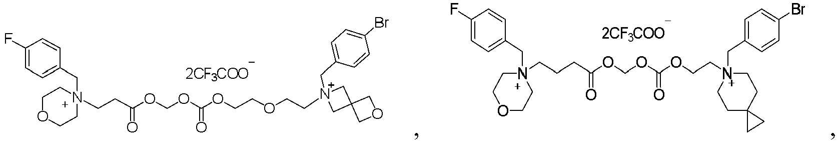

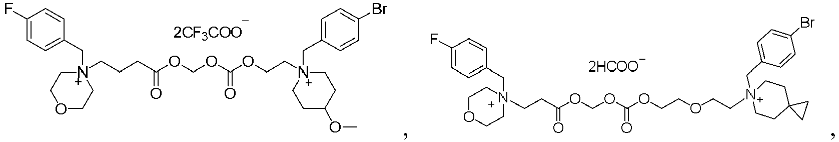

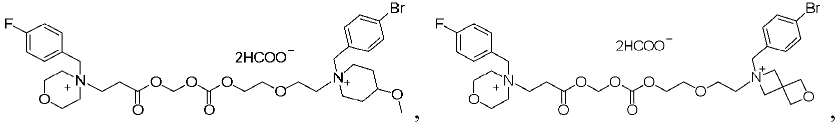

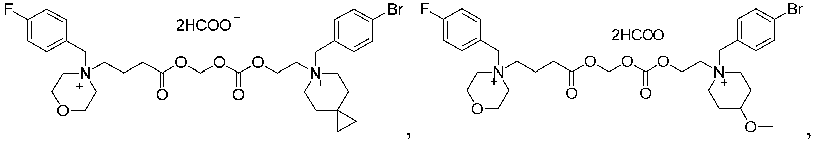

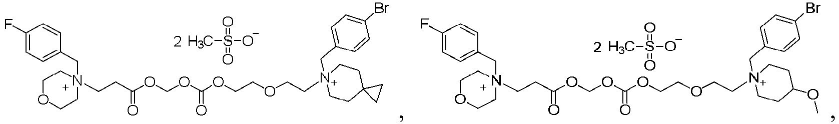

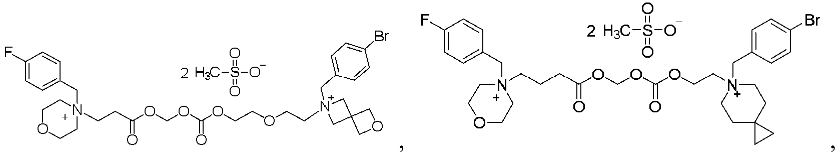

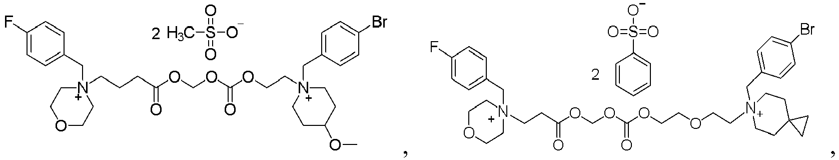

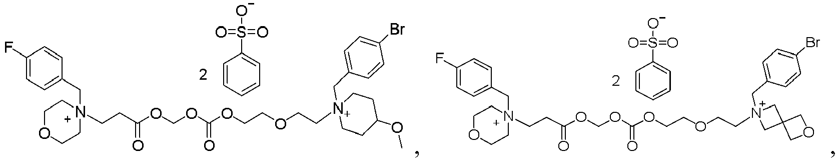

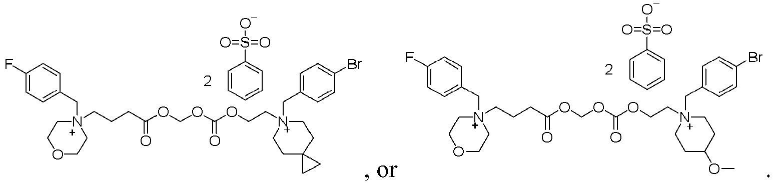

In another aspect, the present invention further discloses a compound or a pharmaceutically acceptable salt thereof, selected from the group consisting of:

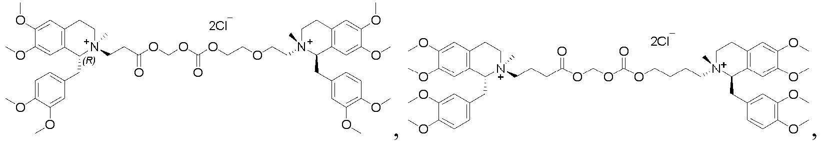

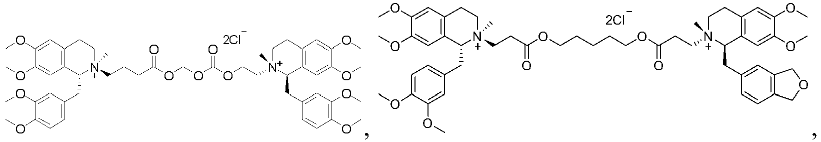

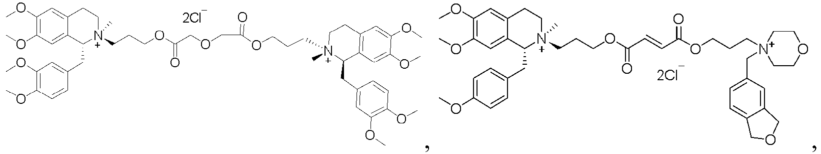

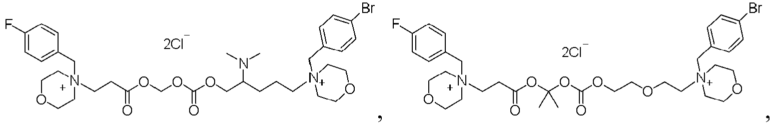

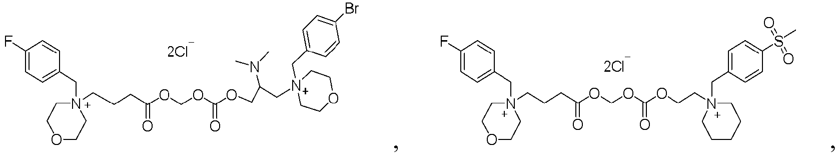

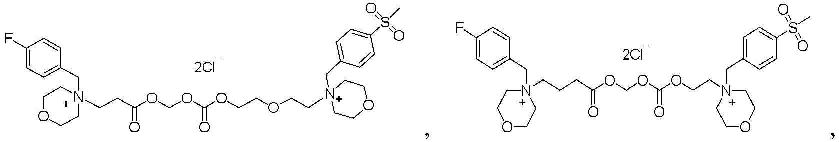

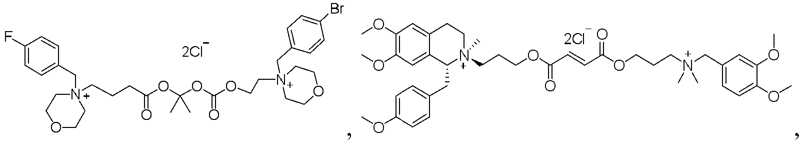

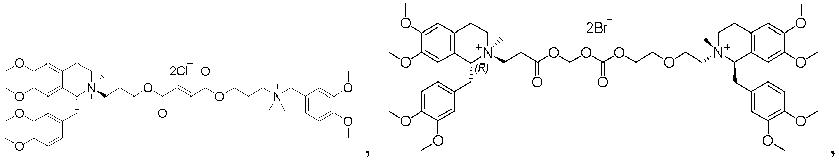

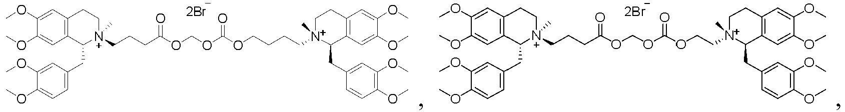

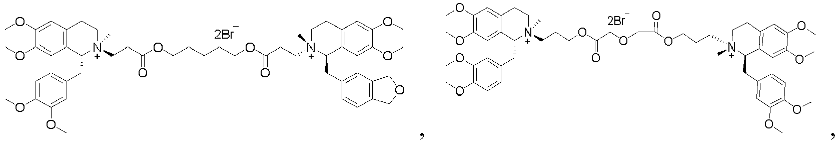

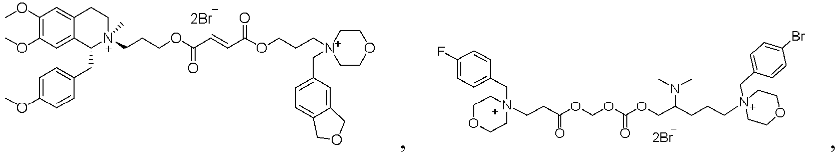

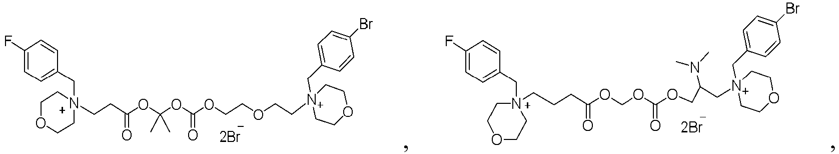

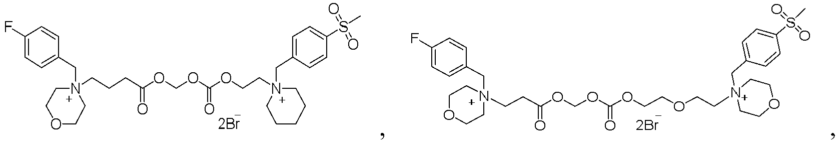

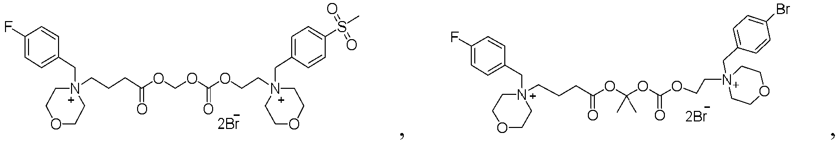

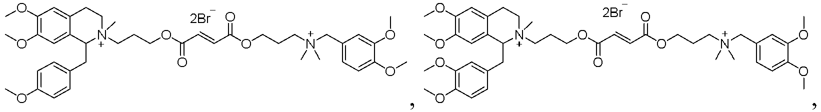

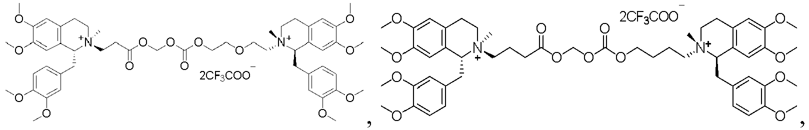

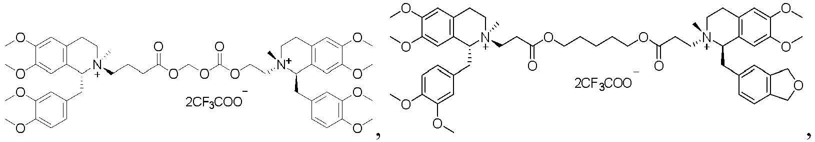

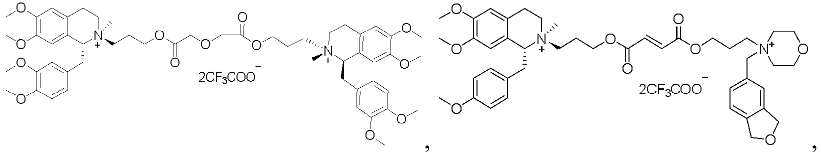

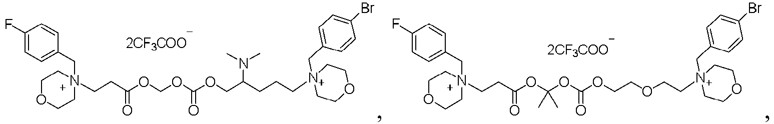

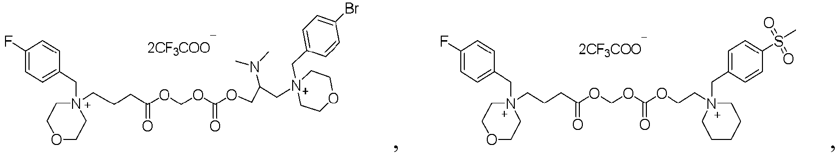

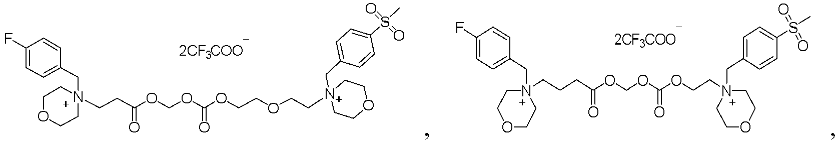

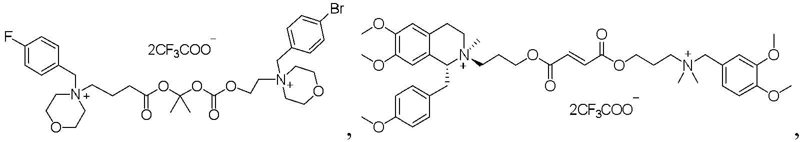

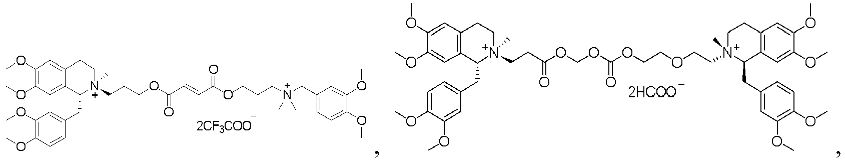

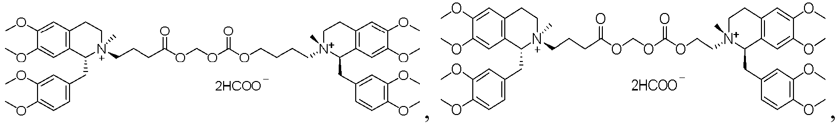

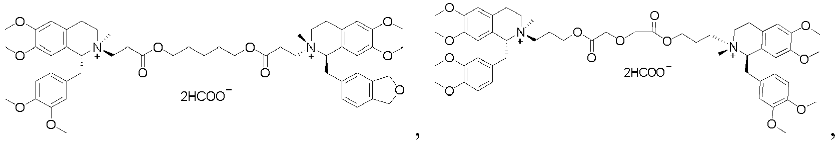

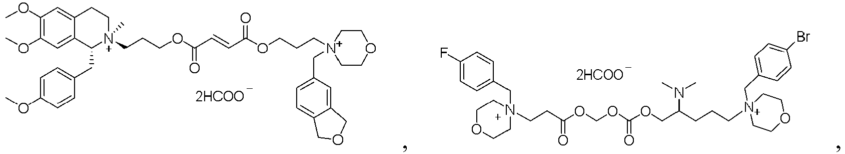

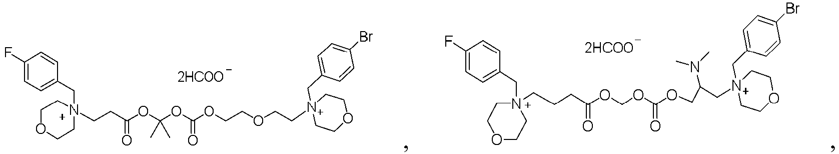

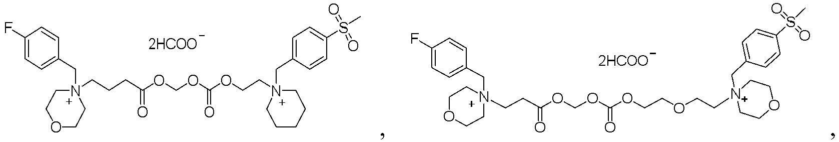

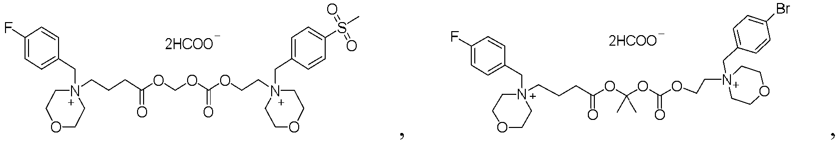

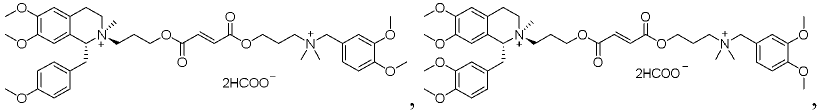

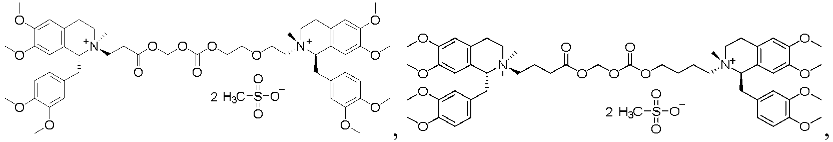

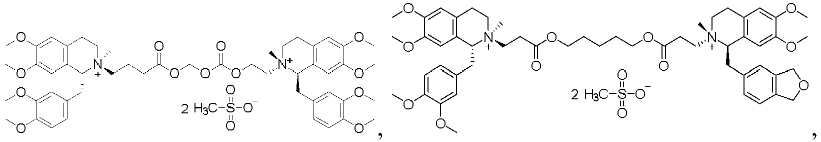

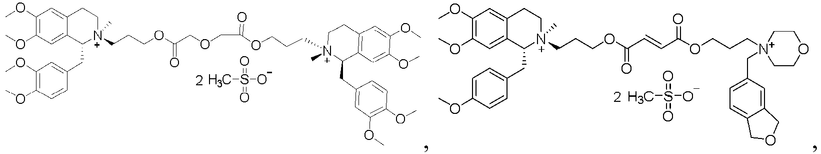

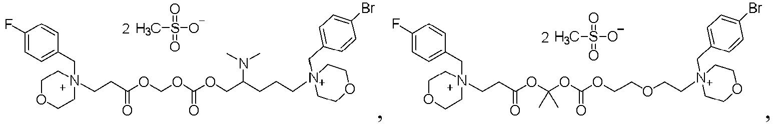

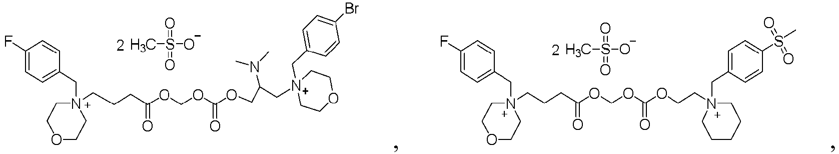

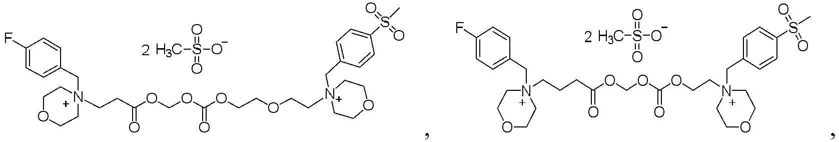

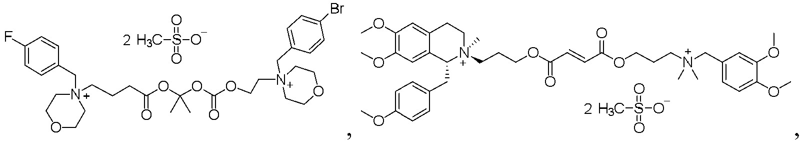

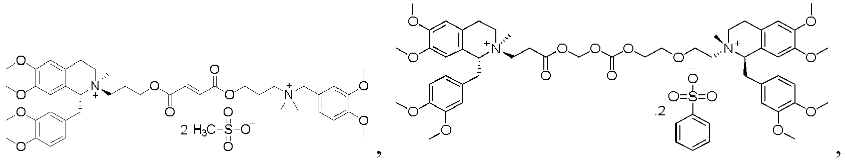

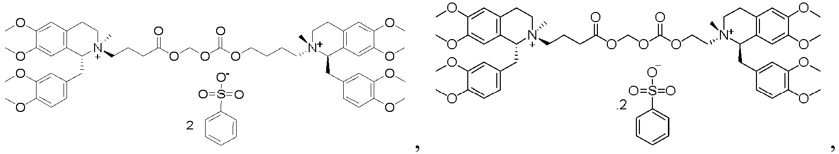

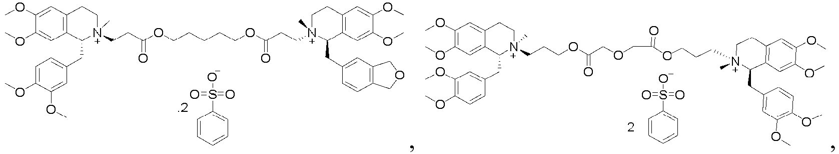

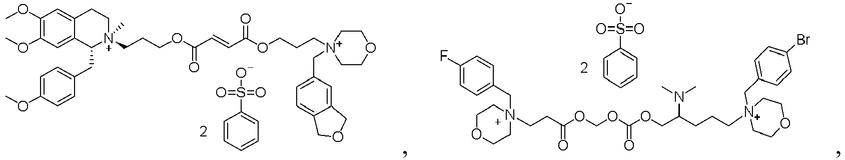

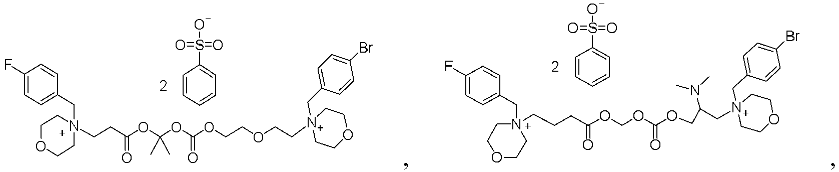

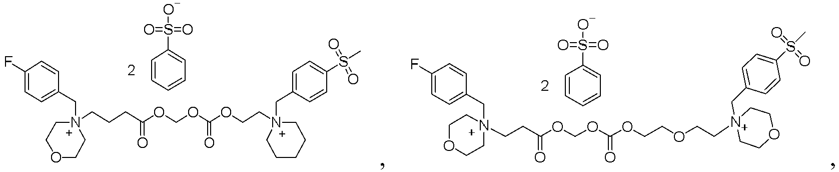

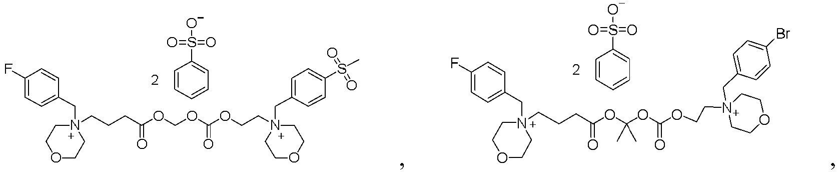

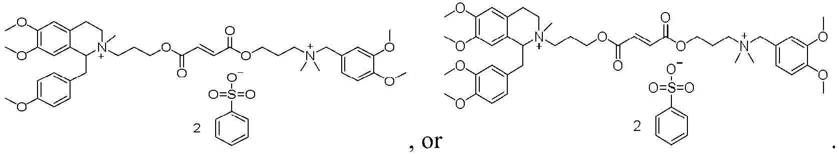

In some embodiments, the compound or the pharmaceutically acceptable salt thereof is selected from the group consisting of:

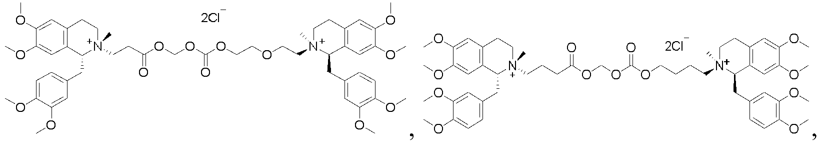

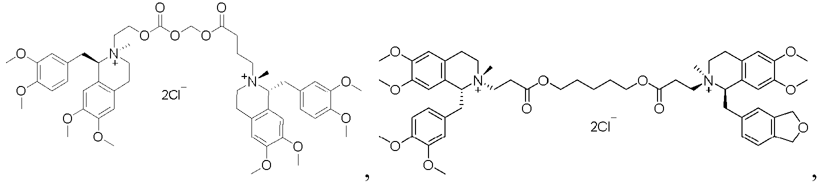

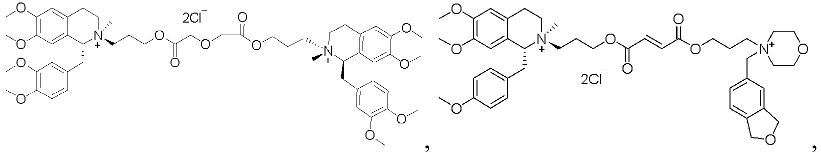

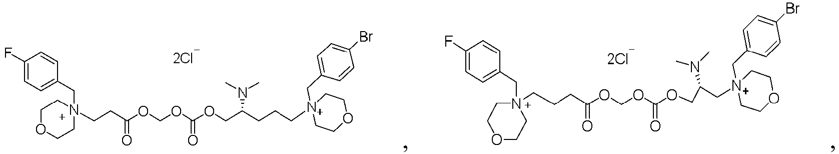

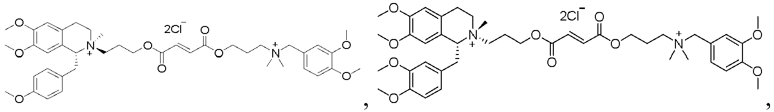

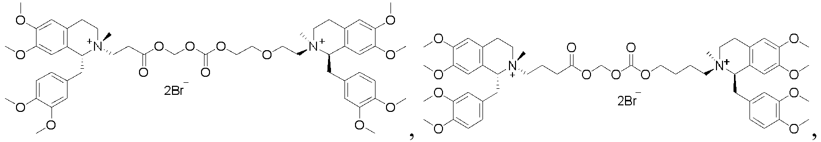

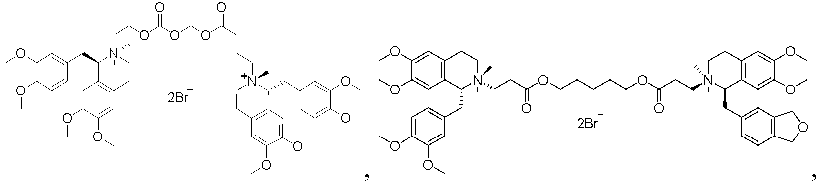

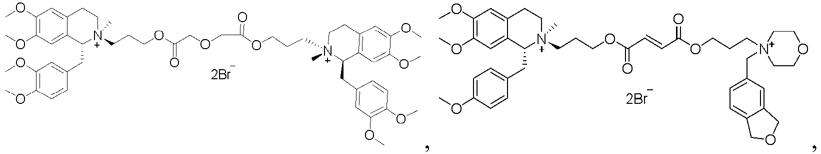

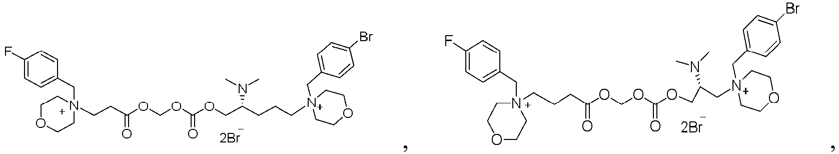

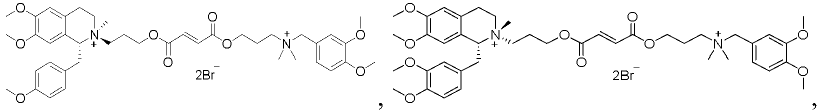

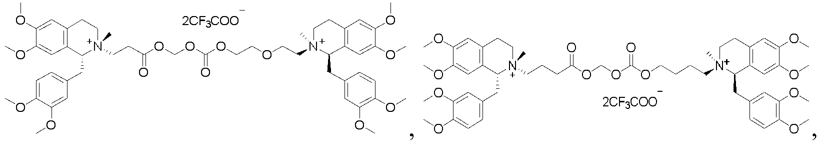

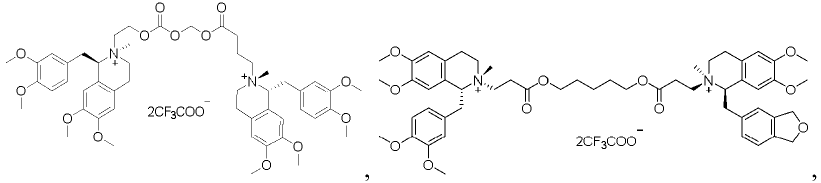

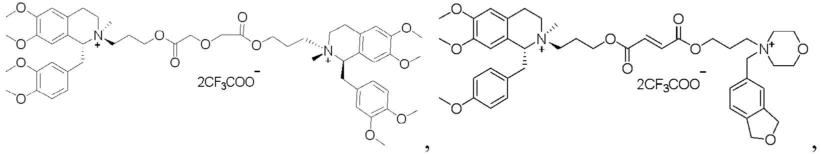

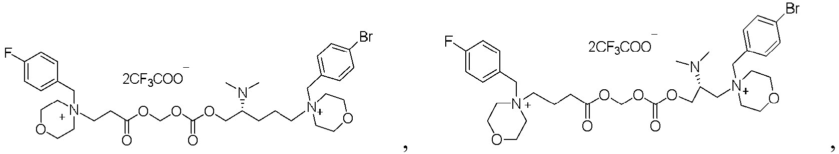

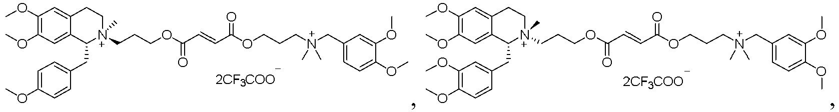

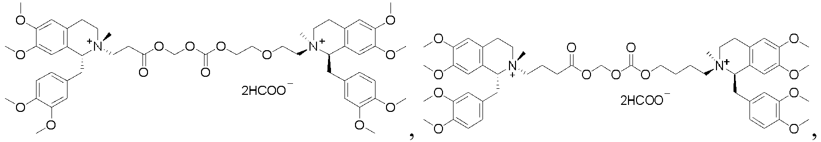

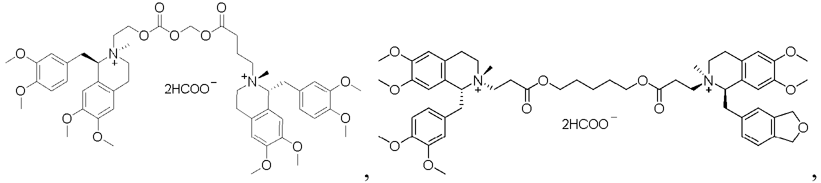

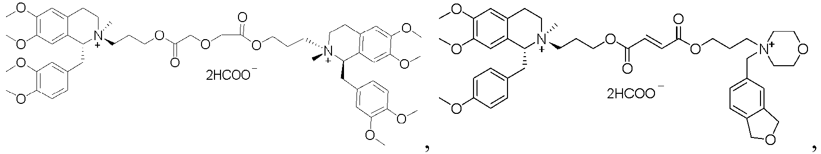

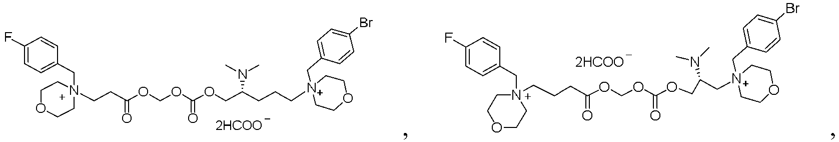

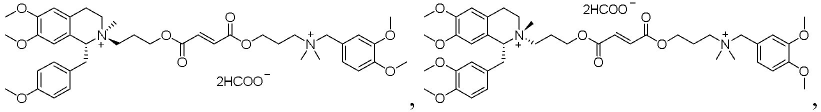

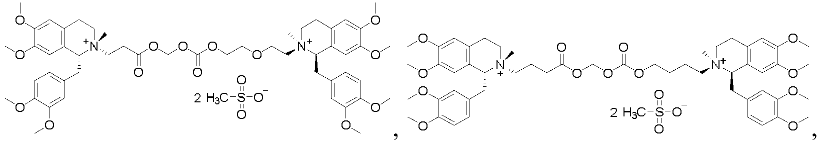

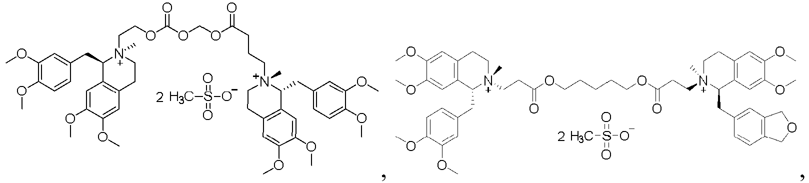

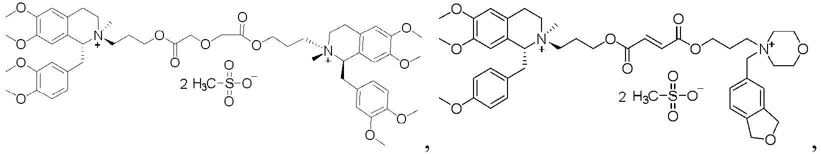

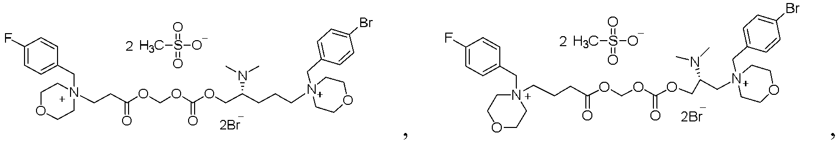

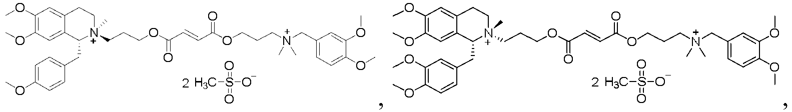

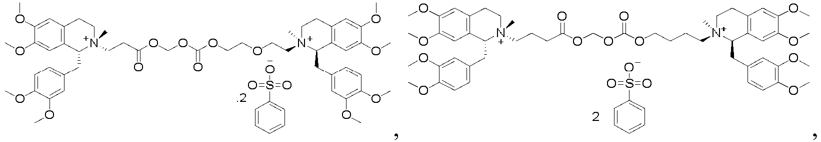

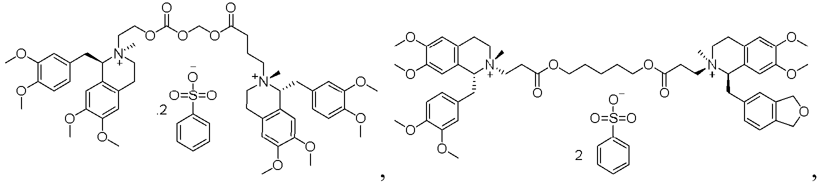

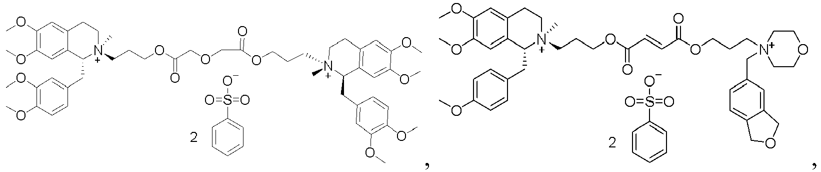

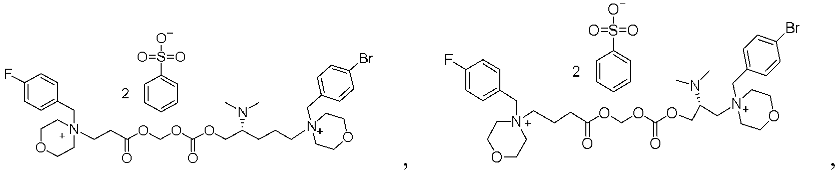

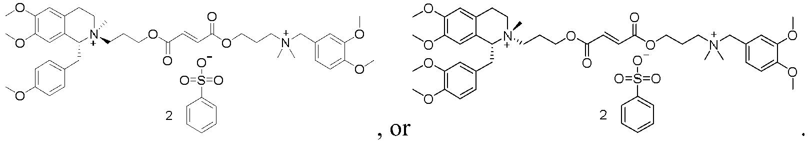

The present invention also provides a compound represented by formula V or a pharmaceutically acceptable salt thereof, 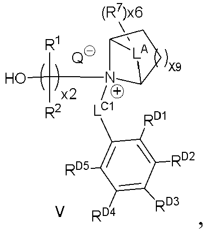 wherein Q^{⊖}, R¹, R², R^{D1}, R^{D2}, R^{D3}, R^{D4}, R^{D5}, R⁷, L^{A}, L^{C1}, x2, x6, and x9 are as defined in the foregoing.

In some embodiments, the compound represented by formula V or the pharmaceutically acceptable salt thereof is selected from the group consisting of the compound represented by formula VI or a pharmaceutically acceptable salt thereof, 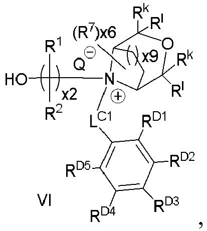 wherein Q^{⊖}, R¹, R², R^{D1}, R^{D2}, R^{D3}, R^{D4}, R^{D5}, R⁷, L^{C1}, x2, x6, x9, R^{k}, and R^{l} are as defined in the foregoing.

In some embodiments, the compound represented by formula V or formula VI or the pharmaceutically acceptable salt thereof is selected from the group consisting of the following compounds or pharmaceutically acceptable salts thereof, 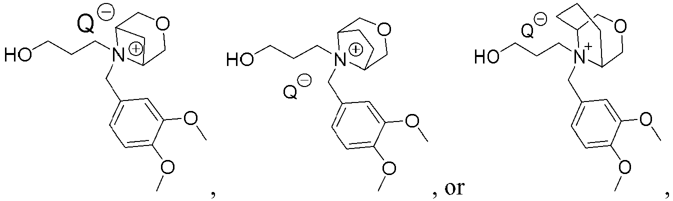 wherein Q^{⊖} is as defined in the foregoing.

In some embodiments, the compound represented by formula V or formula VI or the pharmaceutically acceptable salt thereof is selected from the group consisting of the following compounds or pharmaceutically acceptable salts thereof,

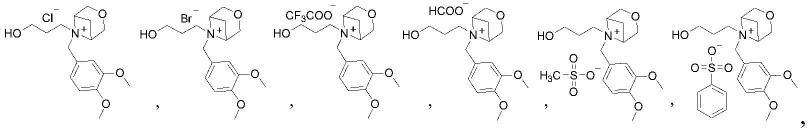

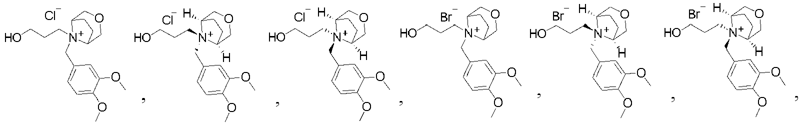

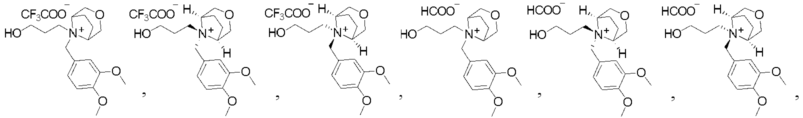

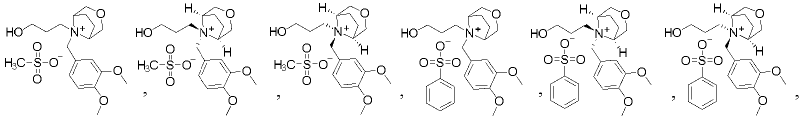

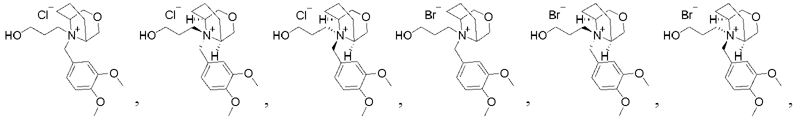

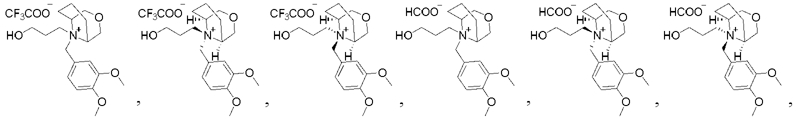

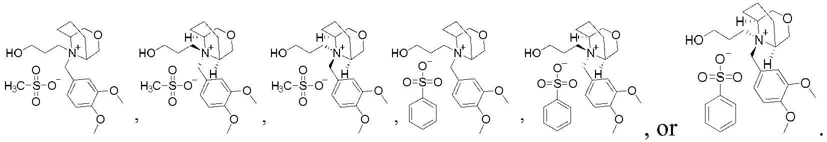

In another aspect, the present invention also provides a method for preparing a compound represented by formula IA or a pharmaceutically acceptable salt thereof, comprising the step of reacting a compound represented by formula A with a compound represented by formula B in the presence of a condensing agent and a catalyst,

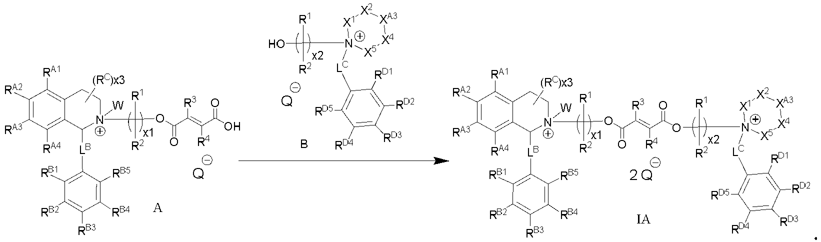

In another aspect, the present invention also provides a method for preparing a compound represented by formula IIA3 or a pharmaceutically acceptable salt thereof, comprising the step of reacting a compound represented by formula A-1 with a compound represented by formula V in the presence of a condensing agent and a catalyst,

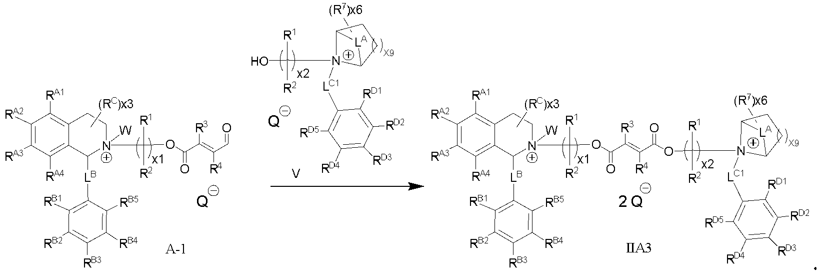

In another aspect, the present invention also provides a method for preparing a compound represented by formula IIIA3-a or a pharmaceutically acceptable salt thereof, comprising the step of reacting a compound represented by formula A-2 with a compound represented by formula VI in the presence of a condensing agent and a catalyst,

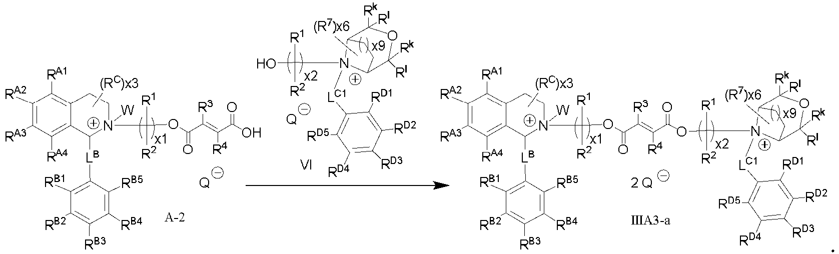

In some embodiments, the condensing agent includes, but is not limited to, HATU.

In some embodiments, the catalyst includes, but is not limited to, DMAP.

The present invention also provides an isotopically substituted compound of the aforementioned compound or the pharmaceutically acceptable salt thereof. In some embodiments, the isotopically substituted compound is a deuterated compound.

The present invention also provides the use of a compound represented by formula V or formula VI or a pharmaceutically acceptable salt thereof in the manufacture of a neuromuscular-blocking drug.

In some embodiments, the unit dose of the pharmaceutical composition is 0.001 mg-1000 mg.

In certain embodiments, the pharmaceutical composition comprises 0.01%-99.99% of the aforementioned compound or the pharmaceutically acceptable salt thereof, or the isotopically substituted compound thereof, on the basis of the total weight of the composition. In certain embodiments, the pharmaceutical composition comprises 0.1%-99.9% of the aforementioned compound or the pharmaceutically acceptable salt thereof, or the isotopically substituted compound thereof. In certain embodiments, the pharmaceutical composition comprises 0.5%-99.5% of the aforementioned compound or the pharmaceutically acceptable salt thereof, or the isotopically substituted compound thereof. In certain embodiments, the pharmaceutical composition comprises 1%-99% of the aforementioned compound or the pharmaceutically acceptable salt thereof, or the isotopically substituted compound thereof. In certain embodiments, the pharmaceutical composition comprises 2%-98% of the aforementioned compound or the pharmaceutically acceptable salt thereof, or the isotopically substituted compound thereof.

In certain embodiments, the pharmaceutical composition comprises 0.01%-99.99% of a pharmaceutically acceptable excipient, on the basis of the total weight of the composition. In certain embodiments, the pharmaceutical composition comprises 0.1%-99.9% of a pharmaceutically acceptable excipient. In certain embodiments, the pharmaceutical composition comprises 0.5%-99.5% of a pharmaceutically acceptable excipient. In certain embodiments, the pharmaceutical composition comprises 1%-99% of a pharmaceutically acceptable excipient. In certain embodiments, the pharmaceutical composition comprises 2%-98% of a pharmaceutically acceptable excipient.

The present invention also provides the use of the aforementioned compound or the pharmaceutically acceptable salt thereof, or the aforementioned pharmaceutical composition in the manufacture of a medicament for neuromuscular blockade.

In certain embodiments, the neuromuscular conduction blockade induced by the medicament for neuromuscular blockade may be reversed by a neuromuscular-blocking antagonist. The neuromuscular-blocking antagonist is, for example, L-cysteine, D-cysteine, or a mixture thereof; N-acetylcysteine; glutathione; homocysteine; methionine; S-adenosylmethionine; or penicillamine; or a combination of the above compounds.

The present invention further provides a method for inducing neuromuscular blockade in a mammal for therapeutic purposes, comprising administering an effective amount of the compound or the pharmaceutically acceptable salt thereof of the present invention, or a pharmaceutical composition comprising the same to the mammal, wherein the mammal may be a human or a non-human mammal, which may be subjected to general anesthesia, and the therapeutic purposes may include surgical procedures.

In certain embodiments, the method may further comprise the step of reversing the neuromuscular conduction blockade in the mammal, comprising administering a neuromuscular-blocking antagonist to the mammal. The neuromuscular-blocking antagonist is, for example, L-cysteine, D-cysteine, or a mixture thereof; N-acetylcysteine; glutathione; homocysteine; methionine; S-adenosylmethionine; or penicillamine; or a combination of the above compounds.

The present invention further provides a kit comprising the compound or the pharmaceutically acceptable salt thereof of the present invention, or a pharmaceutical composition comprising the same. In certain embodiments, the kit may further comprise a neuromuscular-blocking antagonist.

The pharmaceutically acceptable salts of the compounds described in the present invention may be selected from the group consisting of inorganic salts or organic salts. The inorganic salts are selected from the group consisting of hydrochloride, hydrobromide, phosphate, or sulfate; and the organic salts are selected from the group consisting of acetate, trifluoroacetate, methanesulfonate, p-toluenesulfonate, citrate, maleate, tartrate, fumarate, citrate, or lactate.

The compounds of the present invention may exist in specific geometric or stereoisomeric forms. The present invention contemplates all such compounds, including cis and trans isomers, (-)- and (+)-enantiomers, (R)- and (S)-enantiomers, diastereomers, (D)-isomers, (L)-isomers, and racemic mixtures and other mixtures thereof, such as enantiomerically or diastereomerically enriched mixtures, and all such mixtures fall within the scope of the present invention. Additional asymmetric carbon atoms may be present in substituents such as alkyl. All these isomers and mixtures thereof are encompassed within the scope of the present invention. The compounds comprising asymmetric carbon atoms of the present invention may be isolated in optically active pure or racemic forms. The optically active pure forms may be resolved from the racemic mixtures or synthesized by using chiral raw materials or chiral reagents.

Optically active (R)- and (S)-isomers and D and L isomers may be prepared using chiral synthesis or chiral reagents or other conventional techniques. If an enantiomer of a compound of the present invention is desired, it may be prepared by asymmetric synthesis or derivatization with a chiral auxiliary, wherein the resulting diastereomeric mixture is separated, and the auxiliary groups are cleaved to provide the pure desired enantiomer. Alternatively, where the molecule comprises a basic functional group (such as amino) or an acidic functional group (such as carboxy), diastereomeric salts are formed with an appropriate optically active acid or base, followed by resolution of the diastereomers using conventional methods well known in the art, and subsequent recovery of the pure enantiomers. In addition, separation of enantiomers and diastereomers is generally accomplished by using chromatography, which uses chiral stationary phases, optionally in combination with chemical derivatization methods (e.g., formation of carbamates from amines).

**In** the chemical structures of the compounds of the present invention, the bond " 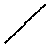 " represents an unspecified configuration; that is, if the chemical structures exist as chiral isomers, the bond " 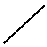 " may be " 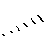 ", " or " 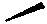 ", or contain both " 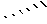 " and " 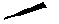 " configurations. For example, the compound of the present invention 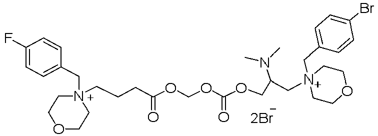 may be either of the following configurations, or contain both of the following configurations:

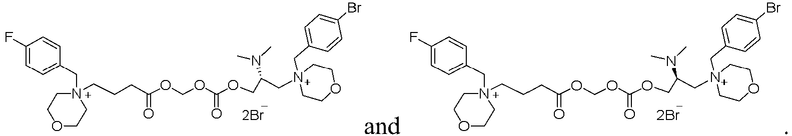

**In** the chemical structures of the compounds of the present invention, the bond " 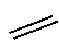 " represents an unspecified configuration, that is, it may be the Z or E configuration, or contain both configurations. For example, the compound of the present invention 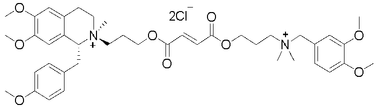 may be either of the following configurations, or contain both of the following configurations:

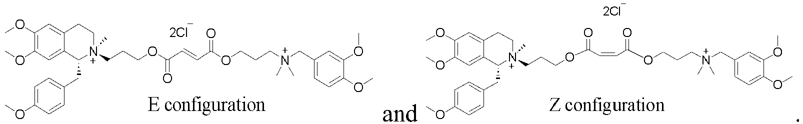

Although all the above structural formulas are depicted as certain isomeric forms for simplicity, the present invention may include all isomers, such as tautomers, geometric isomers, diastereomers, racemates, and enantiomers.

The term "tautomer" or "tautomeric form" refers to structural isomers of different energies which are interconvertible via a low-energy barrier. For example, proton tautomers (also known as prototropic tautomers) involve interconversions via proton transfer. For example, the compound of the present invention includes the tautomeric change between A and B as shown below.

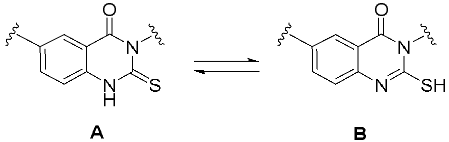

All tautomeric forms are included within the scope of the present invention. The nomenclatures of the compounds do not exclude any tautomers.

The present invention further includes isotopically labeled compounds of the present invention that are identical to those recited herein, but have one or more atoms replaced by an atom having an atomic mass or mass number different from the atomic mass or mass number usually found in nature. Examples of isotopes that can be incorporated into compounds of the present invention include isotopes of hydrogen, carbon, nitrogen, oxygen, phosphorus, sulfur, fluorine, iodine, and chlorine, such as ²H, ³H, ¹¹C, ¹³C, ¹⁴C, ¹³N, ¹⁵N, ¹⁵O, ¹⁷O, ¹⁸O, ³¹P, ³²P, ³⁵S, ¹⁸F, ¹²³I, ¹²⁵I, and ³⁶Cl, respectively.

Unless otherwise specified, when a position is specifically designated as deuterium (D), it should be understood that the position has a deuterium abundance at least 1000 times the natural abundance of deuterium (which is 0.015%) (i.e., at least 10% deuterium incorporation). The expression "having deuterium at an abundance greater than the natural abundance of deuterium" in the compounds in the examples may mean having deuterium at an abundance at least 1000 times, at least 2000 times, at least 3000 times, at least 4000 times, at least 5000 times, at least 6000 times, or having deuterium at a higher abundance. The present invention further includes various deuterated forms of the compound of formula (I). Each available hydrogen atom attached to a carbon atom can be independently replaced with a deuterium atom. Those skilled in the art may synthesize deuterated forms of the compound of formula (I) by referring to relevant literature. The deuterated forms of the compound of formula (I) can be prepared using commercially available deuterated starting materials, or can be synthesized using deuterated reagents via conventional techniques. The deuterated reagents include, but are not limited to, deuterated borane, trideuteroborane tetrahydrofuran solution, deuterated lithium aluminum hydride, deuterated iodoethane, deuterated iodomethane, and the like.

"Optionally" or "optional" means that the subsequently described event or circumstance may but need not occur, and that the description includes instances where the event or circumstance occurs and instances in which it does not. For example, "C₁₋₆ alkyl optionally substituted with halogen or cyano" means that halogen or cyano may but need not be present. The description includes the instance where the alkyl is substituted with halogen or cyano and the instance where the alkyl is not substituted with halogen or cyano.

Compared with control compounds, the compounds of the present invention have significant advantages in terms of both in vivo efficacy and safety window.

### Explanation of terms:

The term "pharmaceutical composition" denotes a mixture containing one or more compounds described herein or physiologically or pharmaceutically acceptable salts or prodrugs thereof and other chemical components, as well as other components, such as physiologically or pharmaceutically acceptable carriers and excipients. The purpose of the pharmaceutical compositions is to facilitate administration to living organisms and facilitate the absorption of active ingredients to exert biological activity.

The term "pharmaceutically acceptable excipient" includes, but is not limited to, any auxiliary, carrier, glidant, sweetener, diluent, preservative, dye/colorant, flavoring agent, surfactant, wetting agent, dispersant, suspending agent, stabilizer, isotonic agent, solvent, or emulsifier that has been approved by the U.S. Food and Drug Administration as acceptable for use in humans or livestock animals.

The term "effective amount" or "therapeutically effective amount" described in the present invention includes an amount sufficient to ameliorate or prevent a symptom or sign of a medical condition. The term effective amount also means an amount sufficient to allow or facilitate diagnosis. The effective amount for a particular patient or veterinary subject may vary depending on factors such as the condition to be treated, the patient's general health, the method, route and dosage of administration, and the severity of side effects. An effective amount may be the maximum dose or administration regimen that avoids significant side effects or toxic effects.

The term "alkyl" refers to a linear or branched saturated aliphatic hydrocarbon group comprising 1 to 6 carbon atoms. The alkyl includes, but is not limited to: methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, and various branched-chain isomers thereof, and the like. The alkyl may be substituted or unsubstituted, and when it is substituted, the substituent may be substituted at any available point of attachment, and is preferably one or more of the following groups, including, but not limited to, halogen, hydroxy, oxo, amino, C₁₋₆ alkyl, C₁₋₆ alkoxy, 3- to 6-membered cycloalkyl, 3- to 6-membered heterocycloalkyl, 6- to 10-membered aryl or 5- to 10-membered heteroaryl, wherein the alkyl, alkoxy, cycloalkyl, heterocycloalkyl, aryl or heteroaryl is optionally substituted with one or more halogen, hydroxy, amino, C₁₋₆ alkyl or C₁₋₆ alkoxy.

The term "cycloalkyl" or "carbocyclic ring" refers to a saturated or partially unsaturated, monocyclic or polycyclic cyclic hydrocarbon substituent. The cycloalkyl ring comprises 3 to 10 carbon atoms. Examples of monocyclic cycloalkyl include, but are not limited to, cyclopropyl, cyclobutyl, cyclopentyl, cyclopentenyl, cyclohexyl, cyclohexenyl, cyclohexadienyl, and the like; and examples of polycyclic cycloalkyl include spiro-fused, fused, and bridged cycloalkyl.

The cycloalkyl or carbocyclic ring may be substituted or unsubstituted, and when it is substituted, the substituent may be substituted at any available point of attachment, and is preferably one or more of the following groups, including, but not limited to, halogen, hydroxy, mercapto, carboxy, amino, cyano, oxo, thioxo, C₁₋₆ alkyl, or C₁₋₆ alkoxy, wherein the alkyl or alkoxy is optionally substituted with one or more halogen, hydroxy, mercapto, carboxy, amino, or cyano.

The term "heterocycloalkyl", "heterocyclyl", or "heterocyclic ring" refers to a saturated or partially unsaturated, monocyclic or polycyclic cyclic hydrocarbon substituent comprising 6 to 10 ring atoms. Examples of monocyclic heterocycloalkyl include, but are not limited to, a piperazine ring, a piperidine ring, an azirane ring, an oxirane ring, a thiirane ring, an oxetane ring, an azetidine ring, a thietane ring, a tetrahydropyrrole ring, a tetrahydrofuran ring, a tetrahydrothiophene ring, a piperidine ring, a tetrahydropyran ring, a tetrahydrothiopyran ring, and the like; and examples of polycyclic heterocycloalkyl include spiro-fused, fused, and bridged heterocycloalkyl.

The heterocycloalkyl, heterocyclyl, or heterocyclic ring may be optionally substituted or unsubstituted, and when it is substituted, the substituent is preferably one or more of the following groups, including, but not limited to, halogen, hydroxy, mercapto, carboxy, amino, cyano, oxo, thioxo, C₁₋₆ alkyl, or C₁₋₆ alkoxy, wherein the alkyl or alkoxy is optionally substituted with one or more halogen, hydroxy, mercapto, carboxy, amino, or cyano.

The term "aryl" or "aromatic ring" refers to any stable, monocyclic or bicyclic carbocyclic ring with up to 7 atoms in each ring, wherein at least one ring is aromatic. Examples of such aryl groups include, but are not limited to, phenyl, naphthyl, tetrahydronaphthyl, indanyl, biphenyl, or binaphthyl. Unless otherwise specified, the aryl or aromatic ring may be substituted or unsubstituted, and when it is substituted, the substituent is preferably one or more of the following groups, including, but not limited to, halogen, hydroxy, mercapto, carboxy, amino, cyano, oxo, thioxo, C₁₋₆ alkyl, or C₁₋₆ alkoxy, wherein the alkyl or alkoxy is optionally substituted with one or more halogen, hydroxy, mercapto, carboxy, amino, or cyano.

The term "heteroaryl" or "heteroaromatic ring" denotes a stable, monocyclic or bicyclic ring with up to 7 atoms in each ring, wherein at least one ring is aromatic and at least one ring contains 1 to 4 heteroatoms selected from the group consisting of O, N, and S. Heteroaromatic rings within the scope of this definition include, but are not limited to, a pyridine ring, a thiazine ring, a pyrimidine ring, a pyridazine ring, a furan ring, a pyrrole ring, a thiophene ring, an imidazole ring, a pyrazole ring, an oxazole ring, an isoxazole ring, a thiazole ring, a benzofuran ring, an isobenzofuran ring, an isoindole ring, an indole ring, a benzothiophene ring, a benzimidazole ring, an indazole ring, a benzoxazole ring, a benzisoxazole ring, a purine ring, a benzothiazole ring, a quinoline ring, an isoquinoline ring, a quinazoline ring, a quinazolinone ring, and a thioquinazolinone ring. Unless otherwise specified, the heteroaryl or heteroaromatic ring may be substituted or unsubstituted.

The heteroaryl may be optionally substituted or unsubstituted, and when it is substituted, the substituent is preferably one or more of the following groups, including, but not limited to, halogen, hydroxy, mercapto, carboxy, amino, cyano, oxo, thioxo, C₁₋₆ alkyl, or C₁₋₆ alkoxy, wherein the alkyl or alkoxy is optionally substituted with one or more halogen, hydroxy, mercapto, carboxy, amino, or cyano.

The term "alkoxy" refers to -O-(alkyl), wherein the alkyl is as defined above. Examples of alkoxy include, but are not limited to: methoxy, ethoxy, propoxy, butoxy, cyclopropoxy, cyclobutoxy, cyclopentoxy, and cyclohexyloxy. The alkoxy may be optionally substituted or unsubstituted, and when it is substituted, the substituent is preferably one or more of the following groups, including, but not limited to: halogen, hydroxy, mercapto, carboxy, amino, cyano, oxo, thioxo, C₁₋₆ alkyl, or C₁₋₆ alkoxy, wherein the alkyl or alkoxy is optionally substituted with one or more halogen, hydroxy, mercapto, carboxy, amino, or cyano.

The term "halogen" refers to fluorine, chlorine, bromine or iodine.

The term "hydroxy" refers to -OH.

The term "amino" refers to -NH₂.

The term "cyano" refers to -CN.

The term "nitro" refers to -NO₂.

The term "oxo group" or "oxo" refers to "=O".

The term "thioxo group" or "thioxo" refers to "=S".

The term "carbonyl" refers to C=O.

The term "carboxy" refers to -C(O)OH.

The term "halo" refers to substitution with one or more atoms selected from the group consisting of fluorine, chlorine, bromine, and iodine.

The term "monovalent group" refers to a group formed by "formally" eliminating a monovalent atom or group from a compound.

The term "-ylene" refers to a group formed by "formally" eliminating two monovalent atoms or atomic groups or one divalent atom or atomic group from a compound.

The term "alkylene" refers to the moiety remaining after removal of two hydrogen atoms from an alkane molecule, including linear and branched -ylene groups comprising 1 to 20 carbon atoms. Examples of alkylene containing 1 to 6 carbon atoms include, but are not limited to: methylene (-CH₂-) and ethylene (such as -CH₂CH₂- or -CH(CH₃)-). Unless otherwise specified, the alkylene may be substituted or unsubstituted.

The term "heteroalkylene" refers to an alkylene in which one or more -CH₂-groups are replaced by a heteroatom selected from the group consisting of N, O, and S, wherein the alkylene is as defined above. Unless otherwise specified, the heteroalkylene may be substituted or unsubstituted.

The term "cycloalkylene" refers to a group with two monovalent radical centers, which are derived by removing two hydrogen atoms from the same carbon atom or two different carbon atoms of the parent cycloalkylene group. Examples of the cycloalkylene include, but are not limited to, cyclopropylene, cyclobutylene, cyclopentylene, and cyclohexylene. The cycloalkyl is as defined above.

The term "heterocyclylene" refers to a heterocyclyl with two monovalent radical centers, which are derived by removing two hydrogen atoms from the same carbon atom or two different carbon atoms of the parent heterocyclic ring, removing two hydrogen atoms from two nitrogen atoms of the parent heterocyclic ring, or removing one hydrogen atom from a nitrogen atom of the parent heterocyclic ring and one hydrogen atom from a carbon atom thereof. Examples of the heterocyclylene include, but are not limited to, piperidine-1,4-diyl, piperazine-1,4-diyl, tetrahydrofuran-2,4-diyl, tetrahydrofuran-3,4-diyl, azetidine-1,3-diyl, and pyrrolidine-1,3-diyl. The heterocyclyl is as defined above.

The term "arylene" refers to a group with two monovalent radical centers, which are derived by removing two hydrogen atoms from the same carbon atom or two different carbon atoms of the parent aryl group. Examples of the arylene include, but are not limited to, 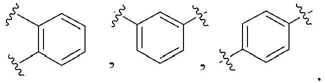 The aryl is as defined above.

The term "heteroarylene" refers to a heteroaryl system with two points of attachment to the rest of the molecule. Examples of the heteroarylene include, but are not limited to, 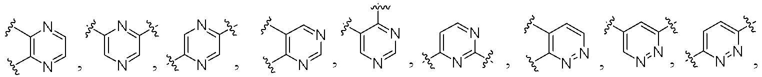 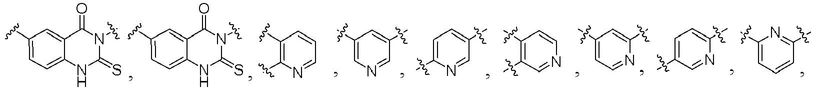 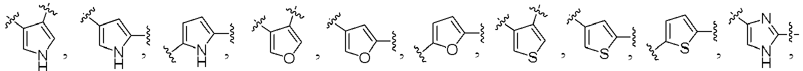 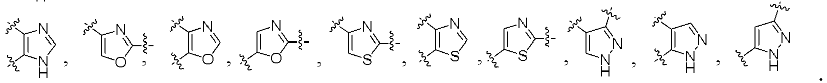 The heteroaryl is as defined above.

The term "substituted" means that one or more hydrogen atoms, preferably at most 5, more preferably 1-3 hydrogen atoms in the group are each independently substituted with a corresponding number of substituents. It goes without saying, the substituents may only be at their chemically possible positions, and a person skilled in the art can determine the possible or impossible substitutions (by experiment or theory) without undue effort.

### Examples

The present invention will be further described below in conjunction with examples, but these examples are not intended to limit the scope of the present invention.

Experimental methods without specific conditions indicated in the examples of the present invention are generally conducted under conventional conditions, or conditions recommended by the manufacturers of the starting materials or commercial products. Reagents for which the specific source is not specified are conventional, commercially available reagents.

The structures of the compounds are determined by nuclear magnetic resonance (NMR) or/and mass spectrometry (MS). The NMR shift (δ) is given in the unit of 10⁻⁶ (ppm). NMR is determined with a nuclear magnetic resonance spectrometer Bruker AVANCE-400. The solvents for determination are deuterated dimethyl sulfoxide (DMSO-*d₆*), deuterated chloroform (CDCl₃), and deuterated methanol (methanol-*d₄*), and the internal standard is tetramethylsilane (TMS).

HPLC is determined with Agilent1100 high pressure liquid chromatograph, GAS15B DAD ultraviolet detector and Water Vbridge C18 150*4.6 mm 5 um chromatographic column.

MS is determined with Agilent6120 triple quadrupole mass spectrometer, G1315D DAD detector and Waters Xbridge C18 4.6*50 mm, 5 um chromatographic column. The sample is scanned in a positive/negative ion mode with a mass scan range of 80-1200.

Yantai Huanghai HSGF254 silica gel plate is used as a thin layer chromatography silica gel plate, and the silica gel plate for the thin layer chromatography (TLC) is of the specification of 0.2 mm ± 0.03 mm, and the specification when separating and purifying a product by thin layer chromatography is 0.4 mm-0.5 mm.

Combiflash Rf150 (TELEDYNE ISCO) or Isolara one (Biotage) is used for the flash column purification system.

For the normal-phase chromatography, Yantai Huanghai silica gel of 200-300 mesh or 300-400 mesh silica gel is generally used as the carrier, or Santai Technologies pre-packed ultra-pure normal phase silica gel column (40-63 µm, 60 g, 24 g, 40 g, 120 g or other specifications) is used.

Known starting materials in the present invention may be synthesized using or according to methods known in the art, or may be purchased from Shanghai Titan Scientific, ABCR GmbH & Co. KG, Acros Organics, Aldrich Chemical Company, Accela ChemBio Inc., Bide Pharmatech Ltd., and other companies.

Unless otherwise specified in the examples, all reactions can be carried out under nitrogen atmosphere.

The nitrogen atmosphere means that the reaction flask is connected to a nitrogen balloon with a volume of about 1 L.

The hydrogen atmosphere means that the reaction flask is connected to a hydrogen balloon with a volume of about 1 L.

Hydrogen is produced by QPH-1L hydrogen generator from Shanghai Quanpu Scientific Instrument Co., Ltd.

The establishment of nitrogen atmosphere or hydrogen atmosphere usually comprises: evacuating, filling with nitrogen or hydrogen, and repeating the operation three times.

Unless otherwise specified in the examples, the solution refers to an aqueous solution.

Unless otherwise specified in the examples, the reaction temperature refers to room temperature, which is 20°C to 30°C.

The reaction progress in the examples is monitored by thin layer chromatography (TLC). The developing solvent used in the reaction, the eluent system for column chromatography and the developing solvent system for thin layer chromatography used to purify the compound, and the volume ratio of the solvent are adjusted according to the polarity of the compound, and a small amount of alkaline or acidic reagents such as triethylamine and acetic acid can be added for adjustment.

### Example 1. Preparation of Compound 1

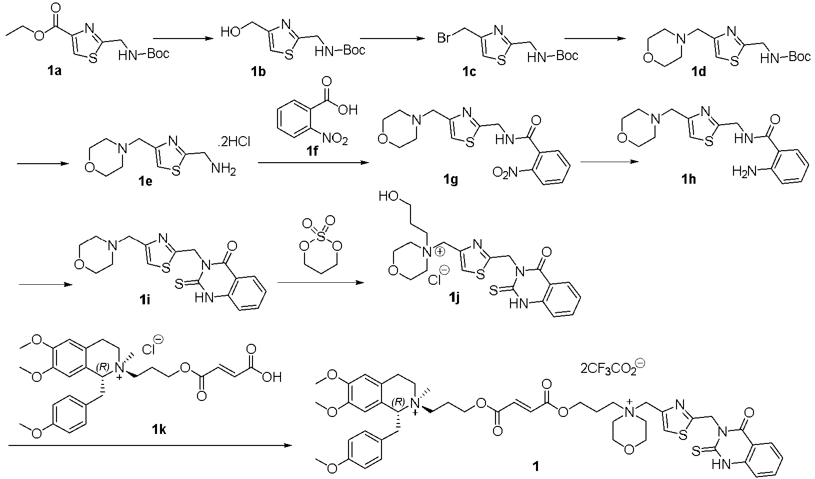

### Step 1)

Compound 1a (1.8 g, 6.2 mmol, purchased from Leyan), methanol (604 mg, 18.86 mmol), and THF (50 ml) were added to a 100 mL three-necked flask. Stirring was initiated, the mixture was purged with nitrogen three times, and lithium borohydride (410 mg, 18.86 mmol) was then added in one portion. The reaction was stirred at elevated temperature until the reaction was complete, and then cooled to room temperature. The reaction solution was added dropwise to 150 mL of water to quench the reaction, followed by extraction with ethyl acetate (60 mL × 3). The organic phases were combined, washed with saturated brine, dried over anhydrous sodium sulfate, filtered, and concentrated to give 1.430 g of Compound 1b (yield: 93%).

MS (ESI): m/z 245.1 [M+H]⁺.

### Step 2)

Compound 1b (1.3 g, 5.32 mmol), acetonitrile (26 ml), and 2,6-dimethylpyridine (228 mg, 2.13 mmol) were added to a 50 mL three-necked flask. The mixture was purged with nitrogen three times, and then cooled to about 5°C. Carbon tetrabromide (3.0 g, 9.05 mmol) was added, and the reaction was stirred further at room temperature until the reaction was complete. The reaction mixture was concentrated under reduced pressure, and 60 mL of water was added. The mixture was extracted with ethyl acetate (30 mL × 3). The organic phases were combined, washed with saturated brine, dried over anhydrous sodium sulfate, filtered, concentrated, and purified by column chromatography (EA/PE = 1 : 3) to give 1.410 g of Compound 1c (yield: 86%).

MS (ESI): m/z 307.1 [M+H]⁺.

### Step 3)

Compound 1c (1.4 g, 4.56 mmol), acetonitrile (28 mL), morpholine (476 mg, 5.47 mmol), and potassium carbonate (945 mg, 6.84 mmol) were added to a 50 mL three-necked flask. The mixture was purged with nitrogen three times, and the reaction was stirred at room temperature until the reaction was complete. The reaction mixture was concentrated under reduced pressure, and 30 mL of water was added. The mixture was extracted with ethyl acetate (30 mL × 3). The organic phases were combined, dried, filtered, and concentrated to give 1.390 g of Compound 1d (yield: 97%).

MS (ESI): m/z 314.1 [M+H]⁺.

### Step 4)

Compound 1d (1.39 g, 4.44 mmol) and 4 N HCl/dioxane (15 mL) were added to a 50 mL three-necked flask, and the reaction was stirred at room temperature until the reaction was complete. The reaction mixture was concentrated under reduced pressure to give 1.26 g of Compound 1e (yield: 99%).

MS (ESI): m/z 214.1 [M+H]⁺.

### Step 5)

Compound 1e (1.26 g, 4.04 mmol), DMF (12 mL), Compound 1f (1.0 g, 6.06 mmol, purchased from Adamas), and DIPEA (2.840 g, 22.01 mmol) were added to a 100 mL three-necked flask. The mixture was purged with nitrogen three times and cooled to about 5°C, followed by the addition of HATU (2.510 g, 6.6 mmol). The reaction was stirred at room temperature until the reaction was complete. The reaction solution was added dropwise to 60 mL of saturated sodium bicarbonate solution, followed by extraction with ethyl acetate (30 mL × 3). The organic phases were combined, washed with saturated brine, dried over anhydrous sodium sulfate, filtered, concentrated, and purified by column chromatography (DCM/MeOH = 5/1) to give 1.060 g of Compound 1g (yield: 66%).

MS (ESI): m/z 363.1 [M+H]⁺.

### Step 6)

Compound 1g (1.060 g, 2.92 mmol), methanol (30 mL), and palladium on carbon (202 mg, 10% Pd/C) were added to a 100 mL single-neck flask under nitrogen atmosphere. The mixture was purged with hydrogen from a hydrogen balloon three times, and the reaction was then stirred at room temperature under hydrogen atmosphere until the reaction was complete. The reaction mixture was filtered and concentrated to give 0.961 g of Compound 1h (yield: 98%).

MS (ESI): m/z 333.1 [M+H]⁺.

### Step 7)

Compound 1h (0.9 g, 2.71 mmol), ethanol (18 mL), potassium hydroxide (0.152 g, 2.71 mmol), and carbon disulfide (0.206 g, 2.71 mmol) were added to a 50 mL single-neck flask. The reaction was heated until the reaction was complete. At room temperature, the reaction solution was added to saturated sodium bicarbonate solution and ethyl acetate (20 mL/30 mL), and the mixture was filtered to give 0.657 g of crude (yield: 64%).

MS (ESI): m/z 375.1 [M+H]⁺.

### Step 8)

Compound 1i (0.430 g, 1.15 mmol), acetonitrile (43 mL), and sulfonolactone (0.475 g, 3.44 mmol) were added to a 100 mL three-necked flask. The mixture was purged with nitrogen three times, and the reaction was stirred under heating until the reaction was complete. The reaction mixture was filtered. The filter cake was dissolved in methanol (10 mL). Concentrated sulfuric acid was added, and the reaction was stirred under oil bath heating until the reaction was complete. The reaction mixture was cooled to room temperature, exchanged with a chloride ion exchange resin, and purified by column chromatography (eluting with methanol) to give 0.356 g of Compound 1j (yield: 71%).

MS (ESI): m/z 433.1 [M-Cl⁻]⁺.

### Step 9)

Compound 1k (0.055 g, 0.115 mmol, prepared by the method disclosed in patent WO 2014005122 A2), dichloromethane (2 mL), and DMF were added to a 50 mL three-necked flask. The mixture was purged with nitrogen three times and cooled to 5°C. Then, a solution of oxalyl chloride in dichloromethane (117 mg of oxalyl chloride dissolved in 1 mL of dichloromethane) was added dropwise. After completion of dropwise addition, stirring was continued until the reaction was complete. The reaction mixture was concentrated under reduced pressure and dissolved in 1 mL of dichloromethane. The solution was then added dropwise to a solution of Compound 1j (0.050 g, 0.115 mmol) in acetonitrile (5 mL) cooled in an ice-water bath. The reaction was stirred at room temperature until the reaction was complete. The reaction mixture was concentrated and purified by preparative HPLC to give 0.021 g of Compound 1 (yield: 20%).

MS (ESI): m/z 449.7 [(M-2CF₃COO⁻)/2]⁺.

¹H NMR (400 MHz, CDCl₃) δ8.07-8.05 (m, 1H), 7.97-7.96 (m, 1H) , 7.66-7.64 (m, 1H), 7.48-7.46 (m, 1H), 7.32-7.30 (m, 1H), 6.83-6.76 (m, 4H), 6.65-6.64 (m, 2H), 6.01 (s, 2H), 5.65 (s, 1H), 5.35-5.33 (m, 1H),4.91-4.89 (m, 2H), 4.68-4.66 (m, 1H), 4.21-4.14 (m, 4H), 4.10-3.86 (m, 2H), 3.85 (s, 3H), 3.84-3.76 (m, 2H), 3.78 (s, 6H), 3.70-3.40 (m, 8H), 3.39 (s, 4H), 2.40-2.10 (m, 2H), 2.03-1.92 (m, 4H), 1.40-1.20 (m, 4H).

### Example 2. Preparation of Compound 2

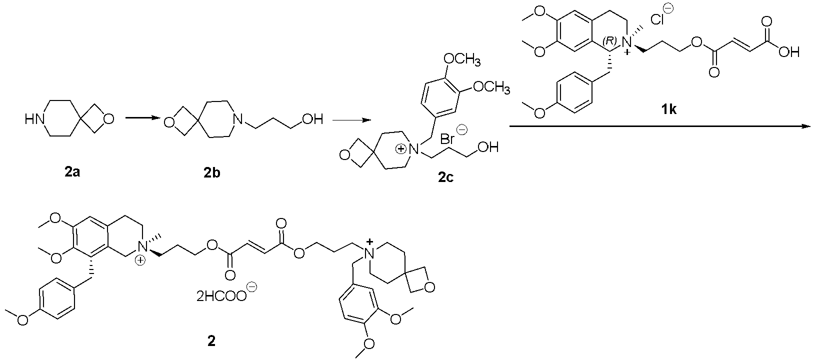

### Step 1)

In a 50 mL reaction flask, compound 2a (300 mg, 1.74 mmol, purchased from Leyan) was weighed, and acetonitrile (6 mL), potassium carbonate (721 mg, 5.22 mmol), and 3-bromopropanol (605 mg, 4.36 mmol) were added. The mixture was heated in an oil bath under nitrogen atmosphere until the reaction was complete. The reaction mixture was filtrated, concentrated, and purified by column chromatography (MeOH/DCM = 0-8%) to give 290 mg of Compound 2b (yield: 90%).

MS (ESI): m/z 186.2 [M+H]⁺.

### Step 2)

Compound 2b (290 mg, 1.57 mmol), acetonitrile (30 mL), potassium carbonate (648 mg, 4.70 mmol), and 3,4-dimethoxybenzyl bromide (434 mg, 1.88 mmol, purchased from Acon) were added to a 100 mL reaction flask. The mixture was heated in an oil bath under nitrogen atmosphere until the reaction was complete. The reaction mixture was filtered, concentrated, and purified by column chromatography (MeOH/DCM = 0-8%) to give 360 mg of Compound 2c (yield: 68%).

¹H NMR (400MHz, DMSO-*d₆*) *δ* 7.08-7.03 (m, 3H), 4.88-4.87 (t, 1H), 4.47 (s, 2H), 4.39 (s, 2H), 4.32 (s, 2H), 3.79-3.78 (d, 6H), 3.53-3.49 (m, 2H), 3.44-3.42 (m, 1H), 3.24-3.20 (m, 6H), 2.24-2.20 (m, 2H), 2.14-2.08 (m, 2H), 1.98-1.95 (m, 1H).

MS (ESI): m/z 336.1 [M-Br⁻]⁺.

### Step 3)

In a 50 mL reaction flask, Compound 2c (200 mg, 0.60 mmol) was dissolved in ultra-dry N,N-dimethylformamide (5 mL), and Compound 1k (288 mg, 0.60 mmol), diisopropylethylamine (307 mg, 2.38 mmol), 4-dimethylaminopyridine (73 mg, 0.60 mmol), and HATU (339 mg, 0.8925 mmol) were added. The mixture was purged with nitrogen three times under nitrogen atmosphere, and the reaction was stirred at room temperature until the reaction was complete. The reaction solution was purified by HPLC (ammonium formate buffer system) to give 36 mg of Compound 2 (yield: 7.5%).

MS (ESI): m/z 402.3 [(M-2HCOO⁻)/2]⁺.

¹H NMR (400 MHz, DMSO-*d₆*) *δ* 8.45 (s, 2H), 7.05-6.98 (m, 5H), 6.87-6.85 (d, 2H), 6.82 (s, 1H), 6.66-6.65 (d, 2H), 5.55 (s, 1H), 4.64-4.62 (d, 1H), 4.50 (s, 2H), 4.40 (s, 2H), 4.33 (s, 2H), 4.25 (s, 2H), 4.18 (s, 2H), 3.94-3.90 (m, 1H), 3.78-3.76 (d, 6H), 3.72-3.70 (d, 6H), 3.62-3.59 (d, 2H), 3.34 (s, 4H), 3.30 (s, 4H), 3.20 (s, 5H), 3.11 (s, 2H), 2.91-2.85 (t, 1H), 2.22 (s, 8H).

### Example 3. Preparation of Compound 3

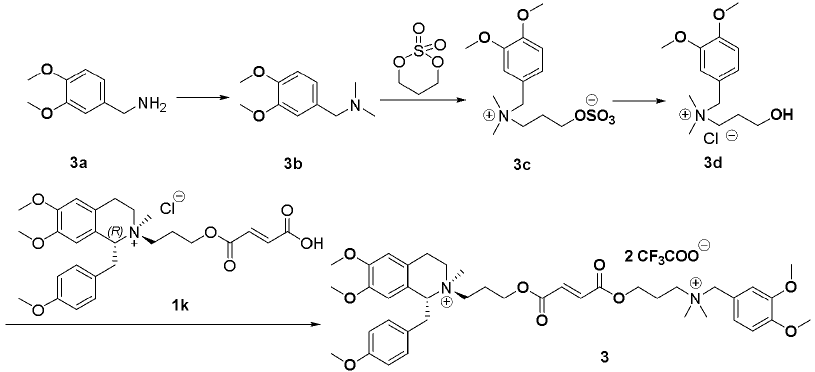

### Step 1)

In a 250 mL reaction flask, Compound 3a (2.2 g, 13.16 mmol, purchased from Bidepharm) was weighed, and methanol (40 mL) and aqueous formaldehyde solution (2.37 g, 78.95 mmol, 37% w/w) were added. Sodium triacetoxyborohydride (13.94 g, 65.79 mmol) was added portionwise, and the reaction was stirred at room temperature until the reaction was complete. The reaction mixture was concentrated, and saturated sodium bicarbonate solution was added to adjust the pH to more than 7, followed by extraction with dichloromethane (50mL × 3). The organic phases were combined, dried, subjected to suction filtration, and concentrated. The mixture was purified by column chromatography (DCM/MeOH = 7/1) to give 2.25 g of Compound 3b (yield: 87.58%).

MS (ESI): m/z 196.2 [M+H]⁺.

¹H NMR (400 MHz, CDCl₃) *δ* 6.88 (d, 1H), 6.80 (m, 2H), 3.89 (s, 3H), 3.87 (s, 3H), 3.36 (s, 2H), 2.23 (s, 6H).

### Step 2)

In a 100 mL reaction flask, Compound 3b (1.2 g, 6.15 mmol) was weighed, and acetonitrile (12 mL) was added, followed by the addition of propylene sulfate (1.70 g, 12.29 mmol, purchased from Bidepharm). The reaction was heated until the reaction was complete. At room temperature, tert-butyl methyl ether (50 mL) was added for slurrying. The mixture was filtered and dried to give 1.8 g of Compound 3c (yield: 88%).

MS (ESI): m/z 334.2 [M+H]⁺.

¹H NMR (400 MHz, DMSO-d₆) *δ* 7.12-7.05 (m, 3H), 4.43 (s, 2H), 3.84 (t, 2H), 3.80 (s, 6H), 3.38-3.31 (m, 2H), 2.93 (s, 6H), 2.13-2.07 (m, 2H).

### Step 3)

In a 50 mL reaction flask, Compound 3c (1.01 g, 3.00 mmol) and concentrated sulfuric acid (23.52 mg, 239.95 µmol) were weighed, and methanol (10 mL) was added. The reaction was heated until the reaction was complete. The reaction solution was cooled to room temperature, exchanged with a chloride ion exchange resin, concentrated, and lyophilized to give 0.78 g of Compound 3d (yield: 102.24%).

MS (ESI): m/z 254.2 [M-Cl⁻]⁺.

### Step 4)

In a 50 mL reaction flask, Compound 1k (450 mg, 0.93 mmol), Compound 3d (283 mg, 1.12 mmol), HATU (530 mg, 1.4 mmol), and 4-methylpyridine (172 mg, 1.4 mmol) were weighed, and N,N-dimethylformamide (10 mL) was added. The reaction was stirred at room temperature until the reaction was complete. The reaction solution was purified by preparative HPLC (trifluoroacetic acid buffer system) and lyophilized to give 205 mg of Compound 3 (yield: 30.6%).

MS (ESI): m/z 360.4 [(M-2CF₃COO⁻)/2]⁺.

¹H NMR (400 MHz, DMSO-*d₆*) *δ* 7.32 - 6.98 (m, 6H), 6.87 - 6.82 (m, 3H), 6.66 (s, 2H), 5.55 (s, 1H), 4.65 - 4.62 (m, 1H), 4.45 (s, 1H), 4.27 - 4.24 (m, 2H), 4.19 - 4.12 (m, 2H), 3.93 - 3.88 (m, 1H), 3.78 (d, J=2.4Hz, 6H), 3.72 (d, J=2.8Hz, 6H), 3.67 - 3.65 (m, 2H), 3.51 - 3.34 (m, 6H), 3.2 (S, 3H), 3.11 - 3.07 (m, 2H), 2.96 (S, 6H), 2.91 -2.85 (m, 1H), 2.85 -2.06 (m, 4H).

### Example 4. Preparation of Compound 4

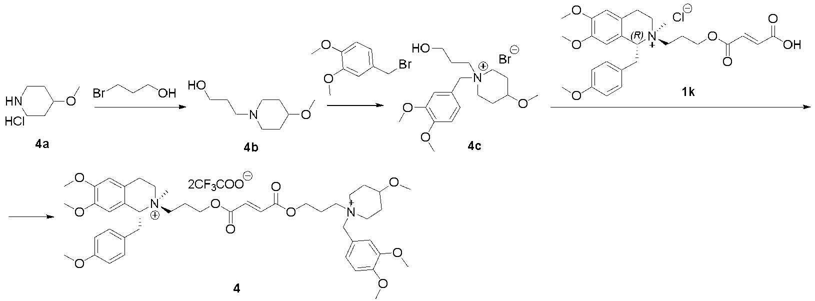

### Step 1)

In a 100 mL reaction flask, Compound 4a (2.00 g, 13.19 mmol, purchased from Leyan), potassium carbonate (5.47 g, 39.57 mmol), and potassium iodide (218 mg, 1.31 mmol) were weighed, acetone (40 ml) was added, and 3-bromopropanol (1.65 g, 11.87 mmol) was added dropwise. The mixture was heated in an oil bath until the reaction was complete. The reaction mixture was subjected to suction filtration. The filtrate was concentrated and purified by column chromatography (MeOH/DCM = 1:10) to give 2.17 g of Compound 4b (yield: 94%).

MS (ESI): m/z 174.3 [M+H]⁺.

### Step 2)

In a 100 mL reaction flask, Compound 4b (1.00 g, 5.77 mmol) was weighed and dissolved in anhydrous acetonitrile (40 mL), and 3,4-dimethoxybenzyl bromide (1.33 g, 5.77 mmol) was added. The mixture was heated in an oil bath until the reaction was complete. The filtrate was concentrated and purified by column chromatography (MeOH/DCM = 1 : 10) to give 1.29 g of Compound 4c (yield: 55%).

MS (ESI): m/z 324.1 [M-Br⁻]⁺.

¹H NMR (400 MHz, CD₃OD) *δ* 7.20-7.15 (m, 2H), 7.10-7.05 (m, 1H), 4.60 (s, 1H), 4.52 (s, 1H), 3.91-3.87 (m, 6H), 3.76-3.67 (m, 2H), 3.63-3.58 (m, 1H), 3.57-3.45 (m, 3H), 3.45-3.38 (m, 4H), 3.38-3.35 (m, 3H), 2.39-2.26 (m, 1H), 2.21-2.06 (m, 3H), 2.05-1.92 (m, 2H).

### Step 3)

In a 50 mL reaction flask, Compound 1k (500 mg, 961.50 µmol), Compound 4c (584 mg, 1.44 µmol), and HATU (549 mg, 1.44 mmol) were weighed and dissolved in anhydrous DMF (10 mL), and 4-methylpyridine (177 mg, 1.45 mmol) was added. The reaction was stirred at room temperature until the reaction was complete. The reaction solution was purified by preparative HPLC (trifluoroacetic acid buffer system) to give 513 mg of Compound 4 (yield: 52%).

MS (ESI): m/z 395.4 [(M-2CF₃COO⁻)/2]⁺.

¹H NMR (400 MHz, DMSO-*d*₆) *δ* 7.14-6.95 (m, 5H), 6.90-6.83 (m, 2H), 6.82 (s, 1H), 6.72-6.60 (m, 2H), 5.54 (s, 1H), 4.64-4.58 (m, 1H), 4.54-4.48 (m, 2H), 4.30-4.23 (m, 2H), 4.18-4.12 (m, 2H), 3.80-3.75 (m, 6H), 3.74-3.69 (m, 6H), 3.68-3.55 (m, 3H), 3.52-3.27 (m, 11H), 3.27-3.21 (m, 4H), 3.20 (s, 3H), 3.14-3.05 (m, 2H), 2.92-2.83 (m, 1H), 2.26-2.15 (m, 4H), 2.14-1.77 (m, 4H).

### Example 5. Preparation of Compound 5

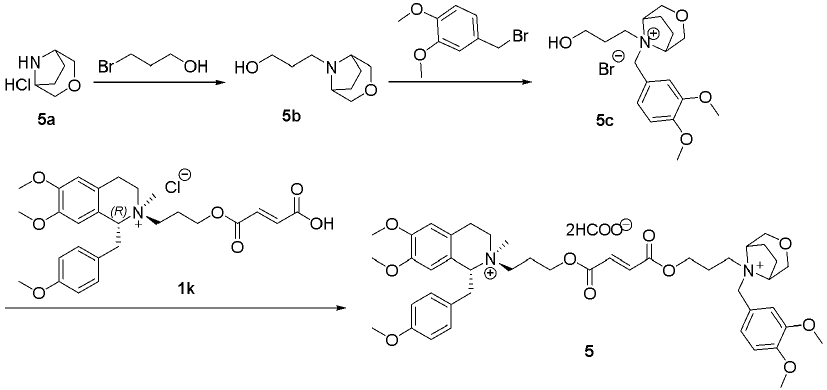

### Step 1)

In a 50 mL reaction flask, Compound 5a (1.00 g, 6.68 mmol, purchased from Leyan), potassium carbonate (2.77 g, 20.05 mmol), and potassium iodide (111 mg, 668.66 µmol) were weighed, and acetone (10 ml) and 3-bromopropanol (0.929 g, 6.68 mmol) were added. The mixture was stirred under heating until the reaction was complete. The reaction mixture was subjected to suction filtration. The filtrate was concentrated and purified by column chromatography (MeOH/DCM = 0-8%) to give 1.02 g of Compound 5b (yield: 89%).

MS (ESI): m/z 172.2 [M+H]⁺.

### Step 2)

In a 50 mL reaction flask, Compound 5b (1.00 g, 5.84 mmol) was weighed, and acetonitrile (10 mL) and 3,4-dimethoxybenzyl bromide (1.48 g, 6.42 mmol) were added. The mixture was stirred under heating until the reaction was complete. The reaction solution was concentrated and purified by column chromatography (MeOH/DCM = 0-8%) to give 1.26 g of Compound 5c (yield: 53%).

MS (ESI): m/z 322.3 [M-Br⁻]⁺.

¹H NMR (400 MHz, DMSO-*d*₆) *δ* 7.17-6.98 (m, 3H), 5.09-4.64 (m, 2H), 4.44-4.20 (m, 3H), 4.02-3.55 (m, 13H), 3.10-2.52 (m, 2H), 2.38-1.80 (m, 5H).

### Step 3)

In a 50 mL reaction flask, Compound 1k (450 mg, 928.66 µmol), Compound 5c (449 mg, 1.12 µmol), HATU (530 mg, 1.39 mmol), DMAP (171 mg, 1.40 mmol), and anhydrous DMF (10 mL) were weighed, and the mixture was stirred at room temperature until the reaction was complete. The reaction solution was purified by preparative HPLC (ammonium formate buffer system) to give 280 mg of Compound 5 (yield: 34%).

MS (ESI): m/z 394.4 [(M-2HCOO⁻)/2]⁺.

¹H NMR (400 MHz, DMSO-*d*₆) *δ* 8.46 (s, 2H), 7.11-6.89 (m, 5H), 6.88-6.79 (m, 3H), 6.69-6.53 (m, 2H), 5.55 (s, 1H), 4.70-4.41 (m, 3H), 4.36-4.23 (m, 3H), 4.19-4.11 (m, 2H), 4.07-3.89 (m, 3H), 3.79 (s, 3H), 3.75 (s, 3H), 3.73-3.68 (m, 7H), 3.64-3.55 (m, 4H), 3.35-3.30 (m, 4H), 3.20 (s, 3H), 3.13-3.01 (m, 3H), 2.91-2.84 (m, 1H), 2.70-2.55 (m, 2H), 2.38-2.25 (m, 4H), 2.24-1.94 (m, 4H).

### Example 6. Preparation of Compound 6

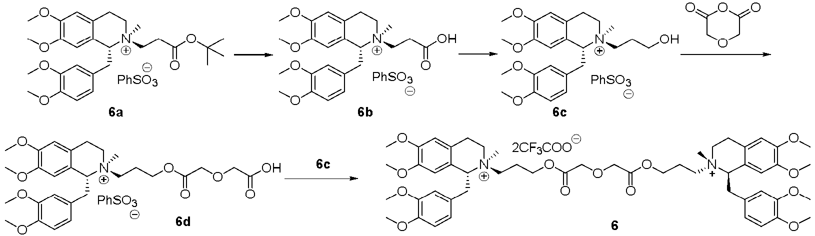

### Step 1)

In a 50 mL reaction flask, Compound 6a (600 mg, 931.98 µmol, prepared by the method disclosed in patent WO 2008/132746 A1) was weighed, and dichloromethane (10 ml) and trifluoroacetic acid (10 ml) were added. The reaction was stirred at room temperature until the reaction was complete. The mixture was concentrated to give 0.53 g of Compound 6b (yield: 96%).

MS (ESI): m/z 430.3 [M-PhSO₃⁻]⁺.

### Step 2)

In a 50 mL reaction flask, Compound 6b (0.53 g, 901.85 µmol) was weighed, and tetrahydrofuran (5 mL) and borane-tetrahydrofuran solution (1.0 M, 10 ml) were added. The reaction was stirred at room temperature until the reaction was complete. The mixture was concentrated and purified by column chromatography (ACN/H₂O = 1 : 10) to give 503 mg of Compound 6c (yield: 97%).

MS (ESI): m/z 416.3 [M-PhSO₃⁻]⁺.

### Step 3)

In a 50 mL reaction flask, Compound 6c (250 mg, 901.85 µmol) was weighed, and acetonitrile (8 mL), 1,4-dioxane-2,6-dione (253 mg, 2.18 mmol, purchased from Accela ChemBio), and triethylamine (221 mg, 2.18 mmol) were added. The reaction was stirred at room temperature until the reaction was complete. The mixture was concentrated and purified by column chromatography (ACN/H₂O = 1 : 10) to give 228 mg of Compound 6d (yield: 75%).

MS (ESI): m/z 532.2 [M-PhSO₃⁻]⁺.

### Step 4)

In a 50 mL reaction flask, Compound 6d (200 mg, 289.95 µmol), Compound 6c (217 mg, 378.25 µmol), HATU (166 mg, 436.57 µmol), and N-methylpyridine (54 mg, 442.00 µmol) were weighed, and N,N-dimethylformamide (4 mL) was added. The reaction was stirred at room temperature until the reaction was complete. The reaction solution was purified by preparative HPLC (trifluoroacetic acid buffer system) to give 96 mg of Compound 6 (yield: 28%).

MS (ESI): m/z 465.3 [(M-2CF₃COO⁻)/2]⁺.

¹H NMR (400 MHz, DMSO-*d*₆) *δ* 6.87-6.82 (m, 4H), 6.69-6.62 (m, 2H), 6.55-6.51 (m, 2H), 5.68-5.63 (m, 2H), 4.67-4.58 (m, 2H), 4.13-3.94 (m, 8H), 3.91-3.81 (m, 2H), 3.74-3.69 (m, 12H), 3.66-3.61 (m, 12H), 3.34-3.28 (m, 8H), 3.26-3.22 (m, 6H), 3.14-3.03 (m, 4H), 2.92-2.84 (m, 2H), 2.21-2.07 (m, 4H).

### Example 7. Preparation of Control Compound 1

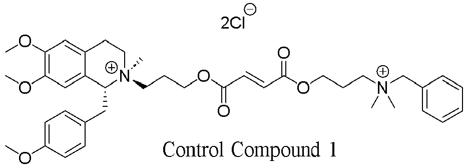

It was prepared with reference to the method disclosed in patent WO 2021115413.

### Example 8. Preparation of Compound 8

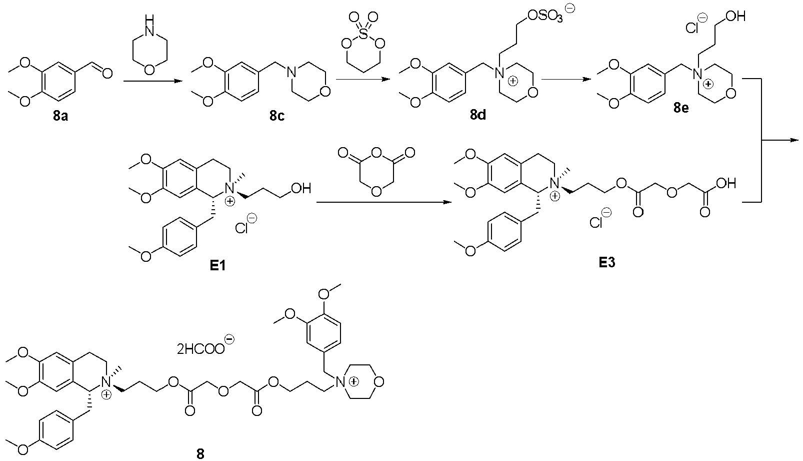

### Step 1)

In a 25 mL reaction flask, Compound E1 (2.4 g, 6.21 mmol, prepared by the method disclosed in patent WO 2021115413A1) and diglycolic anhydride (1.08 g, 9.31 mmol) were weighed, acetonitrile (25 mL) was added, and triethylamine (942.51 mg, 9.31 mmol) was added dropwise in an ice-water bath. The mixture was stirred in an ice-water bath until the reaction was complete. The reaction mixture was concentrated, diluted with dichloromethane (20 mL), and extracted with deionized water (3 × 10 mL). The aqueous phases were combined and adjusted to pH 3 with 1 N HCl. Solid sodium chloride was added to saturate the acidic aqueous solution, followed by extraction with dichloromethane/acetonitrile (5/1 v/v, 5 × 20 mL). The organic phases were combined, dried, subjected to suction filtration, concentrated, and purified by column chromatography (DCM/MeOH = 3/1) to give 1.75 g of Compound E3 (yield: 56.08%).

MS (ESI): m/z 502.2 [M-Cl⁻]⁺.

¹H NMR (400 MHz, DMSO-d₆) *δ* 7.00-6.99 (d, 2H), 6.87-6.84 (m, 3H), 5.57 (s, 1H), 5.34 (d, 1H), 4.64 (dd, 1H), 4.07-4.04 (m, 5H), 3.94-3.86 (m, 1H), 3.72 (s, 6H), 3.64-3.59 (m, 2H), 3.34-3.30 (m, 5H), 3.32 (s, 3H), 3.12-3.05 (m, 2H), 2.87 (t, 1H), 2.20-2.12 (m, 2H).

### Step 2)

In a 250 mL reaction flask, Compound 8a (5 g, 30.09 mmol, purchased from Leyan), morpholine (5.24 g, 60.18 mmol), dichloroethane (75 mL), and sodium triacetoxyborohydride (19.13 g, 90.27 mmol) were weighed, and the mixture was stirred at room temperature until the reaction was complete. 200 mL of water was added, and the mixture was extracted with dichloromethane (50 mL × 3). The organic phases were combined, dried over anhydrous sodium sulfate, subjected to suction filtration, concentrated, and purified by column chromatography (PE/EtOAc = 1/3-0/1) to give 5.2 g of Compound 8c (yield: 72.8%).

MS (ESI): m/z 238.2 [M+H]⁺.

¹H NMR (400 MHz, CDCl₃) *δ* 6.91 (d, 1H), 7.31-7.36 (m, 2H), 3.89 (s, 3H), 3.87 (s, 3H), 3.73 (t, 4H), 3.49 (s, 2H), 2.49 (s, 4H).

### Step 3)

In a 100 mL reaction flask, Compound 8c (2.5 g, 10.54 mmol) and propylene sulfate (4.37 g, 31.61 mmol) were weighed and dissolved in acetonitrile (13 mL), and the reaction was heated in an oil bath until the reaction was complete. At room temperature, methyl tert-butyl ether (50 mL) was added for slurrying to give 2.15 g of Compound 8d (yield: 54.4%).

MS (ESI): m/z 376.2 [M+H]⁺.

¹H NMR (400 MHz, DMSO-d₆) *δ* 7.15-7.04 (m, 3H), 4.57 (s, 2H), 3.98-3.93 (m, 4H), 3.86 (t, 2H), 3.82 (s, 3H), 3.80 (s, 3H), 3.50-3.44 (m, 2H), 3.40-3.36 (m, 4H), 2.18-2.10 (m, 2H).

### Step 4)

In a 100 mL reaction flask, Compound 8d (2.0 g, 5.33 mmol) and concentrated sulfuric acid (41.80 mg, 426.17 µmol) were weighed, and methanol (20 mL) was added. The mixture was heated until the reaction was complete. The reaction mixture was cooled to room temperature, exchanged with a chloride ion exchange resin, concentrated, and lyophilized to give 1.85 g of Compound 8e (yield: 117%).

MS (ESI): m/z 296.2 [M-Cl⁻]⁺.

¹H NMR (400 MHz, DMSO-d₆) *δ* 7.15-7.14 (m, 2H), 7.06 (d, 1H), 5.01 (m, 1H), 4.60 (s, 2H), 3.97-3.92 (m, 4H), 3.80 (s, 6H), 3.56-3.53 (m, 3H), 3.49-3.45 (m, 2H), 3.40-3.31 (m, 3H), 2.01-1.96 (m, 2H).

### Step 5)

In a 25 mL reaction flask, Compound E3 (0.402 g, 799.88 µmol), Compound 8e (355.60 mg, 1.20 mmol), 4-methylpyridine (146.58 mg, 1.20 mmol), and HATU (456.21 mg, 1.20 mmol) were weighed, and N,N-dimethylformamide (4 mL) was added. The mixture was stirred at room temperature until the reaction was complete. The reaction solution was purified by preparative HPLC (ammonium formate buffer system) and lyophilized to give 0.14 g of Compound 8 (yield: 22%).

MS (ESI): m/z 390.4 [(M-2HCOO⁻)/2]⁺.

¹H NMR (400 MHz, DMSO-d₆) *δ* 8.33 (s, 2H), 7.08-7.06 (m, 3H), 7.00-6.96 (m, 2H), 6.88-6.82 (m, 2H), 5.56 (s, 1H), 4.62-4.57 (m, 3H), 4.19-4.15 (m, 4H), 4.09-4.03 (m, 4H), 3.98-3.92 (m, 3H), 3.79 (s, 6H), 3.71 (s, 6H), 3.67-3.61 (m, 2H), 3.60-3.56 (m, 2H), 3.41-3.38(m, 4H), 3.36-3.33 (m, 2H), 3.32-3.28 (m, 5H), 3.21 (s, 3H), 3.12-3.06 (m, 2H), 2.91-2.84 (m, 1H), 2.22-2.14 (m, 4H).

### Example 9. Preparation of Compound 9

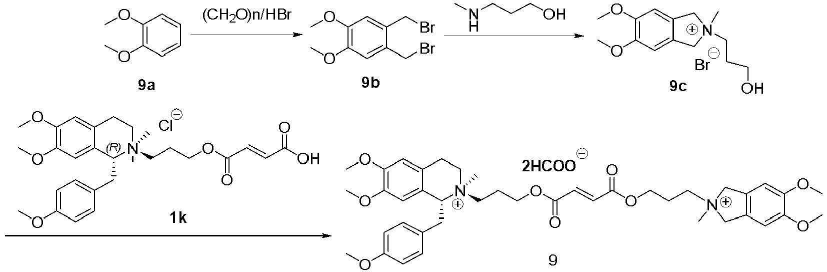

### Step 1)

In a 100 mL reaction flask, Compound 9a (5.0 g, 36.19 mmol, purchased from Bidepharm) and paraformaldehyde (6.3 g, 72.38 mmol, purchased from Titan Tech, CAS: 30525-89-4) were weighed, glacial acetic acid (20 mL) was added, and hydrobromic acid acetic acid solution (33% solution, 15 mL) was added in an ice bath. The mixture was reacted at room temperature until the reaction was complete. The reaction mixture was filtered and dried to give 5.1 g of Compound 9b (yield: 43%).

¹H NMR (400 MHz, CDCl₃-*d₆*) *δ* 6.84 (s, 2H), 4.64 (s, 4H), 3.90 (s,6H).

### Step 2)

In a 100 mL reaction flask, Compound 9b (1.5 g, 4.63 mmol), 3-(methylamino)-1-propanol (412 mg, 4.63 mmol), and N,N-diisopropylethylamine (598.0 mg, 4.63 mmol) were weighed, and dioxane (25 mL) was added. The mixture was heated in an oil bath until the reaction was complete. The reaction mixture was filtered and dried to give 1.0 g of Compound 9c (yield: 86%).

MS (ESI): m/z 252.2 [M-Br⁻]⁺.

¹H NMR (400 MHz, DMSO-*d₆*) *δ* 7.05 (s, 2H), 4.85 - 4.80 (m, 3H), 4.71 (d, J=12.4Hz, 2H), 3.77 (s, 6H), 3.67 - 3.63 (m, 2H), 3.52 - 3.46 (m, 2H), 3.18 (s, 3H), 1.89 - 1.85 (m, 2H).

### Step 3)

In a 50 mL reaction flask, Compound 1k (192 mg, 0.40 mmol), Compound 9c (121 mg, 0.48 mmol), HATU (228 mg, 0.60 µmol), and N,N-diisopropylethylamine (78 mg, 0.60 mmol) were weighed, and N,N-dimethylformamide (8 mL) was added. The reaction was stirred at room temperature until the reaction was complete. The reaction solution was purified by preparative HPLC (ammonium formate buffer system) and lyophilized to give 100 mg of Compound 9 (yield: 34.8%).

MS (ESI): m/z 359.4 [(M-2HCOO⁻)/2]⁺.

¹H NMR (400 MHz, DMSO-*d₆*) *δ* 8.50 (brs, 2H), 7.05 - 6.82 (m, 7H), 6.67 (s, 2H), 5.54 (s, 1H), 4.83 - 4.60 (m, 5H), 4.25 - 4.14 (m, 5H), 3.77 (s, 15H), 3.62 - 3.50 (m, 2H), 3.33 - 3.31 (m, 3H), 3.30 (s, 6H), 3.22 - 3.11 (m, 2H), 2.96 - 2.91 (m, 2H), 2.13 -1.98 (m,4H).

### Example 10. Preparation of Compound 10

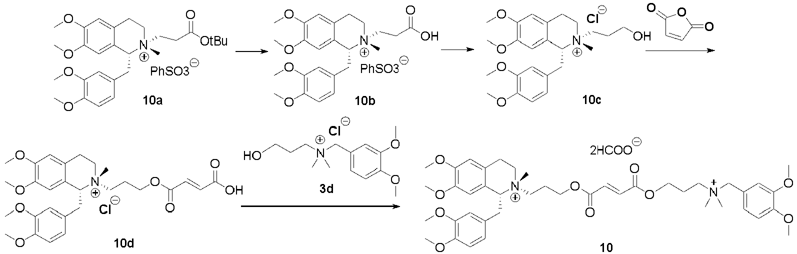

### Step 1)

In a 100 mL reaction flask, Compound 10a (1.5 g, 2.33 mmol, prepared by the method disclosed in patent WO2008/132746 A1) was weighed and dissolved in dichloromethane (15 mL), and trifluoroacetic acid (5 mL) was added. The reaction was stirred at room temperature until the reaction was complete. The reaction mixture was concentrated to give crude Compound 10b, which was used directly in the next step.

MS (ESI): m/z 430.2 [M-PhSO₃⁻]⁺.

### Step 2)

Compound 10b (1.0 g, 2.32 mmol) and tetrahydrofuran (2 mL) were added to a 100 mL reaction flask. Borane tetrahydrofuran complex (35 mL) was added at 0°C, and the mixture was stirred at room temperature until the reaction was complete. The reaction was quenched by addition of methanol with stirring. The resulting mixture was concentrated, purified by column chromatography (DCM : MeOH = 30 : 1-6 : 1), then concentrated, and exchanged with a chloride ion exchange resin to give 700 mg of Compound 10c (yield: 89%).

MS (ESI): m/z 416.2 [M-Cl⁻]⁺.

¹H NMR (400 MHz, DMSO-d₆) *δ* 6.87-6.84 (m, 2H), 6.59-6.53 (m, 2H), 5.73 (s, 1H), 4.84-4.80 (m, 1H), 3.73 (s, 3H), 3.71 (s, 3H), 3.64 (s, 3H), 3.60-3.43 (m, 8H), 3.25 (s, 3H) , 3.12-3.05 (m, 2H), 3.00 (s, 3H), 2.90-2.86 (m, 1H), 2.20-2.09 (m, 2H).

### Step 3)

In a 100 mL reaction flask, Compound 10c (1.6 g, 3.54 mmol) and maleic anhydride (0.52 g, 5.31 mmol) were dissolved in 1,2-dichloroethane (15 mL) and acetonitrile (16 mL), and triethylamine (0.39 g, 3.89 mmol) was then added. The reaction was stirred at low temperature until the reaction was complete. The reaction mixture was concentrated and purified by column chromatography (DCM : MeOH = 30 : 1-6 : 1) to give 800 mg of Compound 10d (yield: 42%).

MS (ESI): m/z 514.1 [M-Cl⁻]⁺.

¹H NMR (400 MHz, DMSO-d₆) *δ* 6.87-6.84 (m, 2H), 6.59-6.39 (m, 4H), 5.76 (s, 1H), 5.70 (s, 1H), 4.84-4.81 (m, 1H), 4.34-4.28 (m, 2H), 3.73 (s, 3H), 3.71 (s, 3H), 3.64 (s, 3H), 3.60-3.43 (m, 7H), 3.25 (s, 3H), 3.14-3.05 (m, 3H), 3.01 (s, 3H), 2.87-2.85 (m, 1H), 2.36-2.33 (m, 2H).

### Step 4)

In a 50 mL reaction flask, Compound 10d (0.2 g, 0.39 mmol) was dissolved in dichloromethane (2 mL). Oxalyl chloride (0.26 g, 2.02 mmol) was added dropwise to the solution in an ice-water bath. The reaction was stirred until the reaction was complete. The reaction mixture was concentrated and dissolved in dichloromethane (2 mL). The solution was added dropwise to a solution of Compound 3d (0.12 g, 0.43 mmol) in acetonitrile (2 mL). The resulting mixture was stirred at room temperature until the reaction was complete. The reaction mixture was concentrated. The reaction solution was purified by HPLC (ammonium formate buffer system) to give 50 mg of Compound 10 (yield: 18%).

MS (ESI): m/z 375.3 [(M-2HCOO⁻)/2]⁺.

¹H NMR (400 MHz, DMSO-d₆) *δ* 8.37 (s, 2H), 7.13-7.05 (m, 3H), 6.89-6.84 (m, 4H), 6.56-6.53 (m, 2H), 5.69 (s, 1H), 4.80-4.74 (m, 1H), 4.46 (s, 2H), 4.38-4.35 (m, 2H), 4.30-4.25 (m, 2H), 3.93 (s, 3H), 3.91 (s, 3H), 3.71 (s, 3H), 3.63 (s, 3H), 3.53-3.52 (m, 2H), 3.45-3.40 (m, 3H), 3.25 (s, 3H), 3.15-3.05 (m, 2H), 3.01 (s, 3H), 2.94 (s, 6H), 2.87-2.85 (m, 1H), 2.39-2.36 (m, 2H), 2.23-2.19 (m, 2H), 2.08 (s, 3H).

### Example 11. Preparation of Compound 11

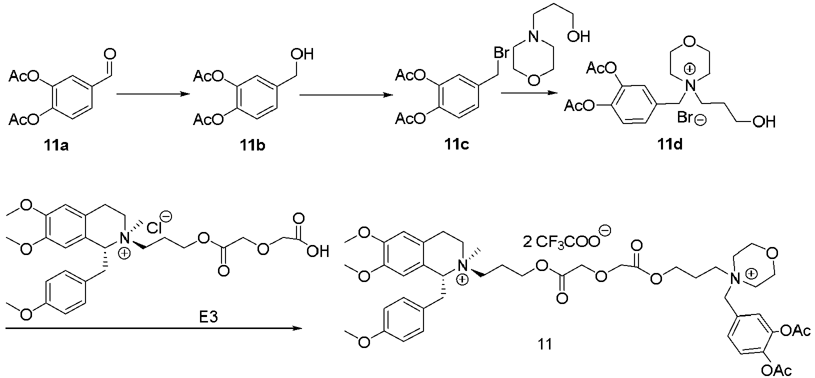

### Step 1)

In a 100 mL reaction flask, Compound 11a (1.1 g, 4.95 mmol, purchased from Energy) was weighed and dissolved in tetrahydrofuran (10 mL). Borane dimethyl sulfide solution (14.9 ml, 1.66 M) was added in an ice bath, and the mixture was reacted in an ice bath until the reaction was complete. The reaction was quenched by addition of methanol (20 ml) with stirring. The resulting mixture was concentrated and purified by column chromatography (EA/PE = 4/6) to give 1.1 g of Compound 11b (yield: 99.1%).

MS (ESI): m/z 225.2 [M+H]⁺.

¹H NMR (400 MHz, CDCl₃-*d*) *δ* 7.28 - 7.20 (m, 3H), 4.58 (s, 2H), 2.48 (brs, 1H), 2.23 (s, 6H).

### Step 2)

In a 100 mL reaction flask, Compound 11b (1.1 g, 4.91 mmol) was weighed and dissolved in dichloromethane (10 mL), and triphenylphosphine (1.67 g, 6.38 mmol) and carbon tetrabromide (2.12 g, 6.38 mmol) were added in an ice bath. The reaction was stirred at room temperature until the reaction was complete. Dichloromethane (50 ml) and water (60 ml) were added. The organic phases were separated, dried, filtered, concentrated, and purified by column chromatography (EA/PE = 3/7) to give 680 mg of Compound 11c (yield: 48.3%).

¹H NMR (400 MHz, CDCl₃-d) *δ* 7.29 - 7.24 (m, 2H), 7.16 (d, J=8.4Hz, 1H), 4.45 (s, 2H), 2.29 (s, 6H).

### Step 3)

In a 100 mL reaction flask, Compound 11c (700 mg, 2.44 mmol) and 3-(4-morpholine)-1-propanol (354 mg, 2.44 mmol, purchased from Accela ChemBio) were weighed, and acetonitrile (15 mL) was added. The mixture was heated until the reaction was complete. The reaction mixture was filtered and dried to give 620 mg of Compound 11d (yield: 72%).

MS (ESI): m/z 352.2 [M-Br⁻]⁺.

### Step 4)

In a 50 mL reaction flask, Compound E3 (156 mg, 0.31 mmol) was weighed, dichloromethane (5 mL) was added, and oxalyl chloride (119 mg, 0.94 mmol) was added in an ice bath. The reaction was stirred in an ice bath until the reaction was complete. The reaction mixture was concentrated at low temperature, dichloromethane (5 mL) was added, and the reaction solution was reserved for later use. In another 50 mL three-necked flask, Compound 11d (110 mg, 0.31 mmol) was weighed, acetonitrile (5 mL) was added, and the reaction solution was added dropwise. The reaction was stirred until the reaction was complete. The reaction mixture was concentrated at low temperature and purified by preparative HPLC (trifluoroacetic acid buffer system) to give 60 mg of Compound 11 (yield: 22.97%).

MS (ESI): m/z 418.4 [(M-2CF₃COO⁻)/2]⁺.

¹H NMR (400 MHz, DMSO-d₆) *δ* 7.50-7.44 (m, 3H), 6.98-6.96 (m, 2H), 6.87-6.84 (m, 3H), 5.56 (s, 1H), 4.71 (s,2H), 4.62-4.60 (m, 1H), 4.18-4.15 (m, 4H), 4.09 (s,2H), 4.07-4.04 (m, 2H), 3.98-3.89 (m, 5H),3.72 (s, 6H), 3.68-3.61 (m, 3H), 3.43-3.31 (m, 9H),3.22 (s, 3H), 3.10-3.06 (m, 3H), 2.90-2.85 (m, 1H), 2.30 (s, 6H), 2.21-2.14 (m, 4H).

### Example 12. Preparation of Compound 12

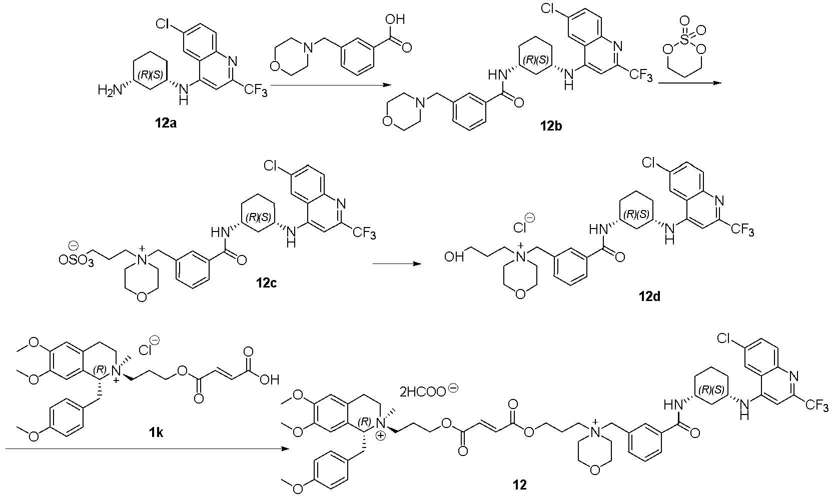

### Step 1)

In a 50 mL reaction flask, the compound 3-morpholin-4-ylmethylbenzoic acid (350 mg, 1.51 mmol, prepared by the method disclosed in patent WO 2018/169777 A1) was weighed, and HATU (600 mg, 1.51 mmol) and diisopropylethylamine (780 mg, 5.80 mmol) were added. The mixture was dissolved in N,N-dimethylformamide (4 mL), and Compound 12a (400 mg, 1.16 mmol, prepared by the method disclosed in patent WO 2022/67094 A1) was added. The mixture was stirred at room temperature until the reaction was complete. Ethyl acetate (100 mL) was added. The organic layer was washed twice with saturated sodium chloride solution, dried over anhydrous sodium sulfate, concentrated, and purified by column chromatography (PE/EA = 1/1) to give 0.59 g of Compound 12b (yield: 80%).

MS (ESI): m/z 547.20 [M+H]⁺.

### Step 2)

In a 50 mL reaction flask, Compound 12b (580.0 mg, 1.06 mmol) was weighed and dissolved in acetonitrile (3 mL), and propylene sulfate (293.0 mg, 2.12 mmol) was added. The mixture was heated until the reaction was complete. The reaction mixture was concentrated and purified by column chromatography (DCM/MeOH = 10/1-1/1) to give 0.18 g of Compound 12c (yield: 30%).

MS (ESI): m/z 685.2 [M+H]⁺.

### Step 3)

In a 50 mL reaction flask, Compound 12c (180.0 mg, 0.27 mmol) was weighed and dissolved in methanol (3 mL), and H₂SO₄ (0.1 mL) was then added. The mixture was heated until the reaction was complete. The reaction solution was exchanged with a chloride ion exchange resin and concentrated to give 0.1 g of Compound 12d (yield: 60%).

MS (ESI): m/z 605.2 [M-Cl⁻]⁺.

¹H NMR (400 MHz, CD₃OD) *δ* 8.80 (d, 1H), 8.11-8.04( m,4H), 7.82-7.66(m, 3H), 7.45-7.30 (m,2H),4.28-3.96(m, 7H), 3.77-3.75 (m, 2H), 3.78-3.52 (m,7H), 2.42-2.38 (m,1H), 2.22-2.17 (m,6H), 1.86-1.71 (m, 3H).

### Step 4)

In a 50 mL reaction flask, Compound 1k (264 mg, 0.55 mmol) was weighed and dissolved in N,N-dimethylformamide (4.4 mL), and HATU (209 mg, 0.55 mmol), diisopropylethylamine (147 mg, 1.08 mmol), and Compound 12d (220 mg, 0.36 mmol) were then added, respectively. The reaction was stirred at room temperature until the reaction was complete. The reaction solution was purified by HPLC (trifluoroacetic acid buffer system) to give 32 mg of Compound 12 (yield: 7%).

MS (ESI): m/z 535.5 [(M-2HCOO⁻)/2]⁺.

¹H NMR (400 MHz, DMSO-d₆) *δ* 8.67 (s, 1H), 8.62 (s, 1H), 8.41 (s, 2H), 8.08-8.03(m, 2H), 7.91-7.88(m, 1H), 7.75-7.54 (m, 4H), 6.99-6.62 (m, 8H), 5.54(s,1H), 4.78(s, 2H), 4.64-4.62 (m, 1H), 4.15-3.80 (m, 14H), 3.71 (s, 3H), 3.70(s, 3H), 3.61-3.58(m, 2H),3.33(s, 3H), 3.21(s, 3H), 3.10-2.84(m, 5H), 2.40-1.29(m, 15H).

### Example 13. Preparation of Compound 13

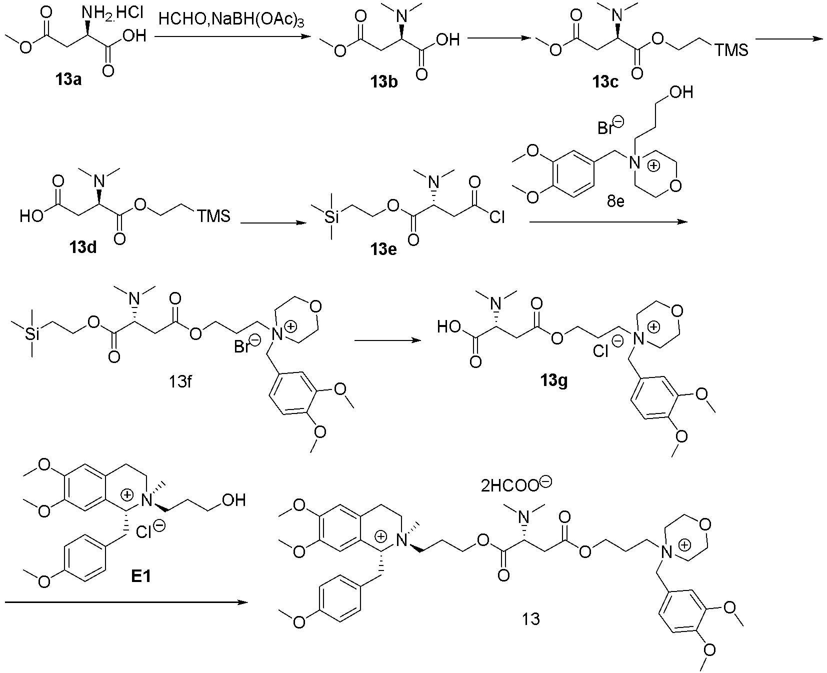

### Step 1)

In a 100 mL reaction flask, Compound 13a (5.51 g, 30 mmol, purchased from Leyan) was weighed, methanol (300 mL) was added, and aqueous formaldehyde solution (5.85 g, 195 mmol, 37%) and sodium borohydride acetate (31.8 g, 150 mmol) were added in an ice-water bath. The mixture was stirred at room temperature under nitrogen atmosphere until the reaction was complete. The reaction mixture was concentrated and purified by column chromatography (MeOH/DCM = 1 : 5) to give 4 g of Compound 13b (yield: 76%).

MS (ESI): m/z 176.2 [M+H]⁺.

¹H NMR(400MHz, CD₃OD) *δ*3.55(s, 3H), 3.38-3.35(m, 1H), 2.65-2.60(m, 1H), 2.51-2.42(m, 1H), 2.28(s, 6H).

### Step 2)

In a 100 mL reaction flask, Compound 13b (4.0 g, 22.83 mmol) was weighed and dissolved in dichloromethane (5 mL), and trimethylsilyl ethanol (4.05 g, 34.25 mmol), HATU (10.42 g, 27.40 mmol), 4-methylpyridine (2.79 g, 22.83 mmol), and diisopropylethylamine (8.85 g, 68.50 mmol, 11.93 mL) were added in an ice-water bath. The mixture was stirred at room temperature until the reaction was complete. 100 mL of water was added to the reaction flask. The mixture was extracted with dichloromethane (50 mL × 4). The organic phases were combined, dried, filtered, concentrated, and purified by column chromatography (PE/EA = 1 : 1) to give 3.2 g of Compound 13c (yield: 50.9%).

MS (ESI): m/z 276.4 [M+H]⁺.

¹H NMR (400 MHz, DMSO-d₆) *δ* 4.14-4.09 (m, 2H), 3.55(s, 3H), 3.53-3.51 (m, 1H), 2.74-2.67 (m, 1H),2.53-2.47 (m, 1H), 2.20 (s, 6H), 0.95-0.91 (m, 2H), 0.01 (s, 9H).

### Step 3)

In a 50 mL reaction flask, Compound 13c (3.2 g, 11.62 mmol) was weighed and dissolved in methanol (50 mL) and water (10 mL), and lithium hydroxide (278.85 mg, 11.62 mmol) were added in an ice-water bath. The mixture was stirred at room temperature until the reaction was complete. The reaction mixture was concentrated and purified by column chromatography (MeOH/DCM = 1 : 10) to give 2.1 g of Compound 13d (yield: 69.1%).

¹H NMR (400MHz, DMSO-d₆) *δ* 4.16-4.11(m, 2H), 3.54-3.50 (m, 1H), 2.67-2.61(m, 1H), 2.45-2.40 (m, 1H), 2.23(s, 6H), 0.99-0.95 (m, 2H), 0.03 (s, 9H).

### Step 4)

In a 25 mL reaction flask, Compound 13d (784 mg, 3.00 mmol) was weighed and dissolved in dichloromethane (20 mL), and oxalyl chloride (1.52 g, 12.00 mmol) and catalytic DMF were added dropwise in an ice-water bath. The mixture was stirred at room temperature under argon atmosphere until the reaction was complete. The reaction mixture was concentrated to give Compound 13e, which was used directly in the subsequent reaction.

### Step 5)

In a 25 mL reaction flask, Compound 8e (592.76 mg, 2 mmol) was weighed and dissolved in dichloromethane (5 mL). The mixture was stirred in an ice-water bath. A solution of Compound 13e (820 mg) in dichloromethane (5 mL) was added dropwise. The mixture was stirred at room temperature under argon atmosphere until the reaction was complete. The reaction mixture was purified by column chromatography (MeOH/DCM = 1 : 3) to give 650 mg of Compound 13f (yield: 60.2%).

MS (ESI): m/z 539.2 [M-Br⁻]⁺.

### Step 6)

In a 25 mL reaction flask, Compound 13f (600 mg, 1.11 mmol) was weighed and dissolved in tetrahydrofuran (10 mL), and tetrabutylammonium fluoride (581.29 mg, 2.22 mmol, 1.0 M) was added in an ice-water bath. The mixture was stirred at room temperature until the reaction was complete. The reaction mixture was exchanged with a chloride ion exchange resin, concentrated, and purified by column chromatography (ACN/H₂O = 1: 3) to give 350 mg of Compound 13g (yield: 71%).

MS (ESI): m/z 439.2 [M-Cl⁻]⁺.

¹H NMR (400 MHz, DMSO-d₆) *δ* 7.15-7.11(m, 3H), 4.65 (s, 2H), 4.48-4.45(m, 1H), 4.22-4.19(m, 2H), 3.99-3.95 (m, 4H), 3.81(s, 6H), 3.80(s, 2H), 3.47-3.42 (m, 4H), 3.26-3.21 (m, 1H), 3.09-3.03 (m, 1H), 2.81 (s, 6H), 3.23-2.20 (m, 1H).

### Step 7)

In a 10 mL reaction flask, Compound 13g (300 mg, 0.68 mmol) was weighed and dissolved in N,N-dimethylformamide (10 mL), and Compound E1 (316.57 mg, 0.82 mmol), HATU (389.29 mg, 1.02 mmol), 4-methylpyridine (83.39 mg, 0.68 mmol), and diisopropylethylamine (352.86 mg, 2.73 mmol) were added in an ice-water bath. The mixture was stirred at room temperature under nitrogen atmosphere until the reaction was complete. The reaction solution was purified by preparative HPLC (ammonium formate buffer system) to give 30 mg of Compound 13 (yield: 5.4%).

MS (ESI): m/z 404.2 [(M-2HCOO⁻)/2]⁺.

¹H NMR (400 MHz, DMSO-d₆) *δ* 8.33(s, 2H),7.09-7.06(m, 3H), 6.99-6.97(m, 2H), 6.87-6.82(m, 3H), 5.57(s, 1H), 4.60(s, 3H), 4.09-3.68 (m, 29H), 33.46-3.34(m, 4H), 3.26-3.21 (m, 9H), 3.21 (s, 3H), 3.17-2.98 (m, 2H), 2.91-2.84 (m, 1H), 2.64-2.59 (m, 1H), 2.20-2.11(m, 4H),2.07-1.99 (m, 4H).

### Example 14. Preparation of Compound 14

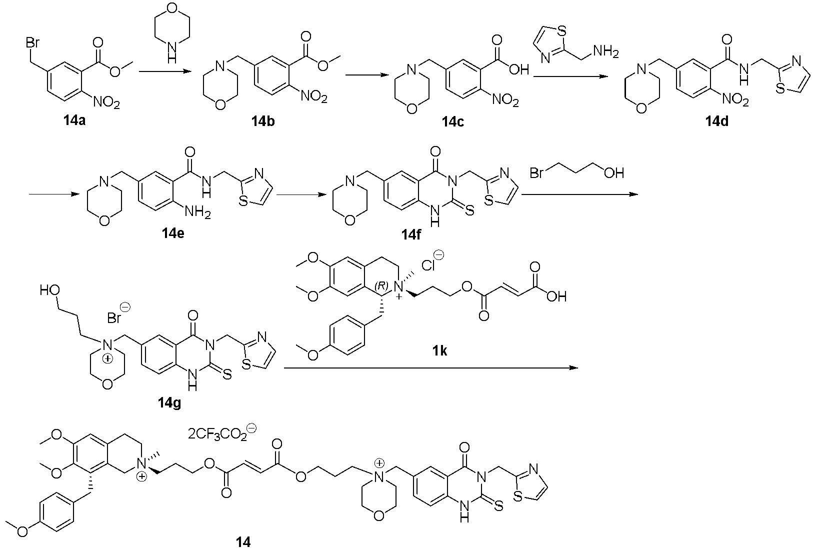

### Step 1)

In a 250 mL reaction flask, Compound 14a (10.0 g, 36.50 mmol, purchased from Jiangsu Aikon) was weighed and dissolved in anhydrous acetonitrile (100 mL), and morpholine (3.81 g, 43.76 mmol) and potassium carbonate (12.59 mg, 91.24 mmol) were added. The reaction was heated under nitrogen atmosphere until the reaction was complete. The reaction mixture was filtered, concentrated, and purified by column chromatography (MeOH/DCM = 1 : 10) to give 7.97 g of Compound 14b (yield: 78%).

MS (ESI): m/z 281.2 [M+H]⁺.

### Step 2)

In a 250 mL reaction flask, Compound 14b (7.97 g, 28.46 mmol) was weighed and dissolved in tetrahydrofuran (40 mL), and lithium hydroxide (1.022 g, 42.69 mmol) and water (40 mL) were added. The mixture was reacted at room temperature until the reaction was complete. The pH was adjusted to 5-6. The resulting mixture was concentrated, and purified by column chromatography (MeOH/DCM = 1 : 10) to give 5.26 g of Compound 14c (yield: 69%).

MS (ESI): m/z 267.2 [M+H]⁺.

### Step 3)

In a 250 mL reaction flask, Compound 14c (1.72 g, 6.47 mmol) was weighed and dissolved in ultra-dry N,N-dimethylformamide (40 mL), and 2-thiazolemethanamine (0.74 g, 6.47 mmol, purchased from Bidepharm), diisopropylethylamine (2.50 g, 19.40 mmol), and HATU (2.95 g, 7.76 mmol) were added. The mixture was purged with nitrogen three times under nitrogen atmosphere, and reacted at room temperature until the reaction was complete. Water (100 mL) was added, and the mixture was extracted with ethyl acetate (200 mL × 3). The organic phases were dried over anhydrous sodium sulfate, filtered, concentrated, and purified by column chromatography (MeOH/DCM = 1 : 10) to give 1.40 g of Compound 14d (yield: 60%).

MS (ESI): m/z 363.2 [M+H]⁺.

### Step 4)

In a 100 mL reaction flask, Compound 14d (1.27 g, 3.51 mmol) was weighed and dissolved in ethanol (20 mL) and water (5 mL), and ammonium chloride (1.92 g, 35.08 mmol) and iron powder (1.566 g, 28.065 mmol) were added. The mixture was reacted at room temperature until the reaction was complete. The reaction mixture was filtered, concentrated, and purified by column chromatography (MeOH/DCM = 1 : 10) to give 1.10 g of Compound 14e (yield: 94%).

MS (ESI): m/z 333.2 [M+H]⁺.

¹H NMR (400 MHz, DMSO-*d₆*) *δ* 9.10-9.07 (t, 1H), 7.73-7.72 (d, 1H), 7.62-7.61 (d, 1H), 7.49 (s, 2H),7.16-7.14 (d, 1H), 6.71-6.69 (d, 1H), 6.47 (s, 2H), 4.70-4.68 (d, 2H), 4.12-4.11 (m, 1H), 3.61 (s, 4H), 3.14 (s, 4H).

### Step 5)

In a 100 mL reaction flask, Compound 14e (1.10 g, 3.31 mmol) was weighed and dissolved in ethanol (20 mL), and carbon disulfide (12.60 g, 165.46 mmol) and potassium hydroxide (0.41 g, 7.28 mmol) were added. The reaction was heated until the reaction was complete. The reaction mixture was concentrated and purified by column chromatography (MeOH/DCM = 1 : 10) to give 0.88 g of Compound 14f (yield: 70%).

MS (ESI): m/z 375.3 [M+H]⁺.

¹H NMR (400 MHz, DMSO-*d₆*) *δ* 13.15 (s, 1H), 7.89-7.88 (d, 1H), 7.74-7.72 (dd, 1H), 7.69-7.68 (d, 1H), 7.62-7.63 (d, 1H), 7.41-7.39(d, 1H), 5.95 (s, 2H), 3.60-3.56 (t, 4H), 3.52 (s, 2H), 2.35 (s, 4H).

### Step 6)

In a 100 mL reaction flask, Compound 14f (0.35 g, 0.94 mmol) was weighed and dissolved in acetonitrile (20 mL), and 3-bromopropanol (0.16 g, 1.122 mmol) and potassium carbonate (0.39 g, 2.81 mmol) were added. The reaction was heated until the reaction was complete. The reaction mixture was concentrated and purified by column chromatography (MeOH/DCM = 1: 10) to give 0.38 g of Compound 14g (yield: 94%).

MS (ESI): m/z 433.3 [M-Br⁻]⁺.

¹H NMR (400 MHz, DMSO-*d₆*) *δ* 8.02-8.01 (d, 1H), 7.79-7.76 (dd, 1H), 7.73-7.72(d, 1H), 7.70-7.69 (d, 1H), 5.61 (s, 2H), 4.64-4.60 (t, 1H), 3.59-3.57 (s, 7H), 3.52-3.48 (m, 2H), 3.33-3.29 (t, 2H), 2.38 (s, 4H), 2.27-2.24 (t, 2H).

### Step 7)

In a 50 mL reaction flask, Compound 14g (200 mg, 0.46 mmol) was weighed and dissolved in ultra-dry N,N-dimethylformamide (5 mL), and Compound 1k (268 mg, 0.55 mmol), diisopropylethylamine (239 mg, 1.85 mmol), 4-dimethylaminopyridine (56 mg, 0.46 mmol), and HATU (263 mg, 0.69 mmol) were added. The mixture was purged with nitrogen three times under nitrogen atmosphere, and the reaction was stirred at room temperature until the reaction was complete. The reaction solution was purified by preparative HPLC (trifluoroacetic acid buffer system) and lyophilized to give 96 mg of Compound 14 (yield: 23%).

MS (ESI): m/z 450.3 [(M-2CF₃COO⁻)/2]⁺.

¹H NMR (400 MHz, DMSO-*d₆*) *δ* 10.06 (s, 1H), 8.31 (s, 1H), 7.89-7.87 (dd, 1H), 7.72-7.70 (m, 2H), 7.65-7.63 (d, 1H), 6.99-6.97 (d, 2H), 6.86-6.84 (d, 2H),6.81 (s, 1H), 6.64-6.63 (d, 1H), 5.63 (s, 1H), 5.55(s, 1H), 4.63-4.59 (dd, 1H), 4.48 (s, 2H), 4.28-4.25 (t, 2H), 4.19-4.17 (m, 2H), 3.95-3.87 (m, 4H), 3.72 (s, 4H), 3.70 (s, 4H), 3.65 (m, 1H), 3.61-3.57 (dd, 4H), 3.35 (s, 5H), 3.21 (s, 4H), 3.11-3.09 (d, 2H), 2.89-2.84 (t, 1H), 2.19-2.18 (m, 2H), 2.11-2.08 (t, 2H).

### Example 15. Preparation of Compounds 5A, 5B, 5C, and 5D

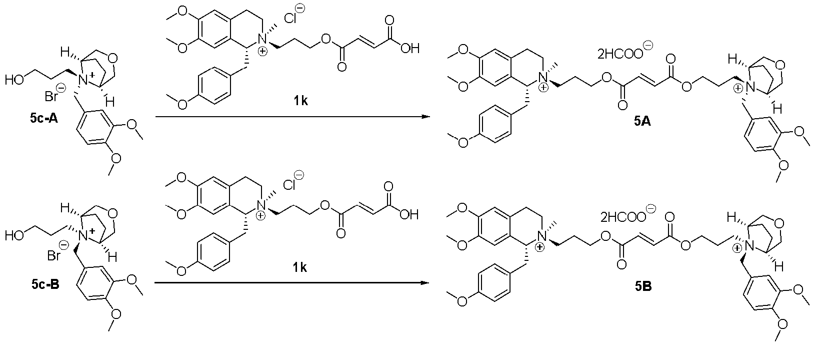

### Step 1: Chiral resolution of Compound 5c

Chiral column: ChiralCel OX, 250 × 30 mm I.D., 10 µm; Mobile phase: CO2 for A, ethanol for B; Gradient: 50% B; Flow rate: 120 mL/min; Pressure: 100 bar; Column temperature: 38°C; and Wavelength: 220 nm. The retention time of Compound 5c-A was 3.095 min, and the retention time of Compound 5c-B was 4.487 min.

5c-A: ¹H NMR (400 MHz, DMSO-*d*₆) *δ* 7.10-6.4 (m, 3H), 5.10-4.96 (m, 2H), 4.83 (s, 1H), 4.75-4.60 (m, 2H), 3.98 (s, 2H), 3.84-3.67 (m, 8H), 3.44-3.37 (m, 2H), 3.08-2.97 (m, 2H) , 2.30-2.14 (m, 4H) , 1.96-1.84 (m, 2H).

5c-B: ¹H NMR (400 MHz, DMSO-*d*₆) *δ* 7.14-7.11 (m, 2H), 7.05 (d, 1H), 5.08 (brs, 1H), 4.41(s, 2H), 4.27(d, 2H), 3.92 (brs, 2H), 3.81(s, 3H), 3.80(s, 3H), 3.63-3.56(m, 6H), 2.59-2.57(m, 2H), 2.28-2.23(m, 2H), 2.13-2.06(m, 2H).

### Step 2A: Preparation of Compound 5A

In a 50 mL reaction flask, Compound 1k (450 mg, 928.66 µmol), Compound 5c-A (449 mg, 1.12 µmol), HATU (530 mg, 1.39 mmol), DMAP (171 mg, 1.40 mmol), and anhydrous DMF (10 mL) were weighed, and the mixture was stirred at room temperature until the reaction was complete. The reaction solution was purified by preparative HPLC (ammonium formate buffer system) to give 280 mg of Compound 5A (yield: 34%).

¹H NMR (400 MHz, DMSO-*d*₆) *δ* 8.33(s, 2H), 7.05-6.90 (m, 5H), 6.89-6.80 (m, 3H), 6.54 (s, 2H), 5.55 (s, 1H), 5.06 (s, 2H), 4.73-4.59 (m, 3H), 4.23-4.11 (m, 4H), 4.10-4.01 (m, 2H), 4.00-3.83 (m, 2H), 3.76 (s, 3H), 3.70 (s, 6H), 3.67-3.56 (m, 3H), 3.42-3.32 (m, 5H), 3.22-3.18 (m, 3H), 3.14-3.00 (m, 4H), 2.92-2.84 (m, 1H), 2.37-2.08 (m, 8H).

MS (ESI): m/z 394.4 [(M-2HCOO⁻)/2]⁺.

Retention time: 11.923 min.

### Step 2B: Preparation of Compound 5B

In a 50 mL reaction flask, Compound 1k (450 mg, 928.66 µmol), Compound 5c-B (449 mg, 1.12 µmol), HATU (530 mg, 1.39 mmol), DMAP (171 mg, 1.40 mmol), and anhydrous DMF (10 mL) were weighed, and the mixture was stirred at room temperature until the reaction was complete. The reaction solution was purified by preparative HPLC (ammonium formate buffer system) to give 280 mg of Compound 5B (yield: 34%).

MS (ESI): m/z 394.4 [(M-2HCOO⁻)/2]⁺.

¹H NMR (400 MHz, DMSO-d₆) *δ* 8.33(s, 2H), *δ* 7.10-6.96 (m, 5H), 6.88-6.80 (m, 3H), 6.70-6.58 (m, 2H), 5.55 (s, 1H), 4.65-4.58 (m, 1H), 4.43 (s, 2H), 4.36-4.21 (m, 4H), 4.18-4.11 (m, 2H), 4.01-3.86 (m, 3H), 3.78 (s, 3H), 3.75 (s, 3H), 3.71 (s, 3H), 3.70 (s, 3H), 3.63-3.54 (m, 5H), 3.48-3.35 (m, 6H), 3.23-3.17 (m, 3H), 3.13-3.04 (m, 2H), 2.92-2.83 (m, 1H), 2.64-2.53 (m, 2H), 2.37-2.24 (m, 4H), 2.24-2.16 (m, 2H).

Retention time: 12.137 min.

### Step 2C: Preparation of Compound 5C

100 mg of Compound 5A was subjected to ion exchange with 2 g Dowex^{®}1×8 chloride form resin using methanol as eluent for a total of five times. Concentration gave 72 mg of Compound 5C (yield: 72%).

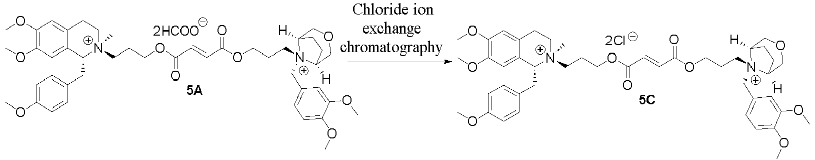

MS (ESI): m/z 394.4 [(M-2Cl⁻)/2]⁺.

¹H NMR (400 MHz, DMSO-*d*₆) *δ* 7.03-6.90 (m, 5H), 6.89-6.80 (m, 3H), 6.55 (s, 2H), 5.55 (s, 1H), 5.05 (s, 2H), 4.70-4.57 (m, 3H), 4.23-4.11 (m, 4H), 4.07-4.01 (m, 2H), 4.00-3.83 (m, 2H), 3.76 (s, 3H), 3.72 (s, 6H), 3.70 (s, 3H), 3.70-3.56 (m, 3H), 3.42-3.32 (m, 5H), 3.22-3.18 (m, 3H), 3.14-3.00 (m, 4H), 2.92-2.84 (m, 1H), 2.31-2.06 (m, 8H).

### Step 2D: Preparation of Compound 5D

200 mg of Compound 5B was subjected to ion exchange with 5 g Dowex^{®}1×8 chloride form resin using methanol as eluent for a total of five times. Concentration gave 160 mg of Compound 5D (yield: 80%).

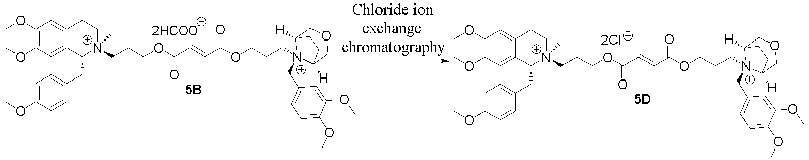

MS (ESI): m/z 394.4 [(M-2Cl⁻)/2]⁺.

¹H NMR (400 MHz, DMSO-d₆) *δ*7.10-7.08 (m, 1H), 7.03-6.98 (m, 4H), 6.88-6.83 (m, 3H), 6.68 (d, 1H), 6.62 (d, 1H), 5.55 (s,1H), 4.69-4.63 (m,1H), 4.46 (s,2H), 4.33-4.30 (m, 3H), 4.27 (s, 1H), 4.18-4.12 (m, 2H), 4.00 (brs, 2H), 3.99-3.96 (m, 1H), 3.79 (s, 3H), 3.76 (s, 3H), 3.72 (s, 3H), 3.71 (s, 3H), 3.63-3.55 (m, 5H), 3.38-3.44 (m, 2H), 3.36 (s, 3H), 3.32-3.30 (m, 1H), 3.23 (s, 3H), 3.13-3.02 (m, 2H), 2.88 (t, 1H), 2.63-2.54 (m, 2H), 2.36-2.21 (m, 6H).

### Example 16. Preparation of Control Compound 2

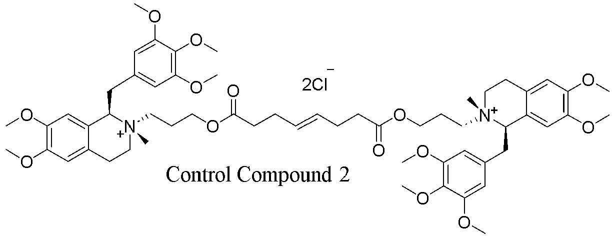

It was prepared with reference to the method disclosed in US 4761418 A.

### Example 17. Preparation of Control Compound 3

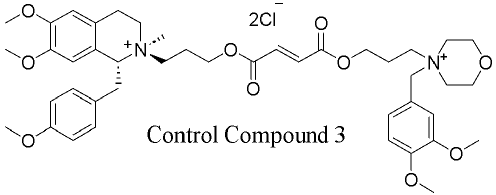

It was prepared with reference to the method disclosed in the reference *(*Anesthesiology November 2018, Vol. 129, 970-988*.).*

### Example 18. Preparation of Compound 15

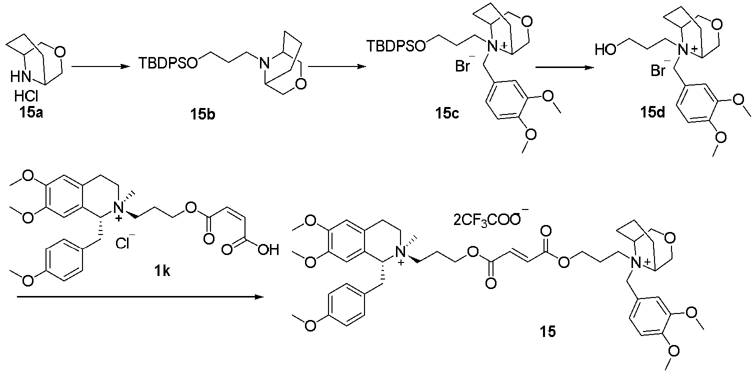

### Step 1): Preparation of Compound 15b

Compound 15a (950 mg, 5.81 mmol, purchased from Jiangsu Aikon) was weighed and dissolved in acetone (19 mL), and potassium carbonate (1.60 g, 11.61 mmol), and sodium iodide (87 mg, 580.41 µmol) and (3-bromopropoxy)(tert-butyl)diphenylsilane (2.19 g, 5.81 mmol, prepared by the well-known method "Angewandte Chemie-International Edition, 2015, vol. 54, # 51, p. 15497-15500") were added with stirring at room temperature. After addition, the mixture was stirred at 65°C for 18 hours. The reaction solution was filtered, spun dry, and purified by column chromatography (0-25% EA in PE) to give Compound 15b (2.29 g, yield: 93%).

MS (ESI): m/z = 424.4 [M+1]⁺.

¹H NMR (400 MHz, CD₃OD) *δ* 7.69-7.64 (m, 4H), 7.45-7.35 (m, 6H), 3.92-3.86 (m, 2H), 3.78-3.71 (m, 4H), 2.85-2.79 (m, 2H), 2.52 (s, 2H), 2.46-2.32 (m, 1H), 2.09-1.98 (m, 2H), 1.73-1.64 (m, 2H), 1.61-1.53 (m, 1H), 1.51-1.43 (m, 2H), 1.04 (s, 9H).

### Step 2): Preparation of Compound 15c

Compound 15b (1.00 g, 2.36 mmol) was weighed and dissolved in anhydrous acetonitrile (10 mL), and 3,4-dimethoxybenzyl bromide (818 mg, 3.54 mmol) was added. After addition, the mixture was stirred at 80°C for 2 hours. The reaction solution was spun dry and purified by column chromatography (0-4% MeOH in DCM) to give Compound 15c (1.08 g, yield: 79%).

MS (ESI): m/z = 574.5 [M-Br⁻]⁺.

### Step 3): Preparation of Compound 15d

Compound 15c (1.06 g, 1.62 mmol) was weighed and dissolved in methanol (10 mL), and a 4.0 M HCl/dioxane solution (10 mL) was added. After addition, the mixture was stirred at room temperature for 3 hours. The reaction solution was spun dry and purified by column chromatography (0-15% MeOH in DCM) to give Compound 15d (561 mg, yield: 83%).

MS (ESI): m/z = 336.3 [M-Br⁻]⁺.

¹H NMR (400 MHz, CD₃OD) *δ* 7.19-7.09 (m, 2H), 7.08-7.03 (m, 1H), 5.04 (s, 1H), 4.94-3.98 (m, 6H), 3.91-3.84 (m, 6H), 3.75-3.46 (m, 6H), 2.95-1.74 (m, 8H).

### Step 4: Preparation of Compound 15

Compound 1k (100 mg, 192.30 µmol) and Compound 15d (96 mg, 230.57 µmol) were weighed and dissolved in anhydrous DMF (2 mL), and HATU (110 mg, 289.29 µmol) and DMAP (35 mg, 286.48 µmol) were added with stirring in an ice bath. After addition, the mixture was stirred at room temperature for 2 hours. The reaction solution was purified by preparative reversed-phase HPLC (trifluoroacetic acid system) to give Compound 15 (76 mg, yield: 45%).

MS (ESI): m/z = 401.2 [(M-2CF₃COO⁻)/2]⁺.

¹H NMR (400 MHz, DMSO-*d*₆) *δ* 7.08-7.02 (m, 1H), 7.01-6.94 (m, 4H), 6.90-6.81 (m, 3H), 6.63-6.55 (m, 2H), 7.08 (s, 1H), 5.55 (s, 1H), 5.09-4.75 (m, 3H), 4.65-4.39 (m, 2H), 4.33-3.87(m, 8H), 3.77-3.76 (m, 3H), 3.75 (s, 3H), 3.73-3.72 (m, 3H), 3.71-3.70 (m, 3H), 3.69-3.45 (m, 8H), 3.42-3.35 (m, 2H), 3.33 (s, 3H), 3.14-3.06 (m, 2H), 2.92-2.71 (m, 2H), 2.39-2.17 (m, 6H), 2.10-1.93 (m, 2H).

### Biological Evaluation

### Test Example 1. Pharmacodynamic Test

### 1. Test compounds

Samples: Compound 4, Compound 5, Control Compound 1, and Control Compound 2, all prepared in physiological saline.

### 2. Laboratory animals

SD rats (source: Beijing Hfk Bioscience Co., Ltd), male, weighing 200-350 g before the experiment, maintained under a 12-h light/dark cycle, and housed in SPF conditions.

### 3. Experimental procedures

1) The rats were weighed. Ethyl carbamate was prepared as a 25% urethane solution and injected intraperitoneally at 1 mL/100 g for anesthesia. After the animals lost consciousness and reflexes, each rat was fixed in a prone position on a foam board, and the right hindlimb area was depilated. The trachea and external jugular veins were dissected, followed by tracheal intubation and venous cannulation, respectively, and an artificial ventilator was prepared.
2) Posterior to the hip joint, an incision was made in the skin over the lateral edge of the femur at the mid-thigh level. Muscles were bluntly dissected using a glass dissecting needle to expose the sciatic nerve. The skin of the lower leg was incised at the ankle joint, the anterior ligament of the ankle joint was transected, and the gastrocnemius muscle was isolated. The tendon of the gastrocnemius muscle at the ankle was ligated, and the tendon was transected distal to the ligature.
3) A biological function experimental system was used to collect and record signals. The ligature around the gastrocnemius muscle was connected to a tension transducer, and a stimulator was connected to the sciatic nerve. The item was set to evaluate the effect of stimulation intensity on skeletal muscle contraction, with the following parameters: square wave, fine voltage, train stimulation, delay time 0.05 ms, pulse width 0.2 ms, pulse interval 10 ms, frequency 8 Hz, intensity 0.350 V, intensity increment 0, train length 4, and main cycle 12 s. The muscle tension curve was recorded.
4) After the signals stabilized, different doses of the test compounds were injected via the jugular vein, and the inhibition of muscle tension after administration was recorded. During the measurement, the muscles and nerves were kept moist with physiological saline, which was applied every five minutes.

Experimental index:
ED₉₅ (drug dose causing 95% neuromuscular blockade) was calculated as follows:
Using X as the logarithmic dose and Y as the percentage inhibition of muscle tension, a non-linear regression method was used to establish a drug dose-response regression equation, and ED₉₅ was calculated therefrom.

### 4. Results

The ED₉₅ values for the neuromuscular blocking effects of the compounds of the present invention in rats are shown in Table 1 below.

**Table 1**

| Compound | ED₉₅ (mg/kg) |
|---|---|
| Control Compound 1 | 0.93 |
| Control Compound 2 | 0.45 |
| Compound 4 | 0.73 |
| Compound 5 | 0.35 |

Compared with the control compounds, Compound 5 of the present invention exhibits excellent neuromuscular blocking efficacy, which is superior to Control Compound 1 and Control Compound 2.

### Test Example 2. Histamine Release Assay

### 1. Test compounds

Samples: Compound 4, Compound 5, Control Compound 1, Control Compound 2, and Control Compound 3, all prepared in physiological saline.

### 2. Laboratory animals

SD rats (source: Beijing Hfk Bioscience Co., Ltd), male, weighing 200-350 g before the experiment, maintained under a 12-h light/dark cycle, and housed in SPF conditions.

### 3. Experimental procedures

1) The rats were weighed. Ethyl carbamate was prepared as a 25% urethane solution and injected intraperitoneally at 1 mL/100 g for anesthesia. After the animals lost consciousness and reflexes, each rat was fixed in a prone position on a foam board, and the right hindlimb area was depilated. One jugular vein was cannulated for drug injection, and the other jugular vein was cannulated for blood collection.
2) After stabilization for 15 min, 0.5 mL of venous blood was collected into an EP tube containing heparinized physiological saline (1 : 9), mixed well, cooled, and centrifuged at 1000 rpm for 10 min at 4°C. 0.2 mL of the upper plasma layer was collected and cryopreserved in dry ice. After stabilization for 15 min, the test drug was injected intravenously at a dose of 20 mg/kg. Immediately after 1 min of administration, 0.5 mL of venous blood was collected, mixed well, cooled, and centrifuged at 1000 rpm for 10 min at 4°C. 0.2 mL of the upper plasma layer was collected and cryopreserved in dry ice.
3) After processing the above plasma samples, the histamine content in the rat plasma before and after administration was determined by LC-MS/MS. Formula: histamine increase fold = histamine content in rat plasma after administration / histamine content in rat plasma before administration.

### 4. Results

The changes in histamine release in the rat plasma before and after intravenous administration of the compounds of the present invention at 20 mg/kg in the rats are shown in Table 2 below (* indicates P < 0.05; and ** indicates P < 0.01).

**Table 2**

| Compound | Histamine increase fold |
|---|---|
| Control Compound 1 | 3.6* |
| Control Compound 2 | 7.6** |
| Control Compound 3 | 2.2* |
| Compound 4 | 4.4* |
| Compound 5 | About 1 |

A common adverse reaction of clinical muscle relaxants is histamine release. Histamine release following muscle relaxant injection may induce bronchospasm, increased heart rate, and blood pressure reduction, which are harmful to patient health. In this test, significant histamine release was induced after the administration of the control compounds (P < 0.05 or P < 0.01). In contrast, no significant increase in the histamine content was observed following administration of Compound 5 of the present invention. Compound 5 of the present invention possesses significantly superior safety profiles relative to the control compounds.

### Test Example 3. Pharmacodynamic Test

### 1. Test compounds

Samples: Compound 5A, Compound 5B, and Control Compound 3, all prepared in physiological saline.

### 2. Laboratory animals

SD rats (source: Beijing Hfk Bioscience Co., Ltd), male, weighing 200-350 g before the experiment, maintained under a 12-h light/dark cycle, and housed in SPF conditions.

### 3. Experimental procedures

1) The rats were weighed. Ethyl carbamate was prepared as a 25% urethane solution and injected intraperitoneally at 1 mL/100 g for anesthesia. After the animals lost consciousness and reflexes, each rat was fixed in a prone position on a foam board, and the right hindlimb area was depilated. The trachea and external jugular veins were dissected, followed by tracheal intubation and venous cannulation, respectively, and an artificial ventilator was prepared.
2) Posterior to the hip joint, an incision was made in the skin over the lateral edge of the femur at the mid-thigh level. Muscles were bluntly dissected using a glass dissecting needle to expose the sciatic nerve. The skin of the lower leg was incised at the ankle joint, the anterior ligament of the ankle joint was transected, and the gastrocnemius muscle was isolated. The tendon of the gastrocnemius muscle at the ankle was ligated, and the tendon was transected distal to the ligature.
3) A biological function experimental system was used to collect and record signals. The ligature around the gastrocnemius muscle was connected to a tension transducer, and a stimulator was connected to the sciatic nerve. The item was set to evaluate the effect of stimulation intensity on skeletal muscle contraction, with the following parameters: square wave, fine voltage, train stimulation, delay time 0.05 ms, pulse width 0.2 ms, pulse interval 10 ms, frequency 8 Hz, intensity 0.350 V, intensity increment 0, train length 4, and main cycle 12 s. The muscle tension curve was recorded.
4) After the signals stabilized, different doses of different test compounds were injected via the jugular vein, and the inhibition of muscle tension after administration was recorded. During the measurement, the muscles and nerves were kept moist with physiological saline, which was applied every five minutes.

Experimental indexes:
ED₉₅ (drug dose causing 95% neuromuscular blockade) was calculated as follows, along with the corresponding onset time and duration pharmacodynamic indexes:
Calculation method for ED₉₅: Using X as the logarithmic dose and Y as the probit of the percentage inhibition of muscle twitch, a linear regression method was used to establish a drug dose-response regression equation, and ED₉₅ was calculated therefrom.

### 4. Results

The ED₉₅ values for the neuromuscular blocking effects of the compounds of the present invention in rats are shown in Table 3 below.

**Table 3**

| Compound | ED₉₅ (mg/kg) |
|---|---|
| Compound 5A | 0.69 |
| Compound 5B | 0.3 |
| Control Compound 3 | 0.56 |

Compared with the control compound, Compound 5B of the present invention exhibits excellent neuromuscular blocking efficacy.

### Test Example 4. Pharmacodynamic Test

### 1. Test compounds

Samples: Compound 5B, Compound 15, and Control Compound 3, all prepared in physiological saline.

### 2. Laboratory animals

SD rats (source: Beijing Hfk Bioscience Co., Ltd), male, weighing 200-350 g before the experiment, maintained under a 12-h light/dark cycle, and housed in SPF conditions.

### 3. Experimental procedures

1) The rats were weighed. Ethyl carbamate was prepared as a 25% urethane solution and injected intraperitoneally at 1 mL/100 g for anesthesia. After the animals lost consciousness and reflexes, each rat was fixed in a prone position on a foam board, and the right hindlimb area was depilated. The trachea and external jugular veins were dissected, followed by tracheal intubation and venous cannulation, respectively, and an artificial ventilator was prepared.
2) Posterior to the hip joint, an incision was made in the skin over the lateral edge of the femur at the mid-thigh level. Muscles were bluntly dissected using a glass dissecting needle to expose the sciatic nerve. The skin of the lower leg was incised at the ankle joint, the anterior ligament of the ankle joint was transected, and the gastrocnemius muscle was isolated. The tendon of the gastrocnemius muscle at the ankle was ligated, and the tendon was transected distal to the ligature.
3) A biological function experimental system was used to collect and record signals. The ligature around the gastrocnemius muscle was connected to a tension transducer, and a stimulator was connected to the sciatic nerve. The item was set to evaluate the effect of stimulation intensity on skeletal muscle contraction, with the following parameters: square wave, fine voltage, train stimulation, delay time 0.05 ms, pulse width 0.2 ms, pulse interval 10 ms, frequency 8 Hz, intensity 0.350 V, intensity increment 0, train length 4, and main cycle 12 s. The muscle tension curve was recorded.
4) After the signals stabilized, 0.3 mg/kg of different test compounds were injected via the jugular vein, and the inhibition of muscle tension after administration was recorded. During the measurement, the muscles and nerves were kept moist with physiological saline, which was applied every five minutes.

Experimental index: 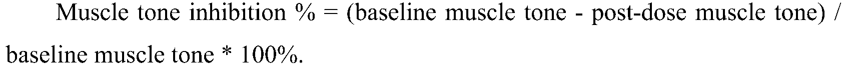 Muscle tone inhibition % = (baseline muscle tone - post-dose muscle tone) / baseline muscle tone * 100%.

### 4. Results

The muscle tension inhibition rates of the compounds of the present invention at 0.3 mg/kg in the rats are shown in Table 4 below.

**Table 4**

| Compound | Muscle tone inhibition % |
|---|---|
| Compound 5B | 97.04% |
| Compound 15 | 79.05% |
| Control Compound 3 | 45.77% |

Compared with the control compound, Compound 5B and Compound 15 of the present invention exhibit excellent neuromuscular blocking efficacy at the same dose, which is superior to Control Compound 3.

### Test Example 5. Histamine Release Assay

### 1. Test compounds

Samples: Compound 5B, Compound 15, and Control Compound 3, all prepared in physiological saline.

### 2. Laboratory animals

SD rats (source: Beijing Hfk Bioscience Co., Ltd), male, weighing 200-350 g before the experiment, maintained under a 12-h light/dark cycle, and housed in SPF conditions.

### 3. Experimental procedures

1) The rats were weighed. Ethyl carbamate was prepared as a 25% urethane solution and injected intraperitoneally at 1 mL/100 g for anesthesia. After the animals lost consciousness and reflexes, each rat was fixed in a prone position on a foam board, and the right hindlimb area was depilated. One jugular vein was cannulated for drug injection, and the other jugular vein was cannulated for blood collection.
2) After stabilization for 15 min, 0.5 mL of venous blood was collected into an EP tube containing heparinized physiological saline (1 : 9), mixed well, cooled, and centrifuged at 1000 rpm for 10 min at 4°C. 0.2 mL of the upper plasma layer was collected and cryopreserved in dry ice. After stabilization for 15 min, the test drug was injected intravenously at a dose of 20 mg/kg. Immediately after 1 min of administration, 0.5 mL of venous blood was collected, mixed well, cooled, and centrifuged at 1000 rpm for 10 min at 4°C. 0.2 mL of the upper plasma layer was collected and cryopreserved in dry ice.
3) After processing the above plasma samples, the histamine content in the rat plasma before and after administration was determined by LC-MS/MS. Formula: histamine increase fold = histamine content in rat plasma after administration / histamine content in rat plasma before administration.

### 4. Results

The changes in histamine release in the rat plasma before and after intravenous administration of the compounds of the present invention at 20 mg/kg in the rats are shown in Table 5 below (* indicates P < 0.05).

**Table 5**

| Compound | Histamine increase fold |
|---|---|
| Compound 5B | About 1.6 |
| Compound 15 | About 1.5 |
| Control Compound 3 | About 3.0* |

A common adverse reaction of clinical muscle relaxants is histamine release. Histamine release following muscle relaxant injection may induce bronchospasm, increased heart rate, and blood pressure reduction, which are harmful to patient health. In this test, significant histamine release was induced after the administration of the control compound (P < 0.05). In contrast, no significant increase in the histamine content was observed following administration of Compound 5B and Compound 15 of the present invention. Compound 5B and Compound 15 of the present invention possess significantly superior safety profiles relative to the control compound.

## Claims

1. A compound represented by formula IA or a pharmaceutically acceptable salt thereof, 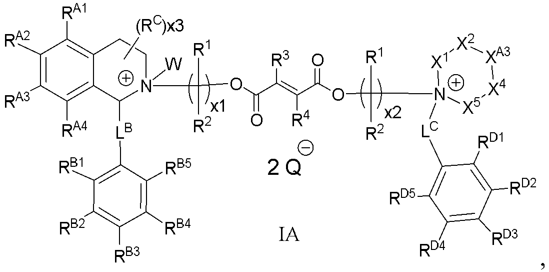 wherein:
W is selected from the group consisting of -C(R^{a}R^{b}R^{c}) or 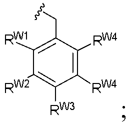
each Q^{⊖} is independently selected from the group consisting of pharmaceutically acceptable anions;
R³ and R⁴ are each independently selected from the group consisting of hydrogen, halogen, C₁₋₆ alkyl, or C₁₋₆ alkoxy, wherein the C₁₋₆ alkyl or C₁₋₆ alkoxy is optionally substituted with one or more halogen, hydroxy, mercapto, carboxy, amino, or cyano;
L^{B} and L^{C} are each independently selected from the group consisting of - C(R^{a}R^{b}R^{c}) or alkylene, wherein the alkylene is optionally substituted with one or more halogen, hydroxy, mercapto, nitro, carboxy, amino, cyano, C₁₋₆ alkyl, C₁₋₆ alkoxy, oxo, thioxo, or sulfonyl, and the C₁₋₆ alkyl or C₁₋₆ alkoxy is optionally substituted with one or more halogen, hydroxy, mercapto, carboxy, amino, or cyano;
when L^{B} is -C(R^{a}R^{b}R^{c}), the phenyl with R^{B1} to R^{B5} substituents is absent;
when L^{C} is -C(R^{a}R^{b}R^{c}), the phenyl with R^{D1} to R^{D5} substituents is absent;
X¹³ is selected from the group consisting of a chemical bond, -O-, -C(R^{d}R^{e})-, - N(R^{f})-, or -S(O)₂-;
X¹ and X⁵ are each independently -C(R^{g}R^{h})-;
X² and X⁴ are each independently -C(RⁱR^{j})-;
R^{f} is selected from the group consisting of hydrogen, C₁₋₆ alkyl, or C₁₋₆ alkoxy, wherein the C₁₋₆ alkyl or C₁₋₆ alkoxy is optionally substituted with one or more halogen, hydroxy, mercapto, carboxy, amino, or cyano;
R¹, R², R^{A1}, R^{A2}, R^{A3}, R^{A4}, R^{B1}, R^{B2}, R^{B3}, R^{B4}, R^{B5}, R^{C}, R^{D1}, R^{D2}, R^{D3}, R^{D4}, R^{D5}, R^{W1}, R^{W2}, R^{W3}, R^{W4}, R^{W5}, R^{a}, R^{b}, R^{c}, R^{d}, R^{e}, R^{g}, R^{h}, Rⁱ, and R^{j} are each independently selected from the group consisting of hydrogen, halogen, hydroxy, mercapto, carboxy, amino, cyano, C₁₋₆ alkyl, C₁₋₆ alkoxy, -C(O)-C₁₋₆ alkyl, -C(O)-C₁₋₆ alkoxy, methanesulfonyl, a 3- to 10-membered carbocyclic ring, a 3- to 10-membered heterocyclic ring, a 6- to 10-membered aromatic ring, or a 5- to 10-membered heteroaromatic ring, wherein the C₁₋₆ alkyl, C₁₋₆ alkoxy, 3- to 10-membered carbocyclic ring, 3- to 10-membered heterocyclic ring, 6- to 10-membered aromatic ring, or 5- to 10-membered heteroaromatic ring is optionally substituted with one or more halogen, hydroxy, mercapto, carboxy, amino, or cyano,
or alternatively, one or more pairs of R¹ and R², R^{d} and R^{e}, R^{g} and R^{h}, or Rⁱ and R^{j} together with the atoms to which they are attached form oxo, thioxo, a 3- to 10-membered carbocyclic ring, a 3- to 10-membered heterocyclic ring, a 6- to 10-membered aromatic ring, or a 5- to 10-membered heteroaromatic ring, wherein the 3- to 10-membered carbocyclic ring, 3- to 10-membered heterocyclic ring, 6- to 10-membered aromatic ring, or 5- to 10-membered heteroaromatic ring is optionally substituted with one or more halogen, hydroxy, mercapto, carboxy, amino, cyano, C₁₋₆ alkyl, or C₁₋₆ alkoxy, and the C₁₋₆ alkyl or C₁₋₆ alkoxy is optionally substituted with one or more halogen, hydroxy, mercapto, carboxy, amino, or cyano,
or alternatively, R^{g} and Rⁱ together with the atoms to which they are attached form a 3- to 10-membered carbocyclic ring, a 3- to 10-membered heterocyclic ring, a 6- to 10-membered aromatic ring, or a 5- to 10-membered heteroaromatic ring, wherein the 3- to 10-membered carbocyclic ring, 3- to 10-membered heterocyclic ring, 6- to 10-membered aromatic ring, or 5- to 10-membered heteroaromatic ring is optionally substituted with one or more halogen, hydroxy, mercapto, carboxy, amino, cyano, C₁₋₆ alkyl, or C₁₋₆ alkoxy, and the C₁₋₆ alkyl or C₁₋₆ alkoxy is optionally substituted with one or more halogen, hydroxy, mercapto, carboxy, amino, or cyano,
or alternatively, any two adjacent groups of R^{A1}, R^{A2}, R^{A3}, and R^{A4} together form methylenedioxy, ethylenedioxy, or -CH₂-O-CH₂-,
or alternatively, any two adjacent groups of R^{B1}, R^{B2}, R^{B3}, R^{B4}, and R^{B5} together form methylenedioxy, ethylenedioxy, or -CH₂-O-CH₂-,
or alternatively, any two adjacent groups of R^{D1}, R^{D2}, R^{D3}, R^{D4}, and R^{D5} together form methylenedioxy, ethylenedioxy, or -CH₂-O-CH₂-,
or alternatively, any two adjacent groups of R^{W1}, R^{W2}, R^{W3}, R^{W4}, and R^{W5} together form methylenedioxy, ethylenedioxy, or -CH₂-O-CH₂-;
when X^{A3} is selected from the group consisting of a chemical bond or -C(R^{d}R^{e})-,
(i) R^{d} and R^{e} cannot both be hydrogen, or alternatively,
(ii) one or more pairs of R^{d} and R^{e}, R^{g} and R^{h}, or Rⁱ and R^{j} together with the atoms to which they are attached form oxo, thioxo, a 3- to 10-membered carbocyclic ring, a 3- to 10-membered heterocyclic ring, a 6- to 10-membered aromatic ring, or a 5- to 10-membered heteroaromatic ring, wherein the 3- to 10-membered carbocyclic ring, 3- to 10-membered heterocyclic ring, 6- to 10-membered aromatic ring, or 5- to 10-membered heteroaromatic ring is optionally substituted with one or more halogen, hydroxy, mercapto, carboxy, amino, cyano, C₁₋₆ alkyl, or C₁₋₆ alkoxy, and the C₁₋₆ alkyl or C₁₋₆ alkoxy is optionally substituted with one or more halogen, hydroxy, mercapto, carboxy, amino, or cyano, or alternatively,
(iii) R^{g} and Rⁱ together with the atoms to which they are attached form a 3- to 10-membered carbocyclic ring, a 3- to 10-membered heterocyclic ring, a 6- to 10-membered aromatic ring, or a 5- to 10-membered heteroaromatic ring, wherein the 3- to 10-membered carbocyclic ring, 3- to 10-membered heterocyclic ring, 6- to 10-membered aromatic ring, or 5- to 10-membered heteroaromatic ring is optionally substituted with one or more halogen, hydroxy, mercapto, carboxy, amino, cyano, C₁₋₆ alkyl, or C₁₋₆ alkoxy, and the C₁₋₆ alkyl or C₁₋₆ alkoxy is optionally substituted with one or more halogen, hydroxy, mercapto, carboxy, amino, or cyano;
when X^{A3} is -O-,
(i) one or both pairs of R^{g} and R^{h} or Rⁱ and R^{j} together with the atoms to which they are attached form oxo, thioxo, a 3- to 10-membered carbocyclic ring, a 3- to 10-membered heterocyclic ring, a 6- to 10-membered aromatic ring, or a 5- to 10-membered heteroaromatic ring, wherein the 3- to 10-membered carbocyclic ring, 3-to 10-membered heterocyclic ring, 6- to 10-membered aromatic ring, or 5- to 10-membered heteroaromatic ring is optionally substituted with one or more halogen, hydroxy, mercapto, carboxy, amino, cyano, C₁₋₆ alkyl, or C₁₋₆ alkoxy, and the C₁₋₆ alkyl or C₁₋₆ alkoxy is optionally substituted with one or more halogen, hydroxy, mercapto, carboxy, amino, or cyano, or alternatively,
(ii) R^{g} and Rⁱ together with the atoms to which they are attached form a 3- to 10-membered carbocyclic ring, a 3- to 10-membered heterocyclic ring, a 6- to 10-membered aromatic ring, or a 5- to 10-membered heteroaromatic ring, wherein the 3- to 10-membered carbocyclic ring, 3- to 10-membered heterocyclic ring, 6- to 10-membered aromatic ring, or 5- to 10-membered heteroaromatic ring is optionally substituted with one or more halogen, hydroxy, mercapto, carboxy, amino, cyano, C₁₋₆ alkyl, or C₁₋₆ alkoxy, and the C₁₋₆ alkyl or C₁₋₆ alkoxy is optionally substituted with one or more halogen, hydroxy, mercapto, carboxy, amino, or cyano;
x1 is selected from the group consisting of 1, 2, 3, 4, 5, or 6;
x2 is selected from the group consisting of 1, 2, 3, 4, 5, or 6; and
x3 is selected from the group consisting of 0, 1, 2, 3, 4, or 5.

2. The compound or the pharmaceutically acceptable salt thereof according to claim 1, wherein X^{A3} is selected from the group consisting of a chemical bond, -C(R^{d}R^{e})-, or -S(O)₂-, wherein R^{d} and R^{e} are as defined in claim 1.

3. The compound or the pharmaceutically acceptable salt thereof according to claim 1 or 2, wherein the compound represented by formula IA is selected from the group consisting of the compound represented by formula IIA1, formula IIA2, formula IIA3, formula IIA4, or formula IIA5, or a pharmaceutically acceptable salt thereof, 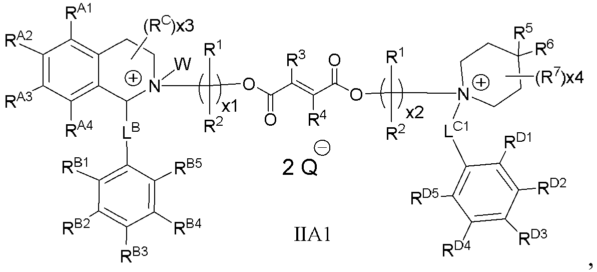 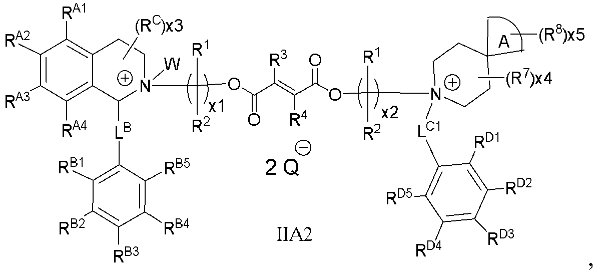 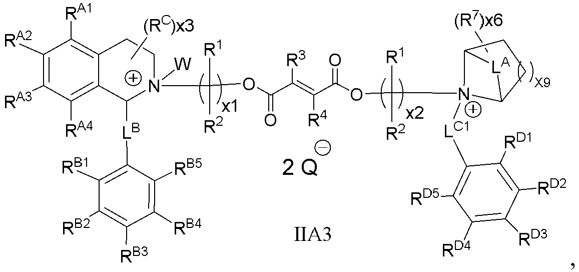 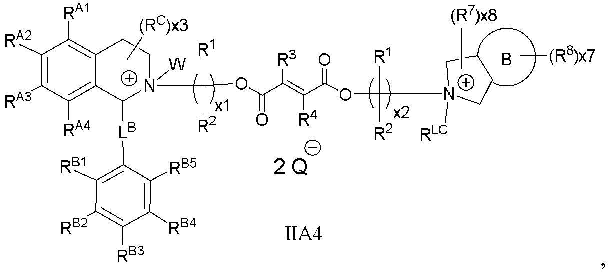 or 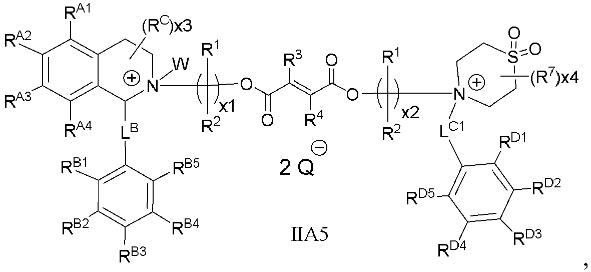 wherein
ring A and ring B are each independently selected from the group consisting of a 3- to 10-membered carbocyclic ring, a 3- to 10-membered heterocyclic ring, a 6-to 10-membered aromatic ring, or a 5- to 10-membered heteroaromatic ring;
L^{A} is selected from the group consisting of alkylene or heteroalkylene, wherein the alkylene or heteroalkylene is optionally substituted with one or more halogen, hydroxy, mercapto, nitro, carboxy, amino, cyano, C₁₋₆ alkyl, C₁₋₆ alkoxy, oxo, thioxo, or sulfonyl, and the C₁₋₆ alkyl or C₁₋₆ alkoxy is optionally substituted with one or more halogen, hydroxy, mercapto, carboxy, amino, or cyano;
L^{C1} is alkylene, wherein the alkylene is optionally substituted with one or more halogen, hydroxy, mercapto, nitro, carboxy, amino, cyano, C₁₋₆ alkyl, C₁₋₆ alkoxy, oxo, thioxo, or sulfonyl, and the C₁₋₆ alkyl or C₁₋₆ alkoxy is optionally substituted with one or more halogen, hydroxy, mercapto, carboxy, amino, or cyano;
R^{LC} is C₁₋₆ alkyl, wherein the C₁₋₆ alkyl is optionally substituted with one or more halogen, hydroxy, mercapto, carboxy, amino, or cyano;
R⁵, R⁶, R⁷, and R⁸ are each independently selected from the group consisting of hydrogen, halogen, hydroxy, mercapto, carboxy, amino, cyano, C₁₋₆ alkyl, C₁₋₆ alkoxy, -C(O)-C₁₋₆ alkyl, -C(O)-C₁₋₆ alkoxy, methanesulfonyl, a 3- to 10-membered carbocyclic ring, a 3- to 10-membered heterocyclic ring, a 6- to 10-membered aromatic ring, or a 5- to 10-membered heteroaromatic ring, wherein the C₁₋₆ alkyl, C₁₋₆ alkoxy, 3- to 10-membered carbocyclic ring, 3- to 10-membered heterocyclic ring, 6- to 10-membered aromatic ring, or 5- to 10-membered heteroaromatic ring is optionally substituted with one or more halogen, hydroxy, mercapto, carboxy, amino, or cyano, and R⁵ and R⁶ cannot both be hydrogen,
or alternatively, R⁵ and R⁶ together with the atoms to which they are attached form oxo or thioxo;
each x4 is independently selected from the group consisting of 0, 1, 2, 3, 4, 5, 6, 7, or 8;
x5 is selected from the group consisting of 0, 1, 2, 3, or 4;
each x6 is independently selected from the group consisting of 0, 1, 2, 3, 4, 5, or 6;
x7 is selected from the group consisting of 0, 1, 2, 3, or 4;
x8 is selected from the group consisting of 0, 1, 2, 3, or 4;
x9 is selected from the group consisting of 0, 1, 2, or 3; and
W, Q^{⊖}, L^{B}, R¹, R², R³, R⁴, R^{A1}, R^{A2}, R^{A3}, R^{A4}, R^{B2}, R^{B3}, R^{B4}, R^{B5}, R^{C}, R^{D1}, R^{D2}, R^{D3}, R^{D4}, R^{D5}, x1, x2, and x3 are as defined in claim 1.

4. The compound or the pharmaceutically acceptable salt thereof according to claim 3, wherein ring A is a 3- to 10-membered heterocyclic ring, preferably an oxetane ring, an azetidine ring, a thietane ring, a tetrahydropyrrole ring, a tetrahydrofuran ring, a tetrahydrothiophene ring, a piperidine ring, a tetrahydropyran ring, or a tetrahydrothiopyran ring, and most preferably an oxetane ring.

5. The compound or the pharmaceutically acceptable salt thereof according to claim 3, wherein ring B is a 6- to 10-membered aromatic ring or a 5- to 10-membered heteroaromatic ring, preferably a benzene ring, a pyridine ring, a pyran ring, or a thiopyran ring, and most preferably a benzene ring or a pyridine ring.

6. The compound or the pharmaceutically acceptable salt thereof according to claim 3, wherein L^{A} is selected from the group consisting of -(CR^{k}R^{l})ₐ-O-(CR^{k}R^{l})_{b}-, -(CR^{k}R^{l})ₐ-S-(CR^{k}R^{l})_{b}-, -(CR^{k}R^{l})ₐ-N(R^{f})-(CR^{k}R^{l})_{b}-, or -(CR^{k}R^{l})_{c}-, preferably -(CR^{k}R^{l})ₐ-O-(CR^{k}R^{l})_{b}- or -(CR^{k}R^{l})_{c}-, wherein
R^{k} and R^{l} are each independently selected from the group consisting of hydrogen, halogen, hydroxy, mercapto, carboxy, amino, cyano, C₁₋₆ alkyl, C₁₋₆ alkoxy, -C(O)-C₁₋₆ alkyl, -C(O)-C₁₋₆ alkoxy, methanesulfonyl, a 3- to 10-membered carbocyclic ring, a 3- to 10-membered heterocyclic ring, a 6- to 10-membered aromatic ring, or a 5- to 10-membered heteroaromatic ring, wherein the C₁₋₆ alkyl, C₁₋₆ alkoxy, 3- to 10-membered carbocyclic ring, 3- to 10-membered heterocyclic ring, 6- to 10-membered aromatic ring, or 5- to 10-membered heteroaromatic ring is optionally substituted with one or more halogen, hydroxy, mercapto, carboxy, amino, or cyano,
or alternatively, R^{k} and R^{l} together with the atoms to which they are attached form oxo, thioxo, a 3- to 10-membered carbocyclic ring, a 3- to 10-membered heterocyclic ring, a 6- to 10-membered aromatic ring, or a 5- to 10-membered heteroaromatic ring, wherein the 3- to 10-membered carbocyclic ring, 3- to 10-membered heterocyclic ring, 6- to 10-membered aromatic ring, or 5- to 10-membered heteroaromatic ring is optionally substituted with one or more halogen, hydroxy, mercapto, carboxy, amino, cyano, C₁₋₆ alkyl, or C₁₋₆ alkoxy, and the C₁₋₆ alkyl or C₁₋₆ alkoxy is optionally substituted with one or more halogen, hydroxy, mercapto, carboxy, amino, or cyano;
each a is independently selected from the group consisting of 0, 1, 2, 3, 4, or 5;
each b is independently selected from the group consisting of 0, 1, 2, 3, 4, or 5;
each c is independently selected from the group consisting of 1, 2, 3, 4, 5, or 6; and
R^{f} is as defined in claim 1.

7. The compound or the pharmaceutically acceptable salt thereof according to claim 3, wherein R⁵ and R⁶ are each independently selected from the group consisting of hydrogen, halogen, or C₁₋₆ alkoxy, preferably hydrogen, fluorine, chlorine, bromine, iodine, methoxy, ethoxy, or propoxy, and most preferably hydrogen, fluorine, or methoxy,
and R⁵ and R⁶ cannot both be hydrogen.

8. The compound or the pharmaceutically acceptable salt thereof according to any one of claims 1 to 7, wherein the compound represented by formula IA is selected from the group consisting of the compound represented by formula IIIA1, formula IIIA2, formula IIIA3, formula IIIA4, or formula IIIA5, or a pharmaceutically acceptable salt thereof, 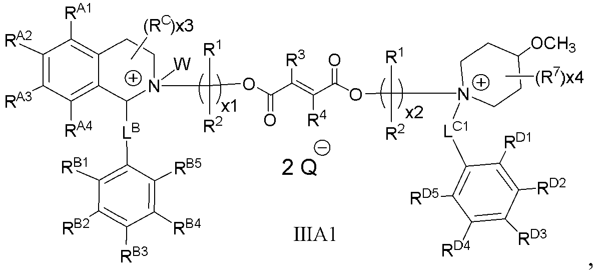 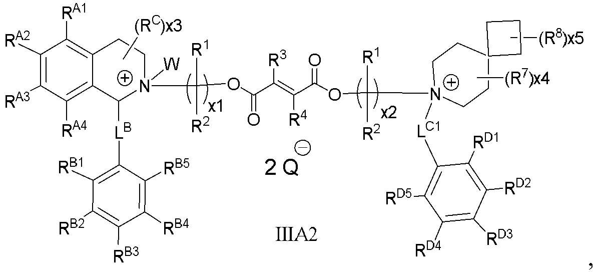 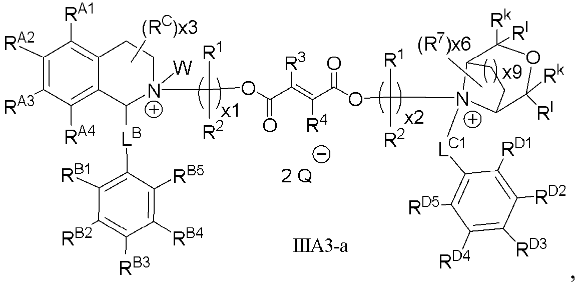 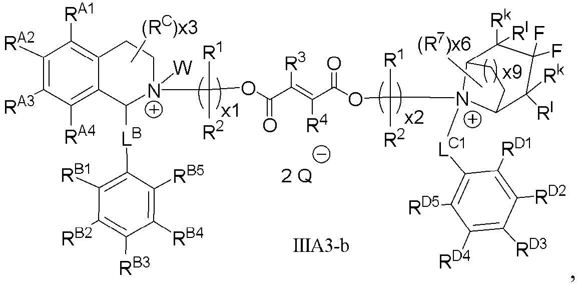 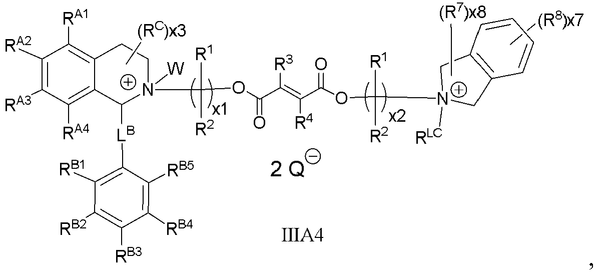 or 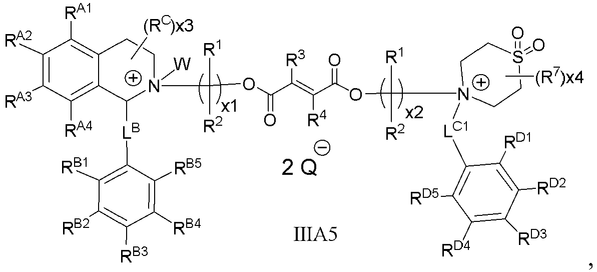 wherein
W, Q^{⊖}, L^{B}, R¹, R², R³, R⁴, R^{A1}, R^{A2}, R^{A3}, R^{A4}, R^{B2}, R^{B3}, R^{B4}, R^{B5}, R^{C}, R^{D1}, R^{D2}, R^{D3}, R^{D4}, R^{D5}, x1, x2, and x3 are as defined in claim 1, L^{C1}, R^{LC}, R⁷, R⁸, x4, x5, x6, x7, x8, and x9 are as defined in claim 3, and R^{k} and R^{l} are as defined in claim 6.

9. The compound or the pharmaceutically acceptable salt thereof according to any one of claims 1 to 8, wherein each L^{B} is independently selected from the group consisting of a chemical bond or C₁₋₆ alkylene, preferably a chemical bond, methylene, ethylene, or propylene, preferably methylene.

10. The compound or the pharmaceutically acceptable salt thereof according to any one of claims 3 to 8, wherein each L^{C1} is independently selected from the group consisting of a chemical bond or C₁₋₆ alkylene, preferably a chemical bond, methylene, ethylene, or propylene, preferably methylene.

11. The compound or the pharmaceutically acceptable salt thereof according to any one of claims 1 to 8, wherein the compound represented by formula IA is selected from the group consisting of the compound represented by formula IVA1, formula IVA2, formula IVA3-a, formula IVA3-b, formula IVA4, or formula IVA5, or a pharmaceutically acceptable salt thereof, 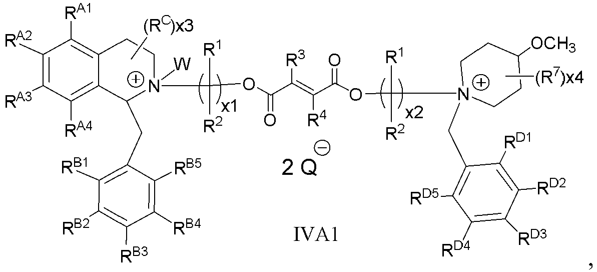 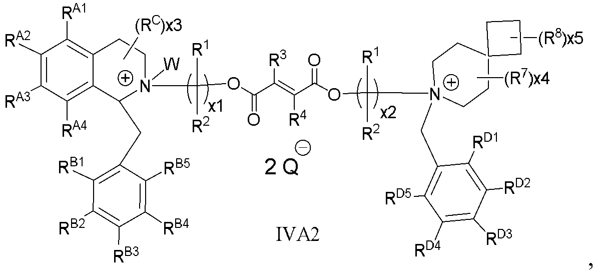 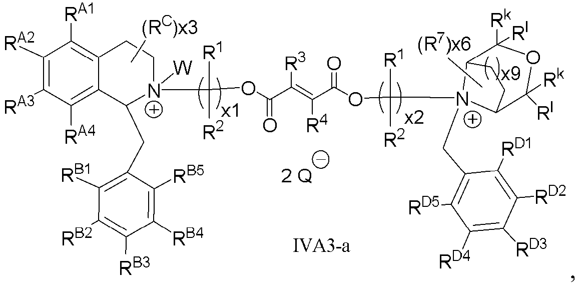 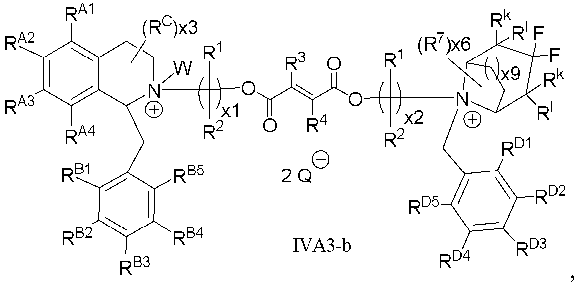 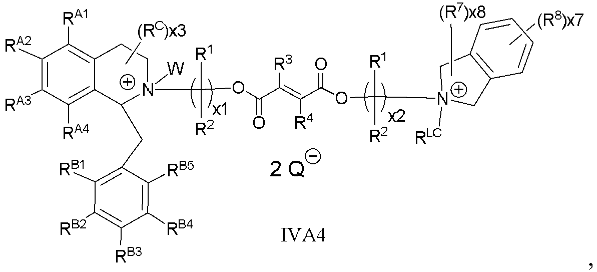 or 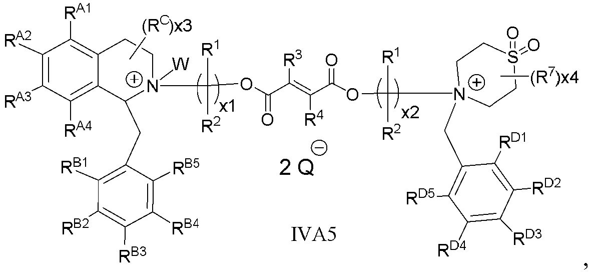 wherein
W, Q^{⊖}, R¹, R², R³, R⁴, R^{A1}, R^{A2}, R^{A3}, R^{A4}, R^{B2}, R^{B3}, R^{B4}, R^{B5}, R^{C}, R^{D1}, R^{D2}, R^{D3}, R^{D4}, R^{D5}, x1, x2, and x3 are as defined in claim 1, R^{LC}, R⁷, R⁸, x4, x5, x6, x7, x8, and x9 are as defined in claim 3, and R^{k} and R^{l} are as defined in claim 6.

12. The compound or the pharmaceutically acceptable salt thereof according to any one of claims 1 to 11, wherein R^{A1}, R^{A4}, R^{B1}, R^{B2}, R^{B5}, R^{D1}, R^{D4}, and R^{D5} are all hydrogen.

13. The compound or the pharmaceutically acceptable salt thereof according to any one of claims 1 to 12, wherein R^{A2}, R^{A3}, R^{B3}, and R^{B4} are each independently selected from the group consisting of hydrogen or C₁₋₆ alkoxy, preferably hydrogen, methoxy, ethoxy, or propoxy, and most preferably hydrogen or methoxy.

14. The compound or the pharmaceutically acceptable salt thereof according to any one of claims 1 to 13, wherein W is methyl.

15. The compound or the pharmaceutically acceptable salt thereof according to any one of claims 1 to 14, wherein R^{D2} and R^{D3} are each independently selected from the group consisting of hydrogen, halogen, -C(O)-C₁₋₆ alkyl, C₁₋₆ alkoxy, or methanesulfonyl, preferably hydrogen, fluorine, chlorine, bromine, iodine, -C(O)-methyl, -C(O)-ethyl, methoxy, ethoxy, or methanesulfonyl, and most preferably hydrogen, fluorine, bromine, -C(O)-ethyl, methoxy, or methanesulfonyl.

16. The compound represented by formula IA or the pharmaceutically acceptable salt thereof, selected from the group consisting of the following compounds or pharmaceutically acceptable salts thereof, 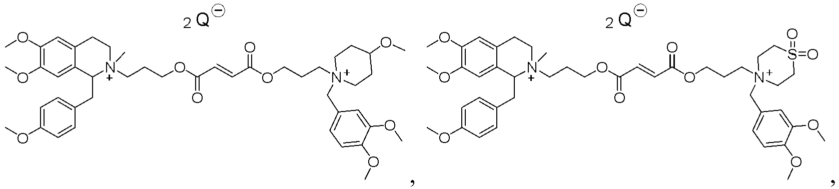 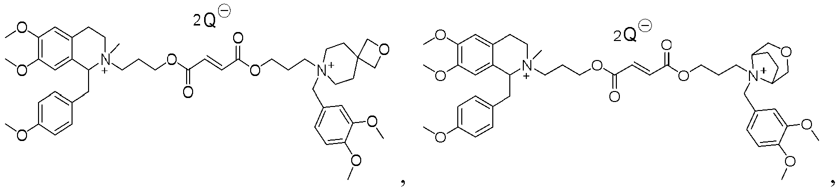 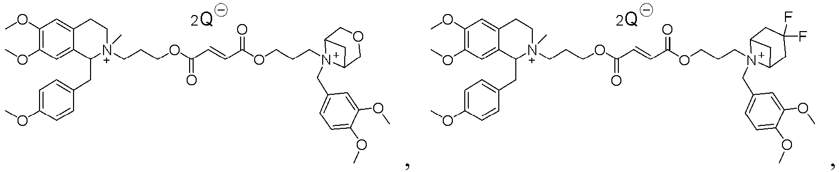 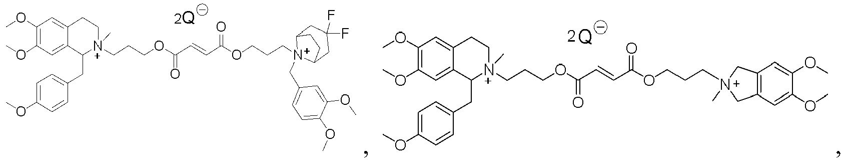 or 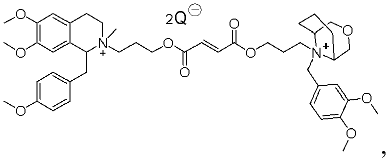 wherein
Q^{⊖} is as defined in claim 1.

17. The compound represented by formula IA or the pharmaceutically acceptable salt thereof, selected from the group consisting of the following compounds or pharmaceutically acceptable salts thereof, 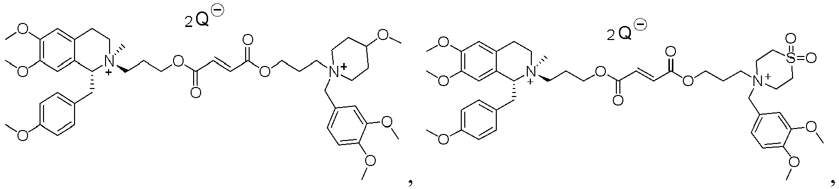 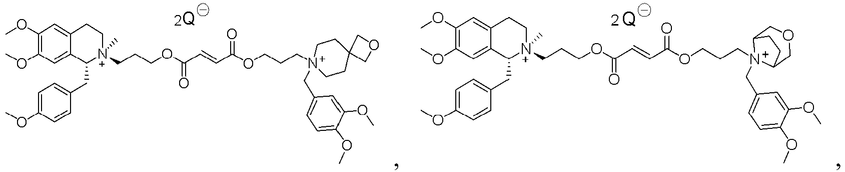 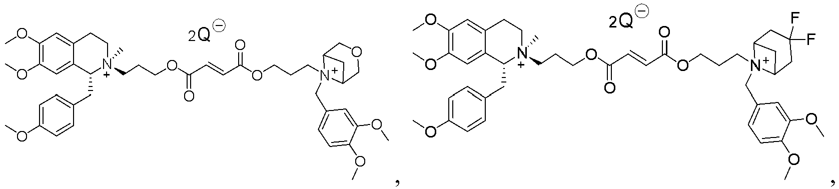 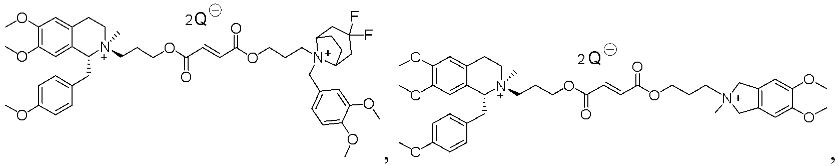 or 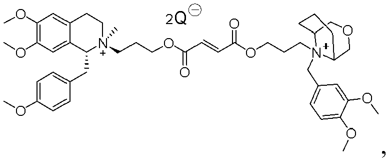 wherein
Q^{⊖} is as defined in claim 1.

18. The compound or the pharmaceutically acceptable salt thereof according to any one of claims 1 to 17, wherein each Q^{⊖} is independently selected from the group consisting of halide ions, acetate, formate, benzoate, benzenesulfonate, camphorsulfonate, citrate, ethanedisulfonate, fumarate, glucoheptonate, gluconate, glucuronate, isethionate, lactate, lactobionate, lauryl sulfate, malate, maleate, methanesulfonate, naphthoate, naphthalenesulfonate, nitrate, stearate, oleate, oxalate, pamoate, phosphate, hydrogen phosphate, dihydrogen phosphate, polygalacturonate, succinate, sulfate, sulfosalicylate, tartrate, toluenesulfonate, or trifluoroacetate, preferably chloride ion, bromide ion, fluoride ion, iodide ion, trifluoroacetate, formate, methanesulfonate, or benzenesulfonate, and most preferably chloride ion, bromide ion, trifluoroacetate, formate, methanesulfonate, or benzenesulfonate.

19. The compound represented by formula IA or the pharmaceutically acceptable salt thereof, selected from the group consisting of the following compounds or pharmaceutically acceptable salts thereof,

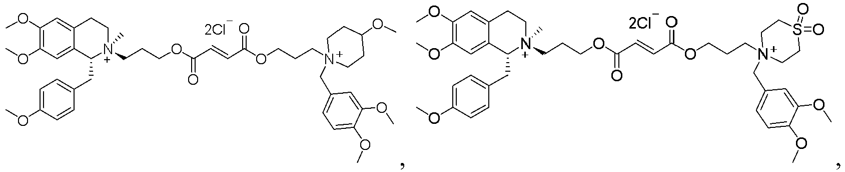

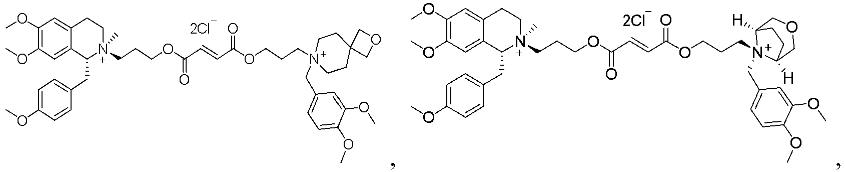

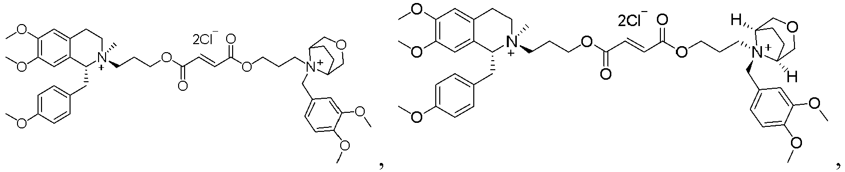

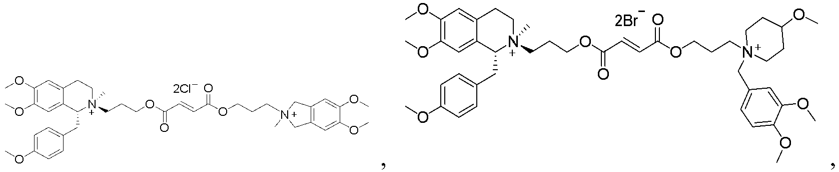

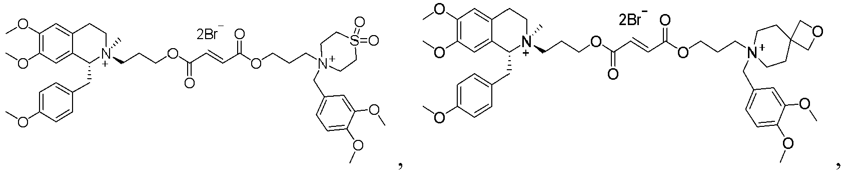

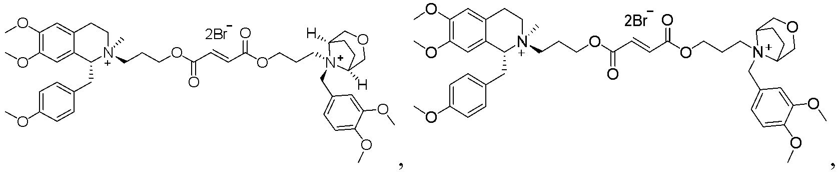

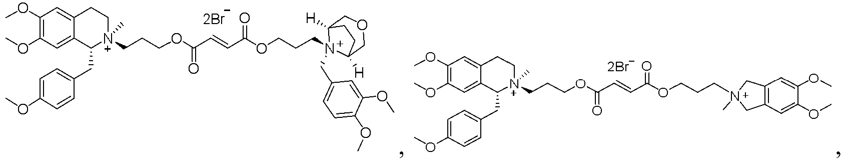

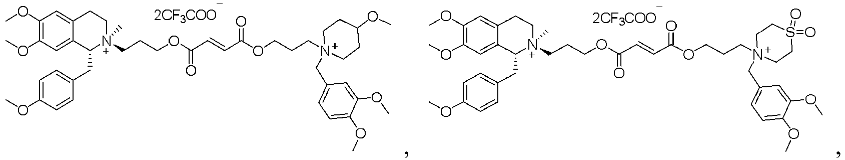

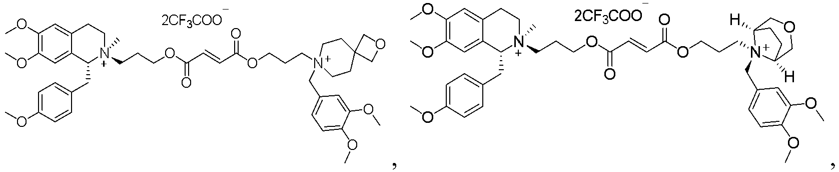

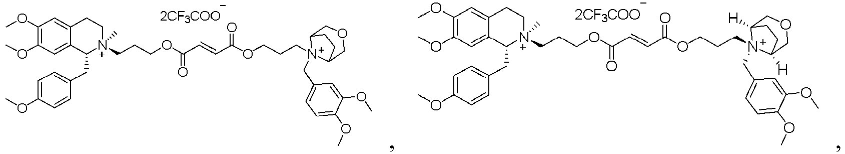

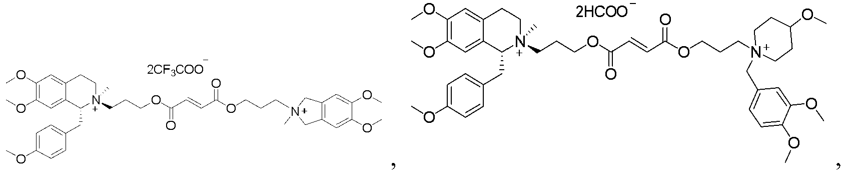

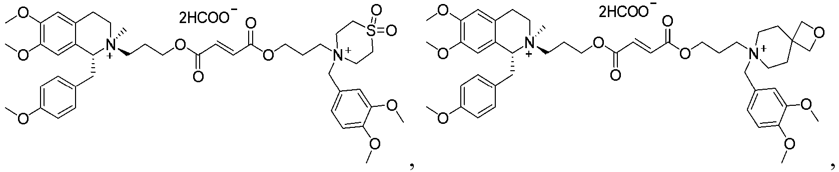

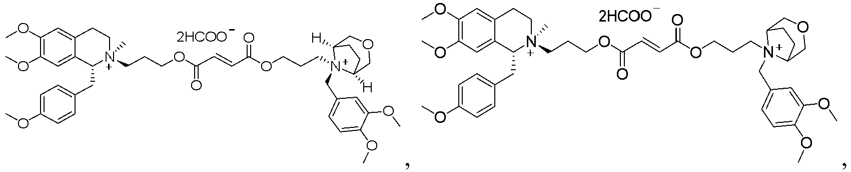

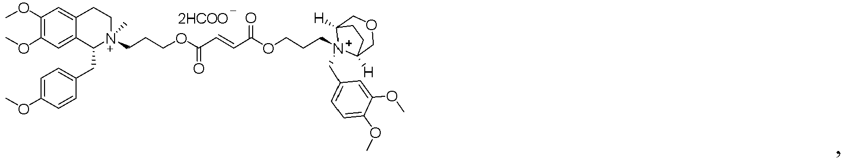

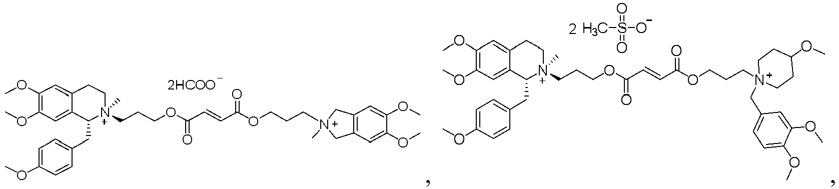

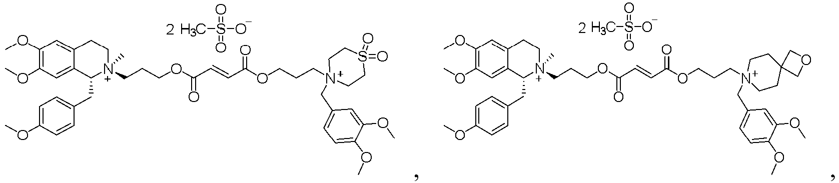

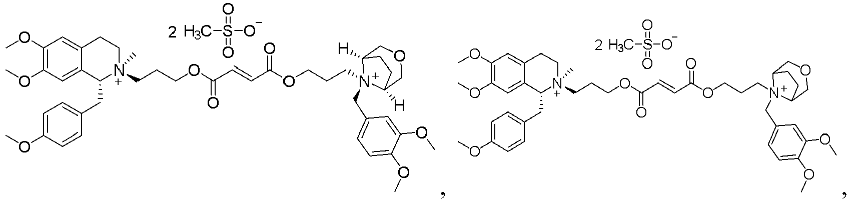

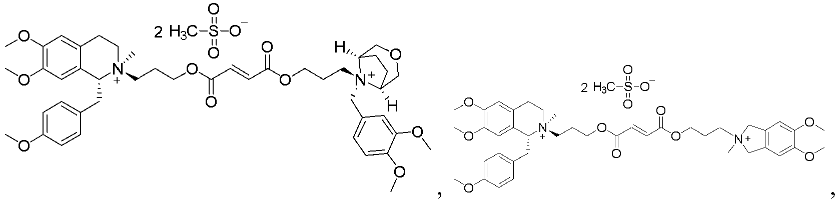

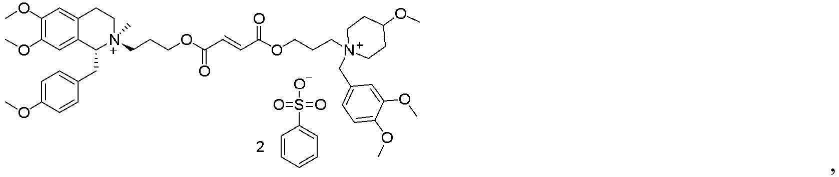

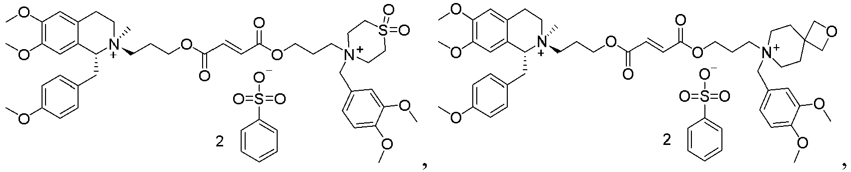

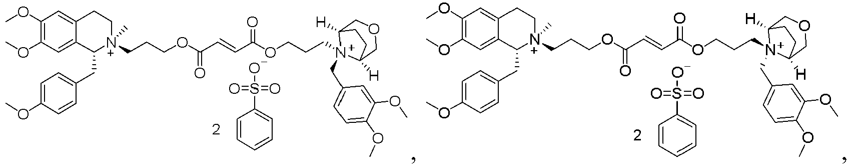

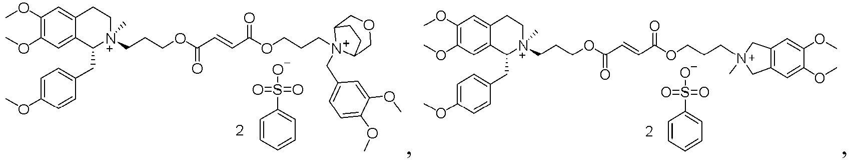

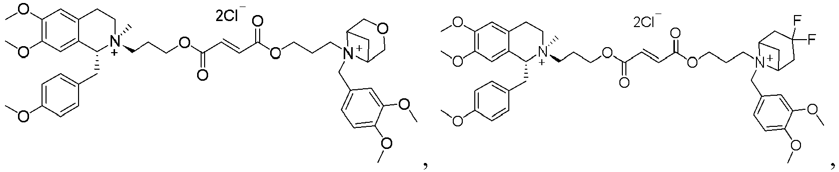

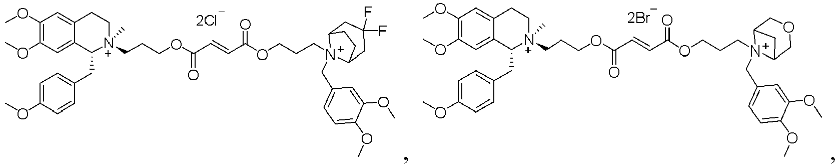

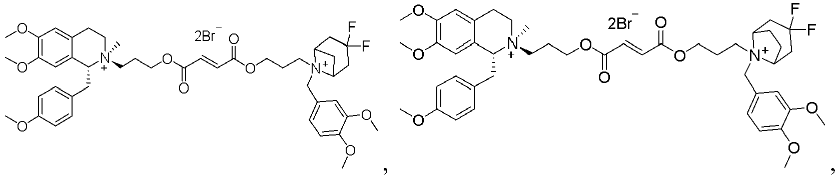

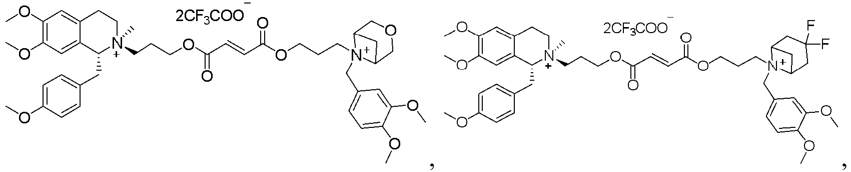

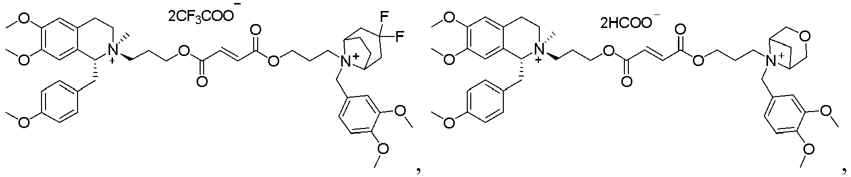

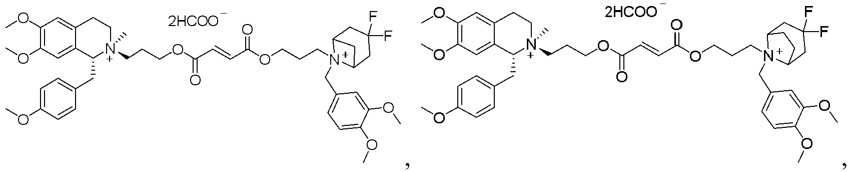

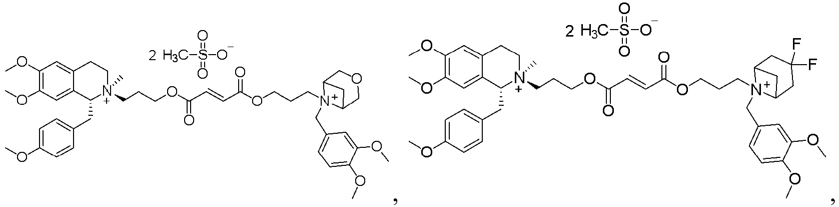

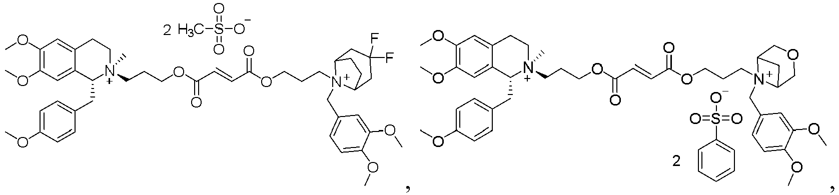

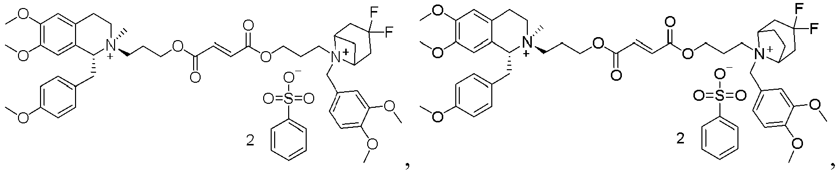

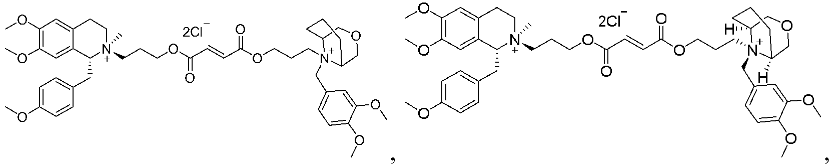

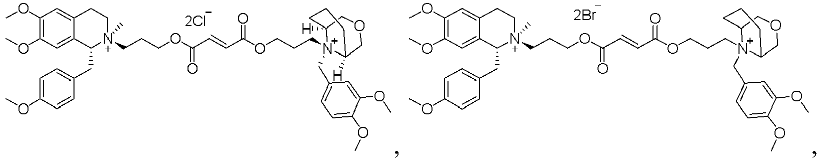

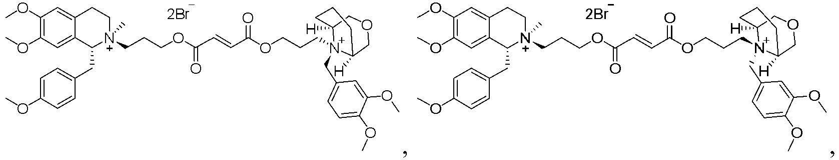

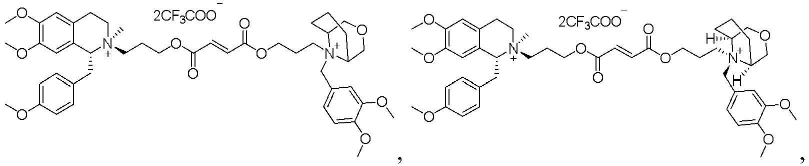

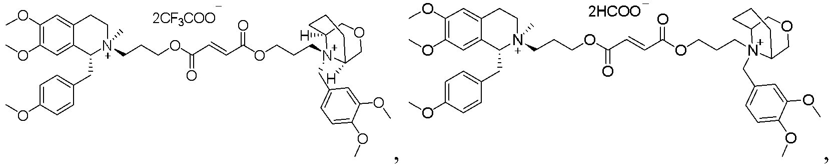

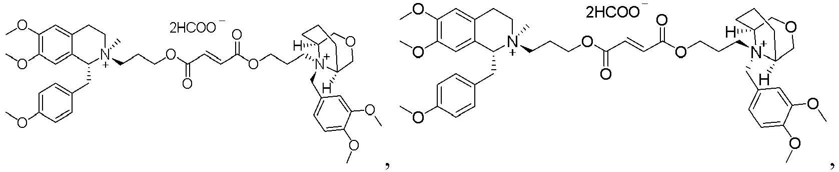

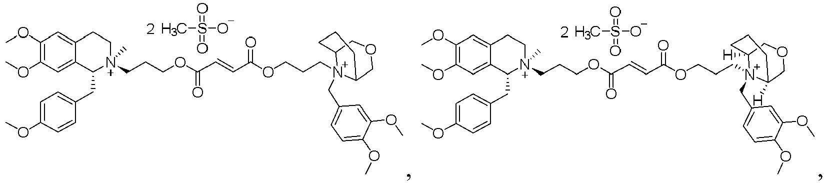

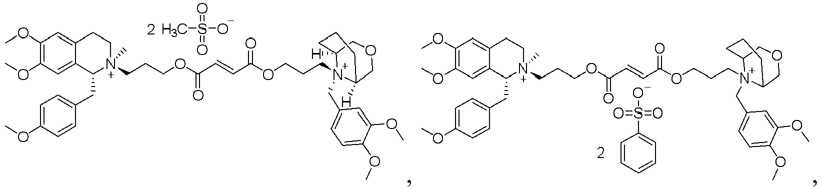

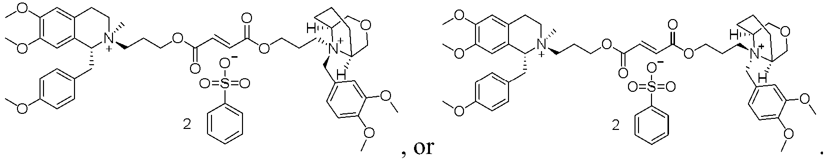

20. An isotopically substituted compound of the compound or the pharmaceutically acceptable salt thereof according to any one of claims 1 to 19, wherein preferably, the isotopically substituted compound is a deuterated compound.

21. A pharmaceutical composition comprising a therapeutically effective amount of at least one of the compound or the pharmaceutically acceptable salt thereof according to any one of claims 1 to 19, or the isotopically substituted compound according to claim 20, and a pharmaceutically acceptable excipient.

22. Use of the compound or the pharmaceutically acceptable salt thereof according to any one of claims 1 to 19, or the isotopically substituted compound according to claim 20, or the pharmaceutical composition according to claim 21 in the manufacture of a medicament for neuromuscular blockade.

23. A compound represented by formula V or a pharmaceutically acceptable salt thereof, 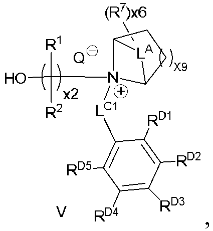 wherein Q^{⊖}, R¹, R², R^{D1}, R^{D2}, R^{D3}, R^{D4}, and R^{D5} are as defined in claim 1, and R⁷, L^{A}, L^{C1}, x2, x6, and x9 are as defined in claim 3.

24. The compound or the pharmaceutically acceptable salt thereof according to claim 23, wherein the compound represented by formula V is selected from the group consisting of the compound represented by formula VI or a pharmaceutically acceptable salt thereof, 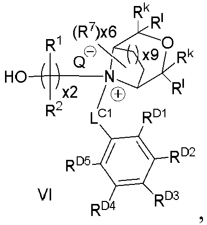 wherein Q^{⊖}, R¹, R², R^{D1}, R^{D2}, R^{D3}, R^{D4}, and R^{D5} are as defined in claim 1, R⁷, L^{C1}, x2, x6, and x9 are as defined in claim 3, and R^{k} and R^{l} are as defined in claim 6.

25. The compound represented by formula V or the pharmaceutically acceptable salt thereof, selected from the group consisting of the following compounds or pharmaceutically acceptable salts thereof, 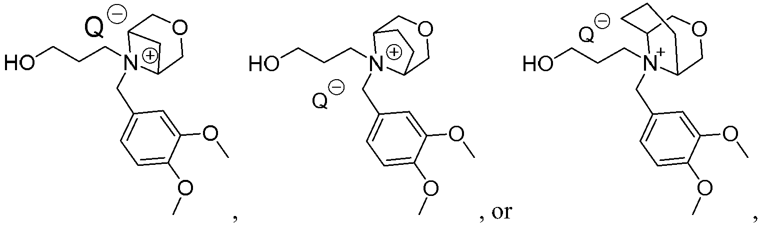 wherein Q^{⊖} is as defined in claim 1.

26. The compound represented by formula V or the pharmaceutically acceptable salt thereof, selected from the group consisting of the following compounds or pharmaceutically acceptable salts thereof,

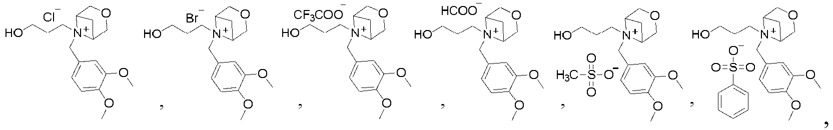

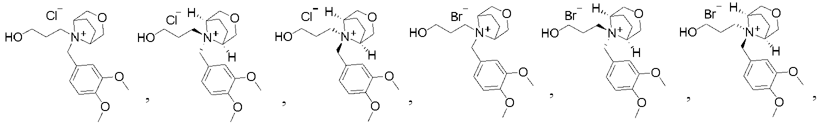

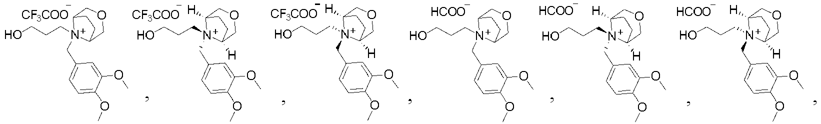

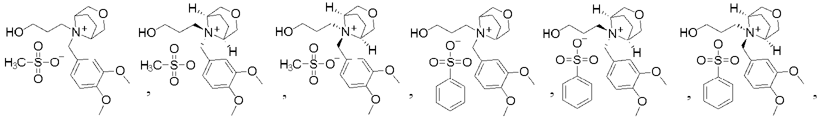

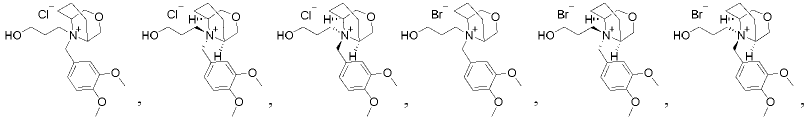

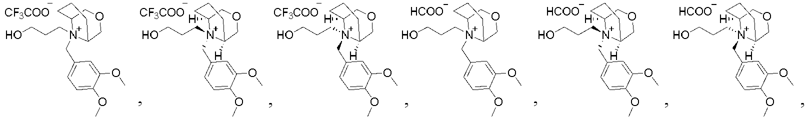

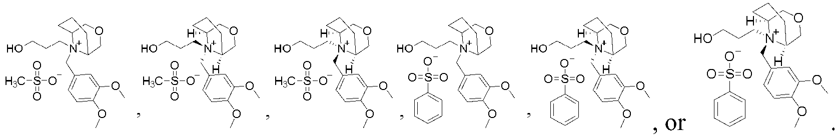

27. Use of the compound or the pharmaceutically acceptable salt thereof according to any one of claims 23 to 26 in the manufacture of a neuromuscular-blocking drug.
